# FASCICULE DE BREVET EUROPEEN

(11) **EP 1 047 664 B1**
(45) Date de publication et mention de la délivrance du brevet: **19.05.2004**
(21) Numéro de dépôt: 98959957.6
(22) Date de dépôt: 11.12.1998
(51) Int. Cl.: C07C 233/18

(54) **NOUVEAUX COMPOSES TRICYCLIQUES, LEUR PROCEDE DE PREPARATION ET LES COMPOSITIONS PHARMACEUTIQUES QUI LES CONTIENNENT**
TRICYCLISCHE VERBINDUNGEN, VERFAHREN ZU IHRER HERSTELLUNG UND DIESE ENTHALTENDE PHARMAZEUTISCHE ZUBEREITUNGEN
NOVEL TRICYCLIC COMPOUNDS, PREPARATION METHOD AND PHARMACEUTICAL COMPOSITIONS CONTAINING SAME

(30) Priorité: 16.01.1998 FR 9800424
(43) Date de publication de la demande: 02.11.2000
(73) Titulaire: LES LABORATOIRES SERVIER, 92200 Neuilly sur Seine (FR)
(72) Inventeur: LANGLOIS, Michel, F-92330 Sceaux (FR); MATHE-ALLAINMAT, Monique, F-91300 Massy (FR); JELLIMANN, Carole, F-75012 Paris (FR); ANDRIEUX, Jean, F-92160 Antony (FR); BENNEJEAN, Caroline, F-94220 Charenton Le Pont (FR); RENARD, Pierre, F-78150 Le Chesnay (FR); DELAGRANGE, Philippe, F-92130 Issy les Moulineaux (FR)
(86) Numéro de dépôt international: PCT/FR1998/002694
(87) Numéro de publication internationale: WO 1999/036392

(56) Documents cités:
- EP-A- 0 708 099
- EP-A- 0 737 670
- EP-A- 0 745 584
- WO-A-95/29173
- WO-A-96/08466
- WO-A-97/32871
- GILBERTO SPADONI ET AL.: "Conformationally restrained melatonin analogues" JOURNAL OF MEDICINAL CHEMISTRY., vol. 40, no. 13, 1997, pages 1990-2002, XP002079610 WASHINGTON US
- HANNAH PICKERING ET AL.: "Analogues of diverse structure are unable to differentiate native melatonin receptors in the chicken retina,..." BRITISH JOURNAL OF PHARMACOLOGY, vol. 119, no. 2, 1996, pages 379-387, XP002079611 Stockton Press

## Description

La présente invention concerne de nouveaux dérivés tricycliques, leur procédé de préparation et les compositions pharmaceutiques qui les contiennent.

On connaît, dans l'art antérieur, des dérivés 2,3-dihydrophénalènes (J. Chem. Soc. C, 1971, 9, pp1607-1609) décrits en tant qu'intermédiaires de synthèse, ainsi que des composés 1,3,4,5-tétrahydrobenzo[*cd*]indoles (EP 353557) utiles pour la préparation d'inhibiteurs d'aggrégation plaquettaire.
Par ailleurs, la demande EP 737670 décrit des composés amides tricycliques en tant que ligands des récepteurs mélatoninergiques.

De nombreuses études ont mis en évidence ces dix dernières années le rôle capital de la mélatonine (N-acétyl-5-méthoxytryptamine) dans de nombreux phénomènes physiopathologiques ainsi que dans le contrôle du rythme circadien. Toutefois, elle possède un temps de demi-vie assez faible dû à une rapide métabolisation. Il est donc très intéressant de pouvoir mettre à la disposition du clinicien des analogues de la mélatonine, métaboliquement plus stables et présentant un caractère agoniste ou antagoniste, dont on peut attendre un effet thérapeutique supérieur à celui de l'hormone elle-même.
Outre leur action bénéfique sur les troubles du rythme circadien (J. Neurosurg. 1985, 63, pp 321-341) et du sommeil (Psychopharmacology, 1990, 100, pp 222-226), les ligands du système mélatoninergique possèdent d'intéressantes propriétés pharmacologiques sur le système nerveux central, notamment anxiolytiques et antipsychotiques (Neuropharmacology of Pineal Sécrétions, 1990, 8 (3-4), pp 264-272) et analgésiques (Pharmacopsychiat., 1987, 20, pp 222-223) ainsi que pour le traitement des maladies de Parkinson (J. Neurosurg. 1985, 63, pp 321-341) et d'Alzheimer (Brain Research, 1990, 528, pp 170-174). De même, ces composés ont montré une activité sur certains cancers (Melatonin - Clinical Perspectives, Oxford University Press, 1988, pp 164-165), sur l'ovulation (Science 1987, 227, pp 714-720), sur le diabète (Clinical Endocrinology, 1986, 24, pp 359-364), et sur le traitement de l'obésité (International Journal of Eating Disorders, 1996, 20 (4), pp 443-446).

Ces différents effets s'exercent par l'intermédiaire de récepteurs spécifiques de la mélatonine. Des études de biologie moléculaire ont montré l'existence de plusieurs sous-types réceptoriels pouvant lier cette hormone (Trends Pharmacol. Sci., 1995, 16, p 50 ; WO 97.04094). Certains de ces récepteurs ont pu être localisés et caractérisés pour différentes espèces, dont les mammifères. Afin de pouvoir mieux comprendre les fonctions physiologiques de ces récepteurs, il est d'un grand intérêt de disposer de ligands spécifiques. De plus, de tels composés, en interagissant sélectivement avec l'un ou l'autre de ces récepteurs, peuvent être pour le clinicien d'excellents médicaments pour le traitement des pathologies liées au système mélatoninergique, dont certaines ont été mentionnées précédemment.

Les composés de la présente invention sont nouveaux et présentent une très grande affinité pour les récepteurs de la mélatonine et/ou une sélectivité pour l'un ou l'autre des sous-types réceptoriels mélatoninergiques.

La présente invention concerne plus particulièrement les composés de formule (I) : 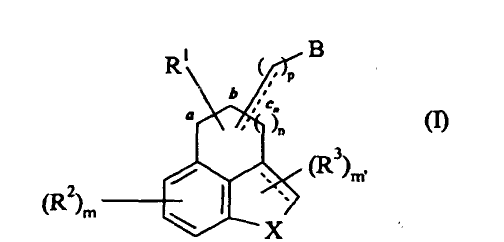 dans laquelle:
◆ R' représente un atome d'hydrogène, un atome d'halogène, un groupement alkyle (C₁-C₆) linéaire ou ramifié, alkoxy (C₁-C₆) linéaire ou ramifié, hydroxy, ou oxo,
◆ R² et R³, identiques ou différents, représentent, un atome d'halogène, un groupement Rₐ, ORₐ, CORₐ, OCORₐ ou COORₐ (avec Rₐ représentant un atome d'hydrogène, un groupement alkyle (C₁-C₆) linéaire ou ramifié éventuellement substitué, trihalogénoalkyle (C₁-C₆) linéaire ou ramifié, un groupement alcényle (C₂-C₆) linéaire ou ramifié éventuellement substitué, un groupement alcynyle (C₂-C₆) linéaire ou ramifié éventuellement substitué, un groupement cycloalkyle (C₃-C₈) éventuellement substitué, un groupement cycloalkyle (C₃-C₈) alkyle (C₁-C₆) linéaire ou ramifié éventuellement substitué, ou un groupement aryle éventuellement substitué),
◆ la représentation (R²)ₘ et (R³)_{m'} signifie que le cycle concerné peut être substitué par 1 à 3 groupements (identiques ou différents) appartenant aux définitions de R² et R³,
◆ X représente un groupement (CH₂)_{q} (où q vaut 1 ou 2) ou -CH=CH-,
◆ n est un entier tel que 0 ≤ n ≤ 3
◆ p est un entier tel que 1 ≤ p ≤ 3 lorsque n vaut 1, 2 ou 3 et que la chaîne 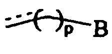 est en position b,
   et tel que 0 ≤ p ≤ 3 dans tous les autres cas,
   la chaîne 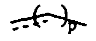 pouvant être substituée ou non par un ou plusieurs groupements, identiques ou différents, choisis parmi Rₐ, ORₐ, CORₐ, COORₐ ou atomes d'halogène,
◆ B représente :
   - un groupement 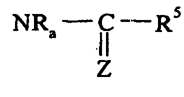
   dans lequel Rₐ est tel que défini précédemment, Z représente un atome d'oxygène ou un atome de soufre, et R⁵ représente un groupement Rₐ ou un groupement NR⁶R⁷ dans lequel R⁶ et R⁷, identiques ou différents représentent un groupement Rₐ,
   - ou un groupement 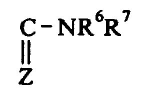
   dans lequel Z, R⁶ et R⁷ sont tels que définis précédemment,
◆ la représentation ----- signifie que la liaison peut être simple ou double étant entendu que la valence des atomes est respectée,
   étant entendu que par la représentation 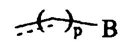 on entend les formules 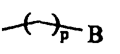 ou 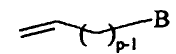 (et dans ce cas, p est différent de 0),
   étant entendu que :
   - le composé de formule (I) ne peut représenter le N-(4-méthyl-2,3-dihydro-*1H*-1-phénalényl)-1-cyclopropanecarboxamide, le N-(4-méthyl-2,3-dihydro-*1H*-1-phénalényl)-2-chloroacétamide, le N-(5-hydroxy-1,2,2a,3,4,5-hexahydro-4-acénaphtylényl)acétamide, le N-(5-hydroxy-1,2,2a,3,4,5-hexahydro-4-acénaphtylényl)benzamide ni le N-(1,2,2a,3,4,5-hexahydro-4-acénaphtylényl)acétamide,
étant entendu que :
- par "aryle" on entend un groupement phényle ou naphtyle éventuellement substitué par un ou plusieurs groupements, identiques ou différents, choisis parmi hydroxy, alkoxy (C₁-C₆) linéaire ou ramifié, alkyle (C₁-C₆) linéaire ou ramifié, cyano, nitro, amino, trihalogénoalkyle, ou atomes d'halogène,
- le terme "éventuellement substitué" affectant les expressions "alkyle", "alkényle", alcynyle", signifie que ces groupements sont substitués par un ou plusieurs groupements, identiques ou différents, choisis parmi hydroxy, alkoxy (C₁-C₆) linéaire ou ramifié, aryle, ou atomes d'halogène,
- le terme "éventuellement substitué" affectant les expressions "cycloalkyle" et "cycloalkyle alkyle" signifie que la partie cyclique est substituée par un ou plusieurs groupements, identiques ou différents, choisis parmi hydroxy, alkoxy (C₁-C₆) linéaire ou ramifié, oxo, ou atomes d'halogène,
leurs énantiomères et diastéréoisomères, ainsi que leurs sels d'addition à un acide ou une base pharmaceutiquement acceptable.

Parmi les acides pharmaceutiquement acceptables, on peut citer les acides chlorhydrique, bromhydrique, sulfurique, phosphonique, acétique, trifluoroacétique, lactique, pyruvique, malonique, succinique, glutarique, fumarique, tartrique, maléïque, citrique, ascorbique, méthanesulfonique, camphorique, oxalique.

Parmi les bases pharmaceutiquement acceptables, on peut citer l'hydroxyde de sodium, l'hydroxyde de potassium, la triéthylamine, la tertbutylamine.

Une variante avantageuse de la présente invention concerne les composés de formule (I) représentés par la formule (I_{A}) : 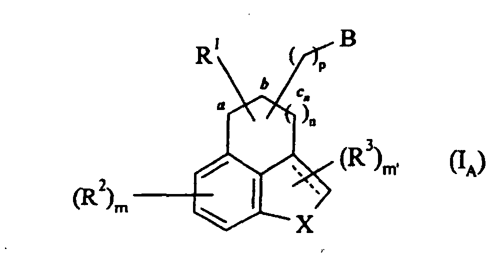 dans laquelle :
◆ R¹ représente un atome d'hydrogène, un atome d'halogène, un groupement alkyle (C₁-C₆) linéaire ou ramifié, alkoxy (C₁-C₆) linéaire ou ramifié, hydroxy, ou oxo,
◆ R² et R³, identiques ou différents, représentent, un atome d'halogène, un groupement Rₐ, ORₐ, CORₐ, OCORₐ ou COORₐ (avec Rₐ représentant un atome d'hydrogène, un groupement alkyle (C₁-C₆) linéaire ou ramifié éventuellement substitué, trihalogénoalkyle (C₁-C₆) linéaire ou ramifié, un groupement alcényle (C₂-C₆) linéaire ou ramifié éventuellement substitué, un groupement alcynyle (C₂-C₆) linéaire ou ramifié éventuellement substitué, un groupement cycloalkyle (C₃-C₈) éventuellement substitué, un groupement cycloalkyle (C₃-C₈) alkyle (C₁-C₆) linéaire ou ramifié éventuellement substitué, ou un groupement aryle éventuellement substitué),
◆ la représentation (R²)ₘ et (R³)_{m'} signifie que le cycle concerné peut être substitué par 1 à 3 groupements (identiques ou différents) appartenant aux définitions de R² et R³,
◆ X représente un groupement (CH₂)_{q} (où q vaut 1 ou 2), -CH=CH-,
◆ n est un entier tel que 0 ≤ n ≤ 3
◆ p est un entier tel que 1 ≤ p ≤ 3 lorsque n vaut 1, 2 ou 3 et que la chaîne -(CH₂)ₚ-B est en position b,
   et tel que 0 ≤ p ≤ 3 dans tous les autres cas,
   la chaîne (CH₂)ₚ, pouvant être substituée ou non par un ou plusieurs groupements identiques ou différents, choisis parmi Rₐ, ORₐ, CORₐ, COORₐ ou atomes d'halogène,
◆ B représente :
   - un groupement 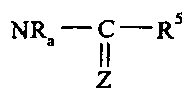 dans lequel Rₐ est tel que défini précédemment, Z représente un atome d'oxygène ou un atome de soufre, et R⁵ représente un groupement Rₐ ou un groupement NR⁶R⁷ dans lequel R⁶ et R⁷, identiques ou différents représentent un groupement Rₐ,
   - ou un groupement 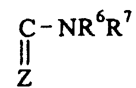
   dans lequel Z, R⁶ et R⁷ sont tels que définis précédemment,
◆ la représentation ----- signifie que la liaison peut être simple ou double étant entendu que la valence des atomes est respectée, étant entendu que :
   - le composé de formule (I) ne peut représenter le N-(4-méthyl-2,3-dihydro-*1H*-1-phénalényl)-1-cyclopropanecarboxamide, le N-(4-méthyl-2,3-dihydro-*1H*-1-phénalényl)-2-chloroacétamide, le N-(5-hydroxy-1,2,2a,3,4,5-hexahydro-4-acénaphtylényl)acétamide, le N-(5-hydroxy-1,2,2a,3,4,5-hexahydro-4-acénaphtylényl)benzamide ni le N-(1,2,2a,3,4,5-hexahydro-4-acénaphtylényl)acétamide,
   étant entendu que :
   - par "aryle" on entend un groupement phényle ou naphtyle éventuellement substitué par un ou plusieurs groupements, identiques ou différents, choisis parmi hydroxy, alkoxy (C₁-C₆) linéaire ou ramifié, alkyle (C₁-C₆) linéaire ou ramifié, cyano, nitro, amino, trihalogénoalkyle, ou atomes d'halogène,
   - le terme "éventuellement substitué" affectant les expressions "alkyle", "alkényle", alcynyle", signifie que ces groupements sont substitués par un ou plusieurs groupements, identiques ou différents, choisis parmi hydroxy, alkoxy (C₁-C₆) linéaire ou ramifié, aryle, ou atomes d'halogène,
   - le terme "éventuellement substitué" affectant les expressions "cycloalkyle" et "cycloalkyle alkyle" signifie que la partie cyclique est substituée par un ou plusieurs groupements, identiques ou différents, choisis parmi hydroxy, alkoxy (C₁-C₆) linéaire ou ramifié, oxo, ou atomes d'halogène,
   leurs énantiomères et diastéréoisomères, ainsi que leurs sels d'addition à un acide ou une base pharmaceutiquement acceptable.

Une autre variante avantageuse de la présente invention concerne les composés de formule (I) représentés par la formule (I_{B}) : 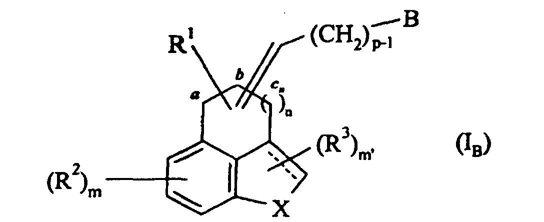 dans laquelle :
◆ R¹ représente un atome d'hydrogène, un atome d'halogène, un groupement alkyle (C₁-C₆) linéaire ou ramifié, alkoxy (C₁-C₆) linéaire ou ramifié, hydroxy, ou oxo,
◆ R² et R³, identiques ou différents, représentent, un atome d'halogène, un groupement Rₐ, ORₐ, CORₐ, OCORₐ ou COORₐ (avec Rₐ représentant un atome d'hydrogène, un groupement alkyle (C₁-C₆) linéaire ou ramifié éventuellement substitué, trihalogénoalkyle (C₁-C₆) linéaire ou ramifié, un groupement alcényle (C₂-C₆) linéaire
ou ramifié éventuellement substitué, un groupement alcynyle (C₂-C₆) linéaire ou ramifié éventuellement substitué, un groupement cycloalkyle (C₃-C₈) éventuellement substitué, un groupement cycloalkyle (C₃-C₈) alkyle (C₁-C₆) linéaire ou ramifié éventuellement substitué, ou un groupement aryle éventuellement substitué),
◆ la représentation (R²)ₘ et (R³)_{m'} signifie que le cycle concerné peut être substitué par 1 à 3 groupements (identiques ou différents) appartenant aux définitions de R² et R³,
◆ X représente un groupement (CH₂)_{q} (où q vaut 1 ou 2), -CH=CH-,
◆ n est un entier tel que 0 ≤ n ≤ 3
◆ p est un entier tel que 1 ≤ p ≤ 3
◆ la chaîne 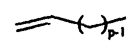 pouvant être substituée ou non par un ou plusieurs groupements, identiques ou différents, choisis parmi Rₐ, ORₐ, CORₐ, COORₐ ou atomes d'halogène,
◆ B représente :
   - un groupement 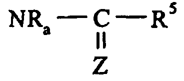 dans lequel Rₐ est tel que défini précédemment, Z représente un atome d'oxygène ou un atome de soufre, et R⁵ représente un groupement Rₐ ou un groupement NR⁶R⁷ dans lequel R⁶ et R⁷, identiques ou différents représentent un groupement Rₐ,
   - ou un groupement 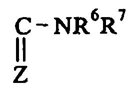 dans lequel Z, R⁶ et R⁷ sont tels que définis précédemment,
◆ la représentation ----- signifie que la liaison peut être simple ou double étant entendu que la valence des atomes est respectée,
étant entendu que :
- par "aryle" on entend un groupement phényle ou naphtyle éventuellement substitué par un ou plusieurs groupements, identiques ou différents, choisis parmi hydroxy, alkoxy (C₁-C₆) linéaire ou ramifié, alkyle (C₁-C₆) linéaire ou ramifié, cyano, nitro, amino, trihalogénoalkyle, ou atomes d'halogène,
- le terme "éventuellement substitué" affectant les expressions "alkyle", "alkényle", alcynyle", signifie que ces groupements sont substitués par un ou plusieurs groupements, identiques ou différents, choisis parmi hydroxy, alkoxy (C₁-C₆) linéaire ou ramifié, aryle, ou atomes d'halogène,
- le terme "éventuellement substitué" affectant les expressions "cycloalkyle" et "cycloalkyle alkyle" signifie que la partie cyclique est substituée par un ou plusieurs groupements, identiques ou différents, choisis parmi hydroxy, alkoxy (C₁-C₆) linéaire ou ramifié, oxo, ou atomes d'halogène,
leurs énantiomères et diastéréoisomères, ainsi que leurs sels d'addition à un acide ou une base pharmaceutiquement acceptable. Les valeurs préférées de n sont 0, 1 ou 2.

L'invention concerne les composés pour lesquels X représente un groupement (CH₂)_{q} (où q est tel que défini précédemment) ou -CH=CH-, comme par exemple un système tricyclique 2,3-dihydrophénalène, 1,2-dihydroacénaphtylène ou 7,8,9,10-tétrahydrocyclohepta[*de*]naphtalène.
Les valeurs préférées de p sont 0, 1 ou 2.

Les substituants R² et R³ préférés de l'invention sont l'atome d'hydrogène, les groupements alkoxy ou alkyle.

Le groupement R¹ préféré de l'invention est l'atome d'hydrogène.

De façon avantageuse, l'invention concerne les composés substitués par la chaîne 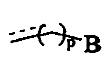 en position a ou c et plus particulièrement ceux pour lesquels p représente un entier égal à 0 (et dans ce cas la liaison ----- est simple ), 1 ou 2.

Les groupements B préférés de l'invention sont le groupement NHCOR⁵ dans lequel R⁵ est tel que défini précédemment (comme par exemple les groupements alkyle, alcényle, alcynyle, cycloalkyle, cycloalkylalkyle, aryle ou arylalkyle),
ou le groupement CONHR⁶ dans lequel R⁶ est tel que défini précédemment (comme par exemple les groupements alkyle, alcényle, alcynyle, cycloalkyle, cycloalkylalkyle, aryle ou arylalkyle).

De façon particulièrement avantageuse, l'invention concerne les systèmes tricycliques 2,3-dihydrophénalène, 1,2-dihydroacénaphtylène ou 7,8,9,10-tétrahydrocyclohepta[*de*] naphtalène, substitués ou non sur la partie naphtalénique par un ou plusieurs groupements alkoxy ou alkyle
et substitués en a ou c par un groupement 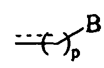 dans lequel B représente un groupement NHCOR⁵ ou CONHR⁶ (où R⁵ et R⁶ sont tels que définis précédemment).

Encore plus préférentiellement, l'invention concerne les systèmes tricycliques 1,2-dihydroacénaphtylène ou 7,8,9,10-tétrahydrocyclohepta[*de*]naphtalène,
substitués ou non sur la partie naphtalènique par un ou deux groupements alkoxy (par exemple un groupement méthoxy),
et substitués en a ou c par un groupement =CH-B, =CH-CH₂-B, -B, -CH₂-B, -(CH₂)₂-B où B représente un groupement NHCOR⁵ ou CONHR⁶ dans lesquels R⁵ et R⁶ représentent un groupement alkyle, alcényle, alcynyle, trihalogénoalkyle, cycloalkyle, cycloalkylalkyle, aryle ou arylalkyle comme par exemple méthyle, éthyle, propyle, isopropyle, butyle, . pentyle, hexyle, vinyle, propargyle, trifluorométhyle, cyclopropyle, cyclobutyle, cyclopentyle, cyclohexyle, phényle, naphtyle ou benzyle.

Très avantageusement, l'invention concerne les composés 2,3-dihydrophénalène,
substitués ou non sur la partie naphtalènique par un ou deux groupements alkoxy (par exemple un groupement méthoxy),
et substitués en a ou c par un groupement =CH-B, =CH-CH₂-B, -CH₂-B, -(CH₂)₂-B où B représente un groupement NHCOR⁵ ou CONHR⁶ dans lesquels R⁵ et R⁶ représentent un groupement alkyle, alcényle, alcynyle, trihalogénoalkyle, cycloalkyle, cycloalkylalkyle, aryle ou arylalkyle comme par exemple méthyle, éthyle, propyle, isopropyle, butyle, pentyle, hexyle, vinyle, propargyle, trifluorométhyle, cyclopropyle, cyclobutyle, cyclopentyle, cyclohexyle, phényle, naphtyle ou benzyle.

Encore plus avantageusement, l'invention concerne le N-[(4-Méthoxy-2,3-dihydro-*1H*-1-phénalényl)méthyl]acétamide, le N-[(4-Méthoxy-2,3-dihydro-*1H*-phénalényl)méthyl] propionamide, le N-[(4-Méthoxy-2,3-dihydro-*1H*-1-phénalényl)méthyl]-cyclopropane carboxamide, le N-[(4-Méthoxy-2,3-dihydro-*1H*-1-phénalényl)méthyl]butanamide, le N-[2-(4-Méthoxy-2,3-dihydro-*1H*-1-phénalényl)éthyl]acétamide, le N-[2-(4-Méthoxy-2,3-dihydro-1*H*-1-phénalényl)éthyl]propanamide, le N-[2-(4-Méthoxy-2,3-dihydro-*1H*-1-phénalényl)éthyl]1-cyclopropanecarboxamide, le N-(8-Méthoxy-1,2-dihydro-1-acénaphtylényl)acétamide, le N-[(4-Méthoxy-2,3-dihydro-*1H*-1-phénalényl)méthyl] butanamide, le N-[2-(9-Méthoxy-2,3-dihydro-*1H*-1-phénalényl)éthyl]acétamide, le N-[2-(9-Méthoxy-2,3-dihydro-*1H*-1-phénalényl)éthyl]butanamide, le N-[2-(4-Méthoxy-2,3-dihydro-*1H*-1-phénalényl)éthyl]butanamide, le N-[2-(4,9-Diméthoxy-2,3-dihydro-*1H*-1-phénalényl) éthyl]propanamide, le N-[2-(4,9-Diméthoxy-2,3-dihydro-*1H*-1-phénalényl)éthyl] butanamide, le N-[2-(4,9-Diméthoxy-2,3-dihydro-*1H*-1-phénalényl)éthyl]-1-cyclopropane carboxamide, le N-[2-(4,9-Diméthoxy-2,3-dihydro-*1H*-1-phénalényl)éthyl]acétamide, le N-[2-(9-Méthoxy-2,3-dihydro-*1H*-1-phénalényl)méthyl]acétamide, le N-[2-(9-Méthoxy-2,3-dihydro-*1H*-1-phénalényl)méthyl]butanamide, le N-[2-(4,9-Diméthoxy-2,3-dihydro-*1H*-1-phénalényl)méthyl]acétamide, le N-[2-(4,9-Diméthoxy-2,3-dihydro-*1H*-1-phénalényl) méthyl]propanamide, le N-[2-(4,9-Diméthoxy-2,3-dihydro-*1H*-1-phénalényl)méthyl] butanamide, le N-[2-(4,9-Diméthoxy-2,3-dihydro-*1H*-1-phénalényl)méthyl]-1-cyclopropane carboxamide, le *(E)*N-Méthyl-2-(4-méthoxy-2,3-dihydro-1*H*-1-phénalényliden)acétamide, le *(Z)*N-Méthyl-2-(4-méthoxy-2,3-dihydro-1*H*-1-phénalényliden)acétamide, le N-(1,2-Dihydro-1-acénaphtylénylméthyl)acétamide, le N-(1,2-Dihydro-1-acénaphtylénylméthyl) propanamide, le N-(1,2-Dihydro-1-acénaphtylénylinéthyl)butanamide, le N-(1,2-Dihydro-1-acénaphtylénylméthyl)1-cyclopropane carboxamide, le N-(8-Méthoxy-1,2-dihydro-1-acénaphtylméthyl)acétamide, le N-(8-Méthoxy-1,2-dihydro-1-acénaphtylméthyl) propanamide, le N-(8-Méthoxy-1,2-dihydro-1-acénaphtylméthyl)1-cyclopropane carboxamide, le N-(8-Méthoxy-1,2-dihydro-1-acénaphtylméthyl)butanamide, le N-[2-(1,2-Dihydro-1-acénaphtyl)éthyl]acétamide, le N-[2-(1,2-Dihydro-1-acénaphtyl)éthyl] propanamide, le N-[2-(1,2-Dihydro-1-acénaphtyl)éthyl]butanamide, le N-[2-(1,2-Dihydro-1-acénaphtyl)éthyl]cyclopropane carboxamide, le N-[2-(8-Méthoxy-1,2-dihydro-1-acénaphtyl)éthyl]acétamide, le N-[2-(8-Méthoxy-1,2-dihydro-1-acénaphtyl)éthyl] propanamide, le N-[2-(8-Méthoxy-1,2-dihydro-1-acénaphtyl)éthyl]butanamide, le N-[2-(8-Méthoxy-1,2-dihydro-1-acénaphtyl)éthyl]-1-cyclopropane carboxamide, le N-[2-(1-Méthoxy-7,8,9,10-tétrahydrocyclohepta[*de*]naphtalèn-7-yliden)éthyl]propanamide.

Les énantiomères et diastéréoisomères, ainsi que les sels d'addition à un acide ou à une base pharmaceutiquement acceptable des composés préférés de l'invention font partie intégrale de la présente invention.

L'invention s'étend également au procédé de préparation des composés de formule (I) caractérisé en ce que l'on utilise comme produit de départ :
◆ le composé de formule (II) : 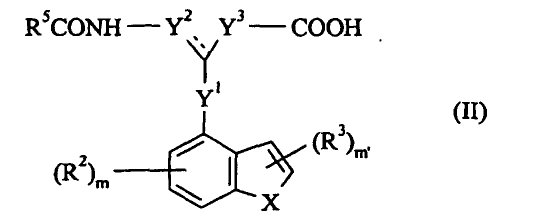
dans laquelle R², R³, R⁵, X, m, m' et la représentation ----- sont tels que définis précédemment,
- Y²: représente un groupement (CH₂)_{q} (où q vaut 1, 2 ou 3, ou q vaut 0 lorsque la représentation ----- est une liaison simple),
- Y¹: représente un groupement (CH₂)_{q'} (où q' vaut 0, 1, 2 ou 3), substitué par un groupement R' tel que défini précédemment,
- Y³: représente un groupement (CH₂)_{q''} (où q" vaut 0, 1, 2 ou 3), substitué par un groupement R¹ tel que défini précédemment avec q'+q" ≤ 3 et R¹ représente obligatoirement un atome d'hydrogène dans au moins un des deux groupements Y¹ et Y³,
qui est cyclisé en milieu basique pour conduire au composé de formule (III) : 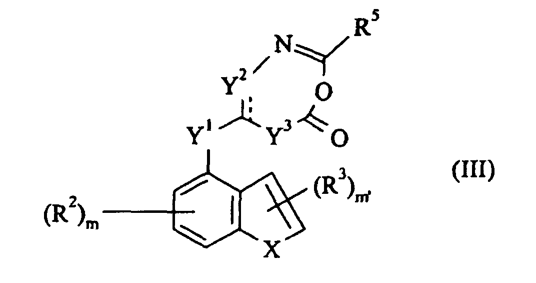 dans laquelle R², R³, R⁵, X, Y¹, Y², Y³, m, m' et la représentation ----- sont tels que définis précédemment,
qui est ensuite mis en réaction avec un acide de Lewis pour obtenir un composé de formule (I/a), cas particulier des composés de formule (I) : 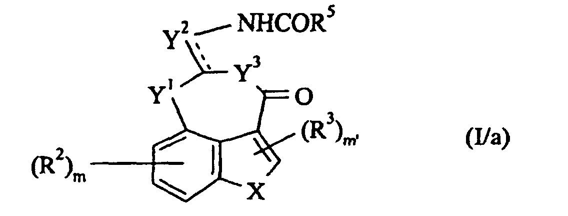 dans laquelle R², R³, R⁵, X, Y¹, Y², Y³, m, m' et la représentation ----- sont définis comme précédemment,
qui est alors réduit pour obtenir le composé de formule (I/b) cas particulier des composés de formule (I) : 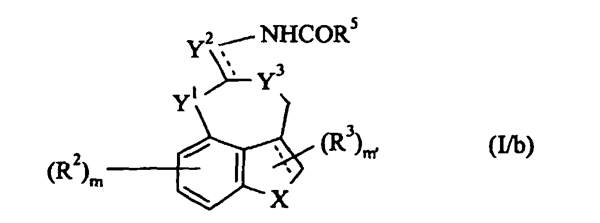 dans laquelle R², R³, R⁵, X, Y¹, Y², Y³, m, m' et la représentation ----- ont la même définition que précédemment,
◆ ou le composé de formule (IV) : 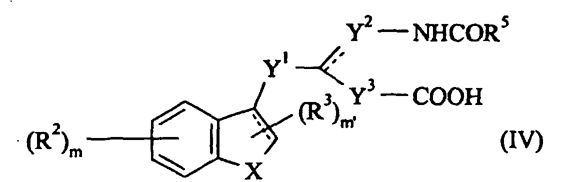
dans laquelle R², R³, R⁵, X, Y¹, Y², Y³, m, m' et la représentation ----- sont tels que définis précédemment, qui est successivement
- cyclisé
- mis en réaction avec un acide de Lewis
pour conduire au composé de formule (I/c), cas particulier des composés de formule (I) : 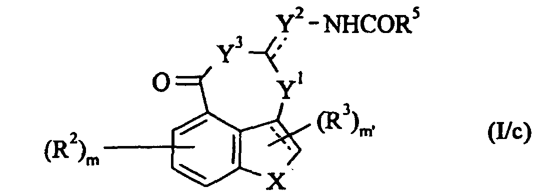 dans laquelle R², R³, R⁵, X, Y¹, Y², Y³, m, m' et la représentation ----- sont définis comme précédemment, qui est réduit pour obtenir le composé de formule (I/d), cas particulier des composés de formule (I) : 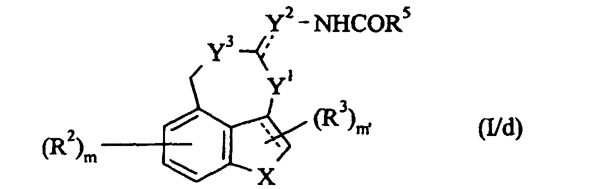 dans laquelle R², R³, R⁵, X, Y¹, Y², Y³, m, m' et la représentation ----- sont définis comme précédemment,
l'ensemble des composés (I/a), (I/b), (I/c) et (I/d) formant le composé de formule (I/e), cas particulier des composés de formule (I) : 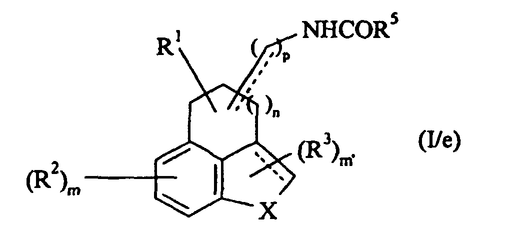 dans laquelle R¹, R², R³, R⁵, n, p, X, m,m' et la représentation ----- sont tels que définis précédemment,
qui est :
- soit soumis à l'action d'un composé de formule (V) : R'ₐ-W (V) dans laquelle R'ₐ peut prendre toutes les valeurs du groupement Rₐ tel que défini précédemment à l'exception de l'atome d'hydrogène, et W représente un groupe partant comme un atome d'halogène
ou un groupement tosyle pour conduire au composé de formule (I/f), cas particulier des composés de formule (I) : 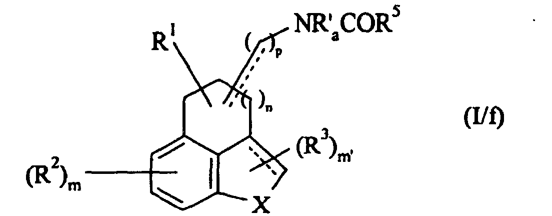 dans laquelle R¹, R², R³, R⁵, R'ₐ, n, p, X, m, m' et la représentation ----- ont la même définition que précédemment,
l'ensemble des composés de formule (I/e) et (I/f) formant le composé de formule (I/g) : 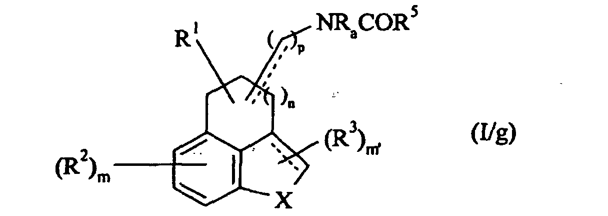 dans laquelle R¹, R², R³, R⁵, Rₐ, n, p, X, m, m'et la représentation ----- sont tels que définis précédemment,
qui peut être soumis à un agent de thionation comme le réactif de Lawesson pour obtenir le composé de formule (I/h), cas particulier des composés de formule (I) : 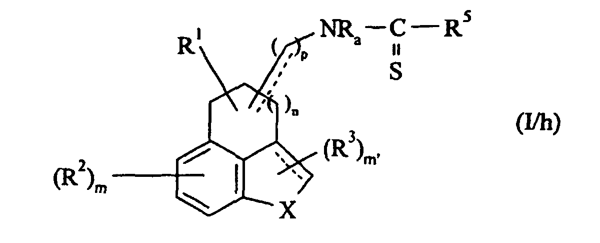 dans laquelle R¹, R², R³, R⁵, Rₐ, n, p, X, m, m' et la représentation ----- sont définis comme précédemment,
- soit hydrolysé en milieu basique afin de conduire au composé de formule (VI) : 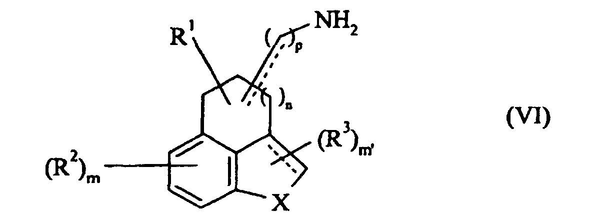
dans laquelle R¹, R², R³, n, p, X, m, m' et la représentation ----- sont tels que définis précédemment, qui est :
- soit soumis à l'action d'un sel de pyrylium pour conduire au composé de formule (VII) : 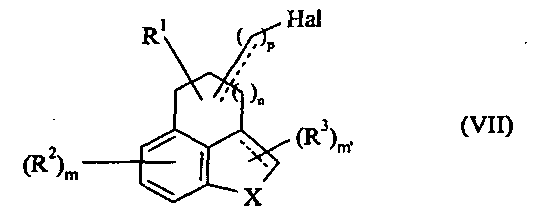
dans laquelle Hal représente un atome d'halogène et R¹, R², R³, n, p, X, m, m' et la représentation ----- sont définis de la même façon que précédemment,
sur lequel on condense un sel de cyanure afin d'obtenir le composé de formule (VIII) : 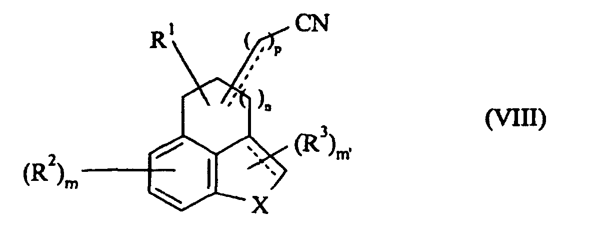 dans laquelle R¹, R², R³, n, p, X, m, m' et la représentation ----- sont tels que définis précédemment, qui est hydrolysé en milieu acide ou basique pour conduire au composé de formule (IX) : 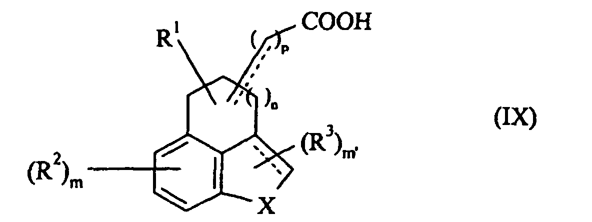 dans laquelle R¹, R², R³, n, p, X, m, m' et la représentation ----- sont définis comme précédemment,
qui est soumis, après activation sous forme de chlorure d'acide ou en présence d'agent de couplage, à l'action d'une amine HNR⁶R⁷ pour conduire au composé de formule (I/i), cas particulier des composés de formule (I) : 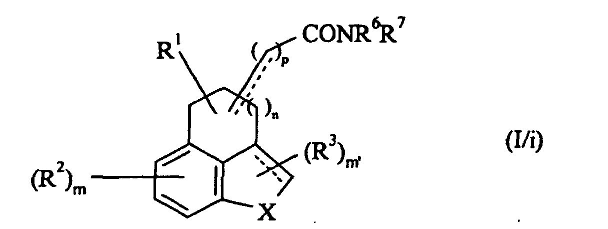 dans laquelle R¹, R², R³, R⁶, R⁷, n, p, X, m, m' et la représentation ----- sont tels que définis précédemment,
qui peut être soumis à un agent de thionation comme le réactif de Lawesson pour obtenir le composé (I/j), cas particulier des composés de formule (I) : 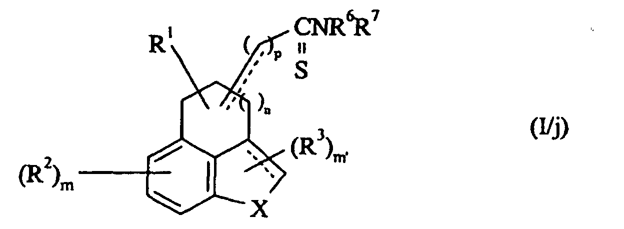 dans laquelle R¹, R², R³, R⁶, R⁷, n, p, X, m, m'et la représentation ----- sont tels que définis précédemment,
- soit soumis à l'action d'un composé de formule (X) :

   Z = C = NR⁶R⁷ (X)
dans laquelle Z, R⁶ et R⁷ sont définis comme précédemment,
pour conduire au composé de formule (I/k), cas particulier des composés de formule (I) : 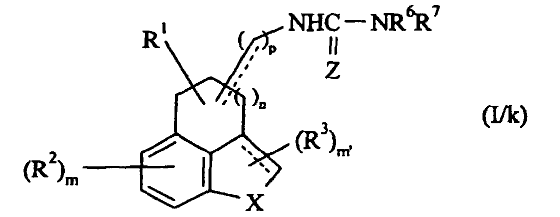 dans laquelle R¹, R², R³, R⁶, R⁷, n, p, Z, m, m' et la représentation ----- sont définis comme précédemment,
sur lequel on peut condenser un composé de formule (V) pour conduire au composé de formule (I/1), cas particulier des composés de formule (I) : 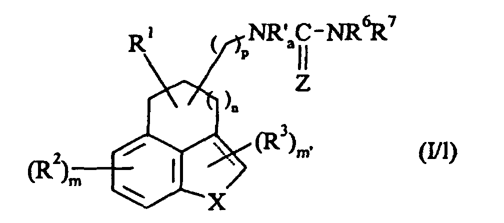 dans laquelle R¹, R², R³, R⁶, R⁷, R'ₐ, n, p, X, Z, m, m'et la représentation ----- sont tels que définis précédemment,
les composés (I/a) à (I/l) pouvant être purifiés selon une technique classique de séparation, que l'on transforme si on le souhaite en leurs sels d'addition à un acide ou à une base pharmaceutiquement acceptable, et dont on sépare éventuellement les isomères selon une technique classique de séparation.

Par ailleurs, les composés de formule (I/a) à (I/I), cas particuliers des composés de formule (I) substitués par la chaîne 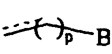 en position a ou c peuvent être obtenus grâce au procédé de préparation caractérisé en ce que l'on utilise comme produit de départ le composé de formule (XIV) : 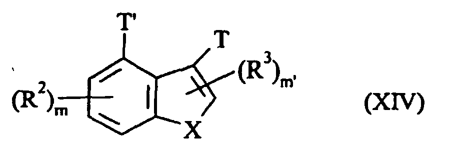 dans laquelle R², R³, X, m et m' sont tels que définis précédemment et T et T', différents, représentent un atome d'hydrogène ou un groupement -CHO,
que l'on soumet à une réaction de Wittig puis à une réduction catalytique pour obtenir le composé de formule (XV) : 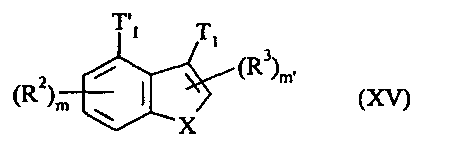 dans laquelle R², R³, X, m et m' sont définis comme précédemment et T'₁ et T₁, représentent un atome d'hydrogène ou un groupement de formule (XVI) : 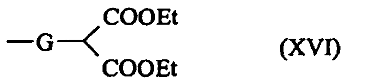 dans laquelle G représente un groupement (CH₂)_{n'} où n'=1, 2 ou 3 éventuellement substitué par un groupement R¹ défini comme précédemment, étant entendu qu'un des deux groupements T'₁ ou T₁ représente un atome d'hydrogène,
qui est successivement saponifié en milieu basique puis décarboxylé par chauffage pour conduire au composé de formule (XVII) : 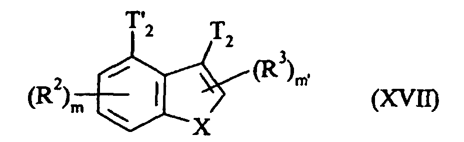 dans laquelle R², R³, X, m et m' sont définis comme précédemment et T'₂ et T₂ représentent un atome d'hydrogène ou un groupement de formule (XVIII) : 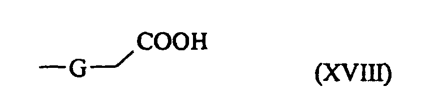 dans laquelle G est défini comme précédemment, étant entendu qu'un des deux groupements T'₂ ou T₂ représente un atome d'hydrogène,
qui est soumis à une cyclisation en présence d'un acide de Lewis après activation au chlorure d'oxalyle, pour conduire au composé de formule (XIX) : 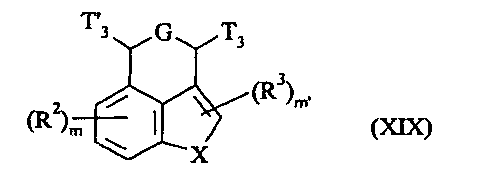 dans laquelle R², R³, X, G, m et m' sont tels que définis précédemment, et T'₃ et T₃, différents, représentent un atome d'hydrogène ou un groupement oxo,
que l'on soumet :
- soit à une réaction de Wittig (éventuellement suivie d'une réduction) puis à une saponification pour conduire au composé de formule (XX) : 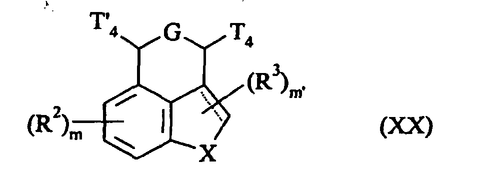
dans laquelle R², R³, X, G, m, m' et la représentation ----- sont définis comme précédemment, et T₄ et T'₄ représentent un atome d'hydrogène ou forment, avec l'atome de carbone qui les porte un groupement 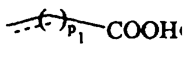 où p₁ vaut 1, 2 ou 3, étant entendu que l'un des deux groupements T₄ ou T'₄ représente un atome d'hydrogène,
- soit successivement
   * à une réduction en alcool correspondant
   * à une halogénation en présence de SOCl₂ par exemple
   * à la condensation d'un sel de cyanure
   * à une hydrolyse acide ou basique
   pour conduire au composé de formule (XXI) : 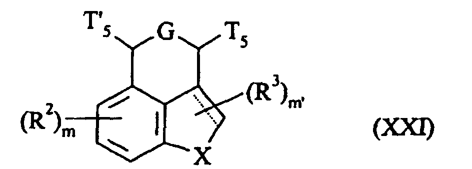 dans laquelle R², R³, X, G, m, m' et la représentation ----- sont définis comme précédemment, et T'₅ et T₅, différents, représentent un atome d'hydrogène ou un groupement COOH,
   l'ensemble des composés (XX) et (XXI) formant le composé de formule (XXII) : 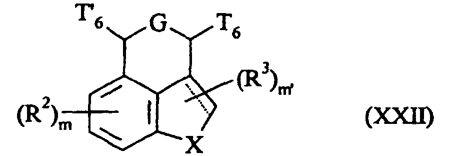 dans laquelle R², R³, X, G, m, m' et la représentation ----- sont définis comme précédemment, et T'₆ et T₆ représentent un atome d'hydrogène ou forment, avec l'atome de carbone qui les porte un groupement 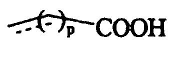 où p est défini comme précédemment, étant entendu qu'un des deux groupements T'₆ ou T₆ représente un atome d'hydrogène,
   le composé (XXII) pouvant être par ailleurs obtenu à partir du composé de formule (XIX) par condensation selon une réaction de Wittig, d'un composé contenant un nitrile (puis réduction éventuelle de la double liaison), et hydrolyse du nitrile, qui est :
   - soit soumis, après activation sous forme de chlorure d'acide ou en présence d'un agent de couplage, à l'action d'une amine HNR⁶R⁷ pour conduire au composé de formule (I/y), cas particulier des composés de formule (I) : 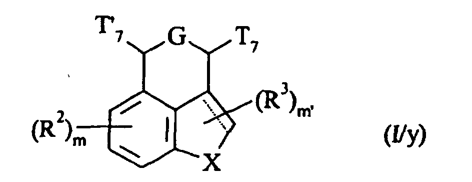
   dans laquelle R², R³, X, G, m, m' et la représentation ----- sont définis comme précédemment, et T'₇ et T₇ représentent un atome d'hydrogène ou forment, avec l'atome de carbone qui les porte un groupement 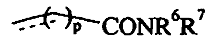 dans lequel p, R⁶ et R⁷ sont définis comme précédemment, étant entendu qu'un des deux groupements T'₇ ou T₇ représente un atome d'hydrogène,
   qui peut être soumis à un agent de thionation comme le réactif de Lawesson pour obtenir le composé (I/z), cas particulier des composés de formule (I) : 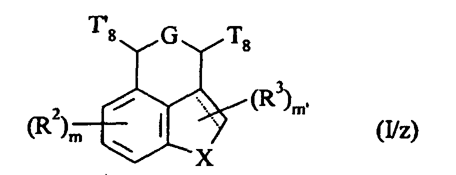 dans laquelle R², R³, X, G, m, m' et la représentation ----- sont définis comme précédemment, et T'₈ et T₈ représentent un atome d'hydrogène ou forment, avec l'atome de carbone qui les porte un groupement 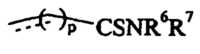 dans lequel p, R⁶ et R⁷ sont définis comme précédemment, étant entendu que l'un des deux groupements T₈ ou T'₈ représente un atome d'hydrogène,
   - soit activé en chlorure d'acide, puis traité par un azoture, chauffé en isocyanate correspondant puis hydrolysé pour conduire au composé de formule (XXIII) : 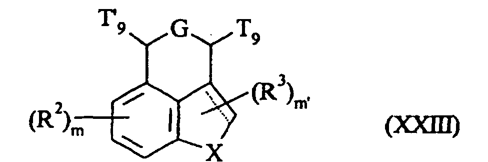
   dans laquelle R², R³, X, G, m, m' et la représentation ----- sont tels que définis précédemment, et T'₉ et T₉ représentent un atome d'hydrogène ou forment, avec l'atome de carbone qui les porte un groupement 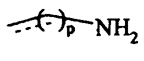 où p est tel que défini précédemment, étant entendu que l'un des deux groupements T₉ ou T'₉ représente un atome d'hydrogène,
   le composé de formule (XXIII) pouvant être par ailleurs obtenu à partir du composé de formule (XIX) par condensation selon une réaction de Wittig d'un composé contenant un nitrile puis réduction du nitrile,
   sur lequel on condense :
   - soit un chlorure d'acyle ClCOR⁵ ou l'anhydride d'acide (mixte ou symétrique) correspondant pour lesquels R⁵ est tel que défini précédemment, pour conduire au composé de formule (I/aa), cas particulier des composés de formule (I) : 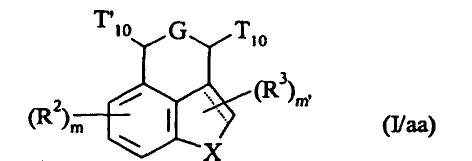 dans laquelle R², R³, X, G, m, m' et la représentation ----- sont définis comme précédemment, et T'₁₀ et T₁₀ représentent un atome d'hydrogène ou forment, avec l'atome de carbone qui les porte un groupement 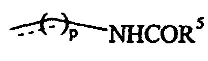 dans lequel p et R⁵ sont définis comme précédemment, étant entendu qu'un des deux groupements T'₁₀ ou T₁₀ représente un atome d'hydrogène,
      qui peut être soumis à un agent de thionation comme le réactif de Lawesson, et/ou substitué après action d'un composé de formule (V) pour conduire au composé de formule (I/ab), cas particulier des composés de formule (I) : 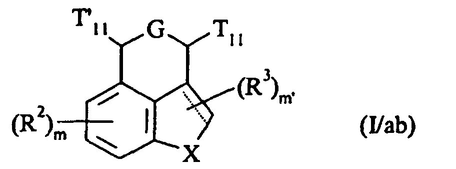 dans laquelle R², R³, X, G, m, m' et la représentation ----- sont tels que définis précédemment, et T'₁₁ et T₁₁ représentent un atome d'hydrogène ou forment, avec l'atome de carbone qui les porte un groupement 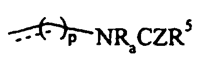 où p, Rₐ, R⁵ et Z sont définis comme précédemment, étant entendu qu'un des deux groupements T'₁₁ ou T₁₁ représente un atome d'hydrogène,
      les composés (I/y) à (I/ab) pouvant être purifiés selon une technique classique de séparation, que l'on transforme, si on le souhaite en leurs sels d'addition à un acide ou à une base pharmaceutiquement acceptable, et dont on sépare éventuellement les isomères selon une technique classique de séparation.

Les composés de départ sont :
- soit commerciaux
- soit aisément accessibles à l'homme de l'art par l'utilisation de réactions chimiques classiques
- soit décrits dans la littérature comme par exemple dans la demande EP 737670.

Les composés de l'invention et les compositions pharmaceutiques les contenant s'avèrent être utiles pour le traitement des troubles du système mélatoninergique.
L'étude pharmacologique des dérivés de l'invention a en effet montré qu'ils étaient atoxiques, doués d'une très haute affinité sélective pour les récepteurs de la mélatonine et possédaient d'importantes activités sur le système nerveux central et, en particulier, on a relevé des propriétés thérapeutiques sur les troubles du sommeil, des propriétés anxiolytiques, antipsychotiques, analgésiques ainsi que sur la microcirculation qui permettent d'établir que les produits de l'invention sont utiles dans le traitement du stress, des troubles du sommeil, de l'anxiété, des dépressions saisonnières, des pathologies cardiovasculaires, des insomnies et fatigues dues aux décalages horaires, de la schizophrénie, des attaques de panique, de la mélancolie, des troubles de l'appétit, de l'obésité, de l'insomnie, des troubles psychotiques, de l'épilepsie, du diabète, de la maladie de Parkinson, de la démence sénile, des divers désordres liés au vieillissement normal ou pathologique, de la migraine, des pertes de mémoire, de la maladie d'Alzheimer, ainsi que dans les troubles de la circulation cérébrale. Dans un autre domaine d'activité, il apparaît que dans le traitement, les produits de l'invention possèdent des propriétés d'inhibiteurs de l'ovulation, d'immunomodulateurs et qu'ils sont susceptibles d'être utilisés dans le traitement des cancers.
Les composés seront utilisés de préférence dans les traitements des dépressions saisonnières, des troubles du sommeil, des pathologies cardiovasculaires, des insomnies et fatigues dues aux décalages horaires, des troubles de l'appétit et de l'obésité.
Par exemple, les composés seront utilisés dans le traitement des dépressions saisonnières et des troubles du sommeil.

La présente invention a également pour objet les compositions pharmaceutiques contenant au moins un composé de formule (I) seul ou en combinaison avec un ou plusieurs excipients pharmaceutiquement acceptables.

Parmi les compositions pharmaceutiques selon l'invention, on pourra citer, plus particulièrement celles qui conviennent pour l'administration orale, parentérale, nasale, per. ou transcutanée, rectale, perlinguale, oculaire ou respiratoire et notamment les comprimés simples ou dragéifiés, les comprimés sublinguaux, les sachets, les paquets, les gélules, les glossettes, les tablettes, les suppositoires, les crèmes, les pommades, les gels dermiques, et les ampoules buvables ou injectables.

La posologie varie selon le sexe, l'âge et le poids du patient, la voie d'administration, la nature de l'indication thérapeutique, ou des traitements éventuellement associés et s'échelonne entre 0,01 mg et 1 g par 24 heures en 1 ou plusieurs prises.

Les exemples suivants illustrent l'invention et ne la limitent en aucune façon. Les préparations suivantes conduisent à des intermédiaires de synthèse utiles dans la préparation des composés de l'invention.

### Préparation 1 : (4-Méthoxy-2,3-dibydro-1H-1-phénalényl) méthylamine

### Stade A : Malonate de diéthyl 2-[(2-méthoxy-1-naphtyl)méthylène]

Le 2-méthoxy-1-naphtaldéhyde (25 g, 1,34.10⁻¹ mol) dans du benzène (200 ml) en présence de diéthyle malonate (25 ml, 1,65.10⁻¹ mol, 1,23 éq.) et de pipéridine (2 ml, 2,02.10⁻² mol, 0,15 éq.) est chauffé au reflux dans un Deans-Stark durant 20 heures. Après ajout de quelques gouttes de pipéridine supplémentaires, le milieu est remis au reflux durant 20 heures. Le milieu réactionnel est dilué dans du toluène (200 ml) et lavé à l'eau (125 ml). Après séparation des phases, la phase organique est traitée avec une solution d'acide chlorhydrique 1N (190 ml), puis par une solution saturée de NaHCO₃ (125 ml) et par une solution saturée de NaCl (125 ml). Après séchage sur MgSO₄ et évaporation sous pression réduite, l'huile obtenue est recristallisée dans le cyclohexane.
Point de fusion : 86°C

| Microanalyse élémentaire : | | |
|---|---|---|
| | C | H |
| % calculé | 69,50 | 6,14 |
| % trouvé | 69,53 | 6,23 |

### Stade B : Malonate de diéthyl 2-[(2-méthoxy-1-naphtyl)méthyl]

Le dérivé insaturé obtenu au stade A (10 g, 3,05.10⁻² mol) solubilisé dans de l'éthanol (510 ml) est hydrogéné en présence de Nickel de Raney à température ambiante sous forte agitation. La réaction est suivie par CCM et CPG après 4 heures d'hydrogénation. Après constatation de la disparition du produit de départ, le catalyseur est filtré sur célite et l'éthanol est évaporé sous pression réduite. Le produit du titre est obtenu sous forme d'une huile incolore.

| Microanalyse élémentaire : | | |
|---|---|---|
| | C | H |
| % calculé | 69,07 | 6,71 |
| % trouvé | 69,14 | 6,76 |

### Stade C : Acide 2-[(2-méthoxy-1-naphtyl)méthyl]malonique

Dans un monocol d'un litre, le composé obtenu au stade B (20 g, 6,05.10-2 mol) est chauffé au reflux en présence de soude (20 g, 5,00.10-1 mol) et d'eau (340 ml) pendant 4h30. Après refroidissement, le milieu est dilué dans 150 ml d'eau, filtré sur papier filtre et acidifié avec de l'acide chlorhydrique concentré à chaud (80-90°C) : les microbilles blanches formées à chaud sont filtrées sur fritté après complet refroidissement. Le diacide est lavé à l'eau froide. Après séchage à l'étuve (110°C) durant une nuit, le composé du titre est séché au dessicateur en présence de P₂O₅.
Point de fusion : 174 - 175°C

| Microanalyse élémentaire : | | |
|---|---|---|
| | C | H |
| % calculé | 65,69 | 5,15 |
| % trouvé | 65,64 | 5,18 |

### Stade D : Acide 3-(2-méthoxy-1-naphtyl)propanoïque

La décarboxylation du diacide obtenu au stade C (8,1 g, 2,95.10⁻² mol) est réalisée dans un monocol de 100 ml purgé à l'argon chauffé par un bain métallique à 165-178°C jusqu'à la fin du dégagement gazeux. L'acide est recristallisé dans un mélange CH₂Cl₂ / éther de pétrole.
Point de fusion: 131°C

### Stade E : 4-Méthoxy-2,3-dihydro-1H-phénalènone

Dans un tricot de 250 ml sous argon, à une solution de l'acide obtenu au stade D (5 g, 2,17.10⁻² mol) dans le dichlorométhane anhydre (225 ml) à 0°C est ajouté goutte à goutte le chlorure d'oxalyle (1,95 ml, 2,19.10⁻² mol, 1 éq.). Quelques gouttes de diméthylformamide anhydre sont ensuite ajoutées. Après 1h à 0°C, un léger dégagement gazeux est encore observé et le tricol est laissé à température ambiante pendant 40 minutes. Après retour à 0°C, le chlorure d'aluminium (7,5 g, 5,62.10⁻² mol, 2,6 éq.) est ajouté à la spatule. La solution initialement jaune devient rouge, orange puis vert-kaki. Après 15 minutes d'agitation à 0°C, le milieu est déversé dans un mélange glace / HCl 1N. Après séparation des phases et lavages de la phase aqueuse acide avec du dichorométhane, les phases organiques rassemblées sont lavées à l'eau, puis traitées avec une solution saturée de NaHCO₃ et enfin lavées avec une solution saturée de NaCl. Après séchage sur MgSO₄ et évaporation sous pression réduite, l'huile jaune obtenue cristallise au congélateur.
Point de fusion : 65°C

### Stade F : 2-(4-Méthoxy-2,3-dihydro-1H-1-phénalényliden)acétate d'éthyle

A une suspension dans le THF anhydre (24 ml) d'hydrure de sodium à 60 % dans l'huile (650 mg, 1,63.10⁻² mol, 1,15 éq.), préalablement lavé au pentane, est ajouté sous argon au goutte à goutte le triéthylphosphonoacétate (3,22 ml, 1,63.10⁻² mol, 1,15 éq.). Après 50 minutes d'agitation à température ambiante, le composé obtenu au stade E (3 g, 1,41.10⁻² mol, 1 éq.), dissout dans le THF anhydre (20 ml) est ajouté durant 10 minutes. Le milieu réactionnel est agité durant une nuit à température ambiante. Le milieu est dilué dans de l'eau, filtré sur célite et extrait plusieurs fois à l'éther. Après séchage sur MgSO₄ et évaporation sous pression réduite, le résidu huileux est chromatographié avec CH₂Cl₂ / éther de pétrole 60 / 40. L'huile jaune orangé obtenue cristallise à température ambiante. Elle correspond au mélange des deux isomères E / Z dont la proportion moyenne de 45 / 55 est donnée par analyse CPG.

| Microanalyse élémentaire : (mélange E/Z) | | |
|---|---|---|
| | C | H |
| % calculé | 76,57 | 6,43 |
| % trouvé | 76,43 | 6,58 |

### Stade G : 2-(4-Méthoxy-2,3-dihydro-1H-1-phénalényl)acétate d'éthyle

55 mg de PdCl₂ dans 5 ml de méthanol sont traités par 25 mg de borohydrure de sodium. Après 15 minutes d'agitation, le dérivé obtenu au stade F (1g, 3,54.10⁻³mol) dilué dans du méthanol (15 ml) est ajouté. Le milieu est purgé à l'argon et mis sous hydrogène. Le suivi de la réaction se fait par analyse CPG. Après 1h30 d'hydrogénation, le milieu réactionnel est filtré sur célite, rincé, puis évaporé sous pression réduite.

### Stade H : 2-(4-Méthoxy-2,3-dihydro-1H-1-phénalényl)acide acétique

L'ester obtenu au stade G (2 g, 7,03.10⁻³ mol) est chauffé au reflux en présence de potasse (4 g, 7.13.10⁻² mol, 10 éq.), d'eau (16 ml) et de méthanol (16 ml) durant une nuit. Après évaporation du solvant, le résidu est repris à l'eau et extrait deux fois à l'éther. La phase aqueuse basique est acidifiée avec HCl concentré à froid. L'acide est extrait à l'acétate d'éthyle et séché sur MgSO₄. Après évaporation du solvant sous pression réduite, une huile marron qui cristallise à température ambiante est obtenue.
Point de fusion : 120,5°C

| Microanalyse élémentaire : | | |
|---|---|---|
| | C | H |
| % calculé | 74,98 | 6,29 |
| % trouvé | 74,80 | 6,34 |

### Stade I : (4-Méthoxy-2,3-dihydro-1H-1-phénalényl)méthylamine, chlorhydrate

Dans un tricol de 100 ml, la triéthylamine (645 µl, 4,63 mmol, 1,15 éq.) est ajoutée goutte à goutte à une solution refroidie à 0°C de l'acide obtenu au stade H (1,03 g, 4,02 mmol) dans un mélange d'acétone (17 ml) et d'eau (1 ml). Le chloroformiate d'éthyle (500 µl, 5,23 mmol, 1,30 éq.) est ensuite additionné lentement à 0°C ; un dégagement gazeux est visible. Le milieu est agité à 0°C pendant 30 minutes ; après constatation de la disparition du produit de départ sur CCM (CH₂Cl₂), une solution d'azoture de sodium (350 mg, 5,23 mmol, 1,30 éq.) dans de l'eau (1,7 ml) est ajoutée à 0°C. Le milieu est maintenu à cette température pendant une heure. Une CCM (CH₂Cl₂) indique la formation de l'azide. Le milieu est déversé sur un mélange glace / eau puis extrait à l'éther. Les phases éthérées sont lavées à l'eau puis séchées sur Na₂SO₄, et évaporées sous vide sans chauffage. L'azide repris dans 10 ml de toluène anhydre est chauffé à 80°C jusqu'à ce qu'il n'y ait plus de dégagement d'azote. Après évaporation du toluène, l'huile correspondant à l'isocyanate est chauffée à 100°C avec une solution d'acide chlorhydrique à 20 % (8 ml) durant 3 heures ; le milieu est agité une nuit à température ambiante. Le milieu réactionnel est dilué dans de l'eau, filtré sur papier filtre, et extrait à l'éther. La phase aqueuse de pH=1 est rendue basique avec du carbonate de sodium solide puis est extraite à l'éther. Les phases éthérées rassemblées sont lavées à l'eau et séchées sur K₂CO₃. Après évaporation du solvant sous pression réduite, l'amine est convertie en chlorhydrate après solubilisation dans l'éther et traitement avec une solution d'éther chlorhydrique 4N.
Point de fusion : 237°C

| Microanalyse élémentaire : | | | |
|---|---|---|---|
| | C | H | N |
| % calculé | 68,30 | 6,88 | 5,31 |
| % trouvé | 68,23 | 6,90 | 5,25 |

En procédant comme dans la préparation 1 à partir de l'aldéhyde convenablement substitué, on obtient les préparations 2 à 18.

### Préparation 2 : 2,3-Dihydro-1H-1-phénalénylméthylamine

### Préparation 3 : (4,9-Diméthoxy-2,3-dihydro-1H-1-phénalényl)méthylamine

### Préparation 4 : (4-Ethyl-2,3-dihydro-1H-1-phénalényl)méthylamine

### Préparation 5 : (4-Chloro-2,3-dihydro-1H-1-phénalényl)méthylamine

### Préparation 6 : (6-Méthoxy-1,3,4,5-tétrahydro-3-acénaphtylényl)méthylamine

### Préparation 7 : 2-(4-Méthoxy-2,3-dihydro-1H-1-phénalényl)éthylamine

### Stade A : 2-(4-Méthoxy-2,3-dihydro-1H-1-phénalényliden)acétonitrile

A une suspension dans le THF anhydre (15 ml) d'hydrure de sodium à 60 % dans l'huile (433 mg, 1,08.10⁻² mol, 1,15 éq.), préalablement lavé au pentane, est ajouté sous argon au goutte à goutte le diéthylcyanométhylphosphonate (1,75 ml, 1,08.10-2 mol, 1,15 éq.). Après 50 minutes d'agitation à température ambiante, le composé obtenu au stade E de la préparation 1 (2 g, 9,42.10⁻² mol, 1 éq.), dissout dans le THF anhydre (15 ml) est ajouté durant 10 minutes. Le milieu réactionnel est agité durant une nuit à température ambiante. Le milieu est dilué dans de l'eau, filtré sur célite et extrait plusieurs fois à l'éther. Après séchage sur MgSO₄ et évaporation sous pression réduite, le résidu huileux est chromatographié avec CH₂Cl₂ / éther de pétrole 60 / 40. L'huile jaune orangé obtenue cristallise à température ambiante. Elle correspond au mélange des deux isomères E / Z de proportion variable de 60 / 40 à 40 / 60.

| Microanalyse élémentaire : | | | |
|---|---|---|---|
| | C | H | N |
| % calculé | 81,68 | 5,57 | 5,95 |
| % trouvé | 81,62 | 5,65 | 5,86 |

### Stade B : 2-(4-Méthoxy-2,3-dihydro-1H-1-phénalényl)acétonitrile

Le catalyseur est préparé à partir de 55 mg de PdCl₂ dans 5 ml de méthanol traités par 25 mg de borohydrure de sodium. Après 15 minutes d'agitation, le dérivé obtenu au stade A (1g, 4,25.10⁻³mol) dilué dans du méthanol (15 ml) est incorporé. Le milieu est purgé à l'argon et mis sous hydrogène. Le suivi de la réaction se fait par analyse CPG. Après 2h30 d'hydrogénation, le milieu réactionnel est filtré sur célite, rincé, puis évaporé sous pression réduite. Le produit du titre est isolé sous forme d'huile incolore.

| Microanalyse élémentaire : | | | |
|---|---|---|---|
| | C | H | N |
| % calculé | 80,98 | 6,37 | 5,90 |
| % trouvé | 80,96 | 6,47 | 5,91 |

### Stade C : 2-(4-Méthoxy-2,3-dihydro-1H-1-phénalényl)éthylamine chlorhydrate

Le nitrile obtenu dans le stade B (1,26 g, 5,31.10⁻³mol) dilué dans du méthanol (15 ml) est hydrogéné sous vive agitation à température ambiante, en présence d'ammoniaque (1 ml) et de nickel de Raney. 48 heures sont nécessaires à la disparition du produit de départ. Après filtration sur célite, puis rinçage et évaporation sous pression réduite du solvant, l'amine est reprise dans de l'éther et traitée avec quelques gouttes d'éther chlorhydrique 4N. Le chlorhydrate est recristallisé dans un mélange éthanol / éther.
Point de fusion : 223°C

| Microanalyse élémentaire : | | | |
|---|---|---|---|
| | C | H | N |
| % calculé | 80,98 | 6,37 | 5,90 |
| % trouvé | 80,96 | 6,47 | 5,91 |

En procédant comme dans la préparation 7 à partir de l'intermédiaire cétonique correspondant on obtient la préparation 8:

### Préparation 8 : 2-(6-Méthoxy-1,3,4,5-tétrahydro-3-acénaphtylényl)éthylamine

### Préparation 9 : 2-(4-Méthoxy-2,3-dihydro-1H-phénalényl)acide acétique

Un mélange du dérivé obtenu au stade B de la Préparation 7 et d'une solution de soude à 10 % est porté à reflux. La réaction est suivie par CCM. Lorsque le produit de départ a disparu, le milieu réactionnel est refroidi et extrait à pH basique, puis le milieu est acidifié par de l'acide chlorhydrique 2N puis 3N et extrait à nouveau. Après évaporation des solvants sous pression réduite, l'acide du titre est obtenu pur.
En procédant comme dans la préparation 9 à partir du nitrile correspondant, on obtient les préparations 10 à 11 :

### Préparation 10 : 2-(4-Chloro-2,3-dibydro-1H-1-phénalényl)acide acétique

### Préparation 11 : 2-(6-Méthoxy-1,3,4,5-tétrahydro-3-acénaphtylényl)acide acétique

### Préparation 12 : 3-(4-Méthoxy-2,3-dihydro-1H-1-phénalényl)propylamine

### Stade A : 3-(4-Méthoxy-2,3-dihydro-1H-1-phénalényliden)propanoate d'éthyle

On procède comme dans le stade F de la Préparation 1 en remplaçant le triéthylphosphonoacétate par le triéthylphosphonopropanoate.

Les stades B, C et D sont identiques aux stades G, H et I de la Préparation 1.

La préparation 13 est obtenue en procédant comme dans la Préparation 12 à partir de la cétone obtenue dans la Préparation 3.

### Préparation 13 : 3-(4,9-Diméthoxy-2,3-dihydro-1H-1-phénalényl)propylamine

### Préparation 14 : Acide 4-(4-Méthoxy-2,3-dihydro-1H-1-phénalényl)butanoique

### Stade A : 4-(4-Méthoxy-2,3-dihydro-1H-1-phénalényl)nitrobutane

La cétone obtenue au stade E de la Préparation 1 est soumise aux conditions des stades A et B de la Préparation 9 en remplaçant le diéthylcyanométhylphosphonate par le diéthylcyanopropylphosphonate.

### Stade B : Acide 4-(4-Méthoxy-2,3-dihydro-1H-1-phénalényl)butanoïque

On procède à une hydrolyse du nitrile obtenu au stade A dans les conditions de la Préparation 9.

### Préparation 15 : 2-(1,6-Diméthoxy-7,8,9,10-tétrahydrocyclohepta[de]naphtalen-7-yl) éthylamine

### Stade A : 4-(2,7-Diméthoxy-1naphtyl)-3-buténoate d'éthyle

On procède comme dans le stade F de la Préparation 1 en condensant le triéthylphosphonopropanoate d'éthyle sur le 2,7-diméthoxy-1-naphtaldéhyde.

### Stade B : 4-(2,7-Diméthoxy-1-naphtyl)-butanoate d'éthyle

On procède à une réduction du composé obtenu au stade A dans les conditions du stade G de la Préparation 1.

### Stade C : Acide 4-(2,7-Diméthoxy-1-naphtyl)-butanoïque

On procède à la saponification de l'ester obtenu au stade B dans les conditions du stade H de la Préparation 1.

### Stade D : 2-(1,6-Diméthoxy-7,8,9,10-tétrahydrocyclohepta[de]naphtalen-7-yl)-éthylamine

On procède comme dans les stades E de la Préparation 1, et A, B, C de la Préparation 7.

### Préparation 16 : 2-(1-Méthoxy-7,8,9,10-tétrahydrocyclohepta[de]naphtalen-7-yl) méthylamine

### Stade A : Acide 4-(2-Méthoxy-1-naphtyl)butanoïque

On procède comme dans les stades A, B, C de la Préparation 15 à partir du 2-Méthoxy-1-naphtaldéhyde.

### Stade B : 2-(1-Méthoxy-7,8,9,10-tétrahydrocyclohepta[de]naphtalen-7-yl) méthylamine

On procède comme dans les stades E, F, G, H, I de la Préparation 1.

### Préparation 17 : 6,7,8,9-Tétrahydro-2-thiabenzo[ed]azulen-9-yl-méthylamine

On procède comme dans la Préparation 16 à partir du benzo[*b*]thiophène-4-carbaldéhyde.

### Préparation 18 : (5-méthoxy-6,7,8,9-tétrahydro-2-oxobenzo[cd]azulen-9-yl) méthylamine

On procède comme dans la Préparation 16 à partir du 5-méthoxybenzo[*b*]furan-4-carbaldéhyde.

### Préparation 19 : (3-Méthoxy-2,3-dihydro-1H-1-phénalényl)méthylamine

On procède comme pour la Préparation 2 en additionnant du méthanol sur la double liaison du malonate de diéthyl 2-(1-naphtylméthylène) obtenu au stade A.
Les préparations 20 à 30 sont obtenues selon les méthodes décrites dans la demande de brevet EP 737670.

### Préparation 20 : 3-(3,8-Diméthoxy-1,2-dihydro-1-acénaphtylényl)propylamine

### Préparation 21 : Acide 4-(3,8-Diméthoxy-1,2-dihydro-1-acénaphtylényl)butanoïque

### Préparation 22 : (4-Méthoxy-2,3-dihydro-1H-2-phénalényl)méthylamine

### Préparation 23 : (4-Ethyl-2,3-dihydro-1H-2-phénalényl)méthylamine

### Préparation 24 : Acide 2-(4,9-diméthoxy-2,3-dihydro-1H-2-phénalényl)acétique

### Préparation 25 : Acide 4-méthoxy-2,3-dihydro-1H-2-phénalènecarboxylique

### Préparation 26 : 2-(4-Méthoxy-2,3-dihydro-1H-2-phénalényl)éthylamine

### Préparation 27 : 3-(4-Méthoxy-2,3-dihydro-1H-2-phénalényl)propylamine

### Préparation 28 : (6-Chloro-2,3-dihydro-1H-2-phénalényl)méthylamine

### Préparation 29 : (1,6-Diméthoxy-7,8,9,10-tétrahydrocyclohepta[de]naphtalèn-8-yl) méthylamine

### Préparation 30 : Acide 2-(1,6-diméthoxy-7,8,9,10-tétrahydrocyclohepta[de]naphtalèn-8-yl)acétique

### Préparation 31 : 8-Méthoxy-1,2-dihydro-1-acénaphtylénylamine

### Préparation 32 : 2-(9-Méthoxy-2,3-dihydro-1H-1-phénalényl)acétonitrile

On procède comme dans les stades A et B de la Préparation 7.
Point de fusion : 116°C

| Microanalyse élémentaire : | | | |
|---|---|---|---|
| | C | H | N |
| % calculé | 80,98 | 6,37 | 5,90 |
| % trouvé | 80,77 | 6,47 | 5,74 |

### Préparation 33 : 2-(4,9-Diméthoxy-2,3-dihydro-1H-1-phénalényl)éthylamine

On procède comme dans la Préparation 7.

### Préparation 34 : (9-Méthoxy-2,3-dihydro-1H-1-phénalényl)méthylamine

### Stade A : Acide 2-(9-méthoxy-2,3-dihydro-1H-1-phénalényl)acétique

Dans un monocol de 250 ml, le nitrile obtenu dans la Préparation 32 (500 mg, 2,11.10⁻³ mol) est chauffé au reflux en présence de soude à 30 % (8 ml), de méthanol (8 ml) et d'éthanol (8 ml) pendant 48 heures. Après refroidissement, le milieu est versé dans un mélange glace / eau. Le milieu est acidifié avec de l'acide chlorhydrique et extrait trois fois avec de l'acétate d'éthyle et une fois au dichlorométhane. Les phases organiques sont lavées séparément à l'eau et avec une solution saturée de NaCl, séchées sur MgSO₄ puis évaporées sous pression réduite. L'acide du titre est obtenu sous la forme d'un solide jaune pale.
Point de fusion : 147°C

| Microanalyse élémentaire: | | |
|---|---|---|
| | C | H |
| % calculé + ¹/₃ H₂O | 73,26 | 6,40 |
| % trouvé | 73,38 | 6,37 |

### Stade B : (9-Méthoxy-2,3-dihydro-1H-1-phénalényl)méthylamine

Le produit du titre est obtenu à partir de l'acide du stade A (515 mg, 2,01.10⁻³ mol) dans un mélange d'acétone (20 ml) et d'eau (600 µl), de triéthylamine (322 µl, 2,31.10⁻³ mol, 1,15 éq.) et de chloroformiate d'éthyle (250 µl, 2,61.10⁻³ mol, 1,30 éq.). L'acyle azide est formé par action d'une solution d'azoture de sodium (175 mg, 2,61.10⁻³ mol, 1,30 éq.) dans l'eau (1 ml). Après chauffage dans du toluène anhydre (5 ml), l'isocyanate est hydrolysé avec une solution d'acide chlorhydrique à 20 % (6 ml). Le milieu est agité une nuit à température ambiante. Le milieu réactionnel est dilué dans de l'eau, filtré sur papier filtre, et extrait à l'éther. La phase aqueuse de pH=1 est rendue basique avec du carbonate de sodium solide puis est extraite à l'éther. Les phases éthérées rassemblées sont lavées à l'eau et séchées sur K₂CO₃. Après évaporation du solvant sous pression réduite, l'amine est convertie en chlorhydrate après solubilisation dans l'éther et traitement avec une solution d'éther chlorhydrique 4N. Après séchage, on obtient le produit du titre sous la forme d'un solide blanc.

### Préparation 35 : Acide [(E)-2-(4-Méthoxy-2,3-dihydro-1H-phénalényl)]acétique

L'ester trans obtenu dans le stade F de la Préparation 1 (760 mg, 2,69.10⁻³ mol) est chauffé au reflux en présence de potasse (377 mg, 6,73.10⁻³ mol, 2,5 éq.), d'eau (10 ml) et de méthanol (10 ml) durant une nuit. Après évaporation du solvant, le résidu est repris à l'eau et extrait deux fois à l'éther. La phase aqueuse basique est acidiée avec HCl concentré à froid. L'acide est extrait à l'acétate d'éthyle, lavé à l'eau et séché sur MgSO₄. Après évaporation du solvant sous pression réduite, le produit du titre est obtenu sous forme d'un solide jaune.
Point de fusion : 208°C

| Microanalyse élémentaire : | | |
|---|---|---|
| | C | H |
| % calculé + ²/₃ H₂O | 72,17 | 5,80 |
| % trouvé | 72,37 | 5,56 |

### Préparation 36 : Acide [(Z)-2-(4-Méthoxy-2,3-dihydro-1H-phénalényl)]acétique

On procède comme dans la Préparation 35 à partir de l'isomère cis.
Point de fusion : 187°C

| Microanalyse élémentaire : | | |
|---|---|---|
| | C | H |
| % calculé + ¹/₃ H₂O | 73,83 | 5,68 |
| % trouvé | 73,55 | 5,63 |

### Préparation 37: 1,2-Dihydro-1-acénaphtylénylméthylamine

### Stade A : 1,2-Dihydro-1-acénaphtylénone

Dans un tricol d'un litre sous argon, à une solution d'acide (napht-1-yl) acétique (6 g, 3,22.10⁻² mol) dans le dichlorométhane anhydre (270 ml) à 0°C est ajouté goutte à goutte le chlorure d'oxalyle (2,87 ml, 3,22.10⁻² mol, 1 éq.). Quelques gouttes de diméthylformamide anhydre sont ensuite ajoutées. Après 1 heure à 0°C, un léger dégagement gazeux est encore observé et le tricol est laissé à température ambiante pendant 40 minutes. Après retour à 0°C, le chlorure d'aluminium (11,2 g, 8,38.10⁻² mol, 2,6 éq.) est ajouté progressivement à la spatule. La solution est agitée durant vingt minutes et devient de couleur vert-noir. Le milieu est versé dans un mélange glace / HCl 1N. Après séparation des phases et lavages de la phase aqueuse acide avec du dichlorométhane, les phases organiques rassemblées sont lavées à l'eau, puis traitées avec une solution saturée de NaHCO₃ et enfin lavées avec une solution saturée de NaCl. Après séchage sur MgSO₄ et évaporation sous pression réduite, le produit du titre est obtenu sous la forme d'un solide jaune.
Point de fusion : 123°C

| Microanalyse élémentaire : | | |
|---|---|---|
| | C | H |
| % calculé | 85,69 | 4,79 |
| % trouvé | 85,59 | 4,80 |

### Stade B : 2-(1,2-Dihydro-1-acénaphtylényliden)acétate d'éthyle

A une suspension dans le THF anhydre (50 ml) d'hydrure de sodium à 60 % dans l'huile (1,43 g, 3,58.10⁻² mol, 1,15 éq.), préalablement lavé au pentane, est ajouté sous argon au goutte à goutte le triéthylphosphonoacétate (7,11 ml, 3,58.10⁻² mol, 1,15 éq.). Après 40 minutes d'agitation à température ambiante, le composé obtenu au stade A (5,24 g, 3,12.10⁻² mol, 1 éq.), dissout dans le THF anhydre (55 ml) est ajouté durant 10 minutes. Le milieu réactionnel est agité durant une nuit à température ambiante, puis dilué dans de l'eau, filtré sur célite et extrait plusieurs fois à l'éther. Après séchage sur MgSO₄ et évaporation sous pression réduite, le résidu huileux est chromatographié sur colonne de gel de silice avec CH₂Cl₂ / éther de pétrole 50 / 50. Un mélange des deux isomères E / Z est isolé.

| Microanalyse élémentaire : | | |
|---|---|---|
| | C | H |
| % calculé | 80,65 | 5,92 |
| % trouvé | 80,68 | 5,97 |

### Stade C : 2-(1,2-Dihydro-1-acénaphtylényl)acétate d'éthyle

55 mg de PdCl₂ dans 5 ml de méthanol sont traités par 25 mg de borohydrure de sodium. Après 15 minutes d'agitation, le dérivé obtenu au stade B (1 g, 4,20.10⁻³ mol) dilué dans du méthanol (25 ml) est ajouté. Le milieu est purgé à l'argon et mis sous atmosphère d'hydrogène. Après 2 heures 30 d'hydrogénation, le milieu réactionnel est filtré sur célite, rincé, puis évaporé sous pression réduite.

### Stade D : Acide 2-(1,2-Dihydro-1-acénaphtylényl)acétique

L'ester obtenu au stade C (1,9 g, 7,91.10⁻³ mol) est chauffé au reflux en présence de potasse (2,34 g, 4,17.10⁻² mol, 5,3 éq.), d'eau (16 ml) et de méthanol (16 ml) durant une nuit. Après évaporation du solvant, le résidu est repris à l'eau et extrait deux fois à l'éther. La phase aqueuse basique est acidifiée avec HCl concentré à froid. L'acide est extrait à l'acétate d'éthyle et séché sur MgSO₄. Après évaporation du solvant sous pression réduite, le composé du titre est obtenu sous la forme d'un solide jaune.
Point de fusion: 123°C

| Microanalyse élémentaire : | | |
|---|---|---|
| | C | H |
| % calculé | 79,23 | 5,70 |
| % trouvé | 79,12 | 5,77 |

### Stade E : 1,2-Dihydro-1-acénaphtylénylméthylamine

Dans un tricol de 250 ml, la triéthylamine (3,49 ml, 2,50.10⁻² mol, 1,15 éq.) est ajoutée goutte à goutte à une solution refroidie à 0°C de l'acide obtenu au stade D (4,62 g, 2,18.10⁻² mol) dans un mélange d'acétone (95 ml) et d'eau (5,4 ml). Le chloroformiate d'éthyle (2,71 ml, 2,83.10⁻² mol, 1,30 éq.) est ensuite additionné lentement à 0°C ; un dégagement gazeux est visible. Le milieu est agité à 0°C pendant 30 minutes ; après constatation de la disparition du produit de départ sur CCM (CH₂Cl₂), une solution d'azoture de sodium (1,89 g, 2,83.10⁻² mol, 1,30 éq.) dans de l'eau (9,2 ml) est ajoutée à 0°C. Le milieu est maintenu à cette température pendant une heure. Le milieu est versé sur un mélange glace / eau puis extrait à l'éther. Les phases éthérées sont lavées à l'eau puis séchées sur Na₂SO₄, et évaporées sous vide sans chauffage. L'acyle azide repris dans 50 ml de toluène anhydre est chauffé à 80°C jusqu'à ce qu'il n'y ait plus de dégagement d'azote. Après évaporation du toluène, l'huile correspondant à l'isocyanate est chauffée à 100°C avec une solution d'acide chlorhydrique à 20 % (52 ml) durant 3 heures ; le milieu est agité une nuit à température ambiante. Le milieu réactionnel est dilué dans de l'eau, filtré sur papier filtre, et extrait à l'éther. La phase aqueuse de pH=1 est rendue basique avec du carbonate de sodium solide puis est extraite trois fois au dichlorométhane. Les phases organiques rassemblées sont lavées à l'eau et séchées sur K₂CO₃. Après évaporation du solvant sous pression réduite, l'amine sous forme d'une huile est ainsi obtenue (820 mg). Le solide, filtré et repris dans du dichlorométhane, est traité de la même façon que le filtrat. L'amine ainsi obtenue est convertie en chlorhydrate après solubilisation dans l'éther et traitement avec une solution d'éther chlorhydrique 4N. Après séchage, le produit du titre est obtenu sous forme d'un solide blanc.
Point de fusion : > 250°C

| Microanalyse élémentaire : | | | |
|---|---|---|---|
| | C | H | N |
| % calculé | 71,07 | 6,42 | 6,37 |
| % trouvé | 70,84 | 6,49 | 6,40 |

### Préparation 38 : (8-Méthoxy-1,2-dihydro-1-acénaphtylényl)méthylamine

On procède comme dans la Préparation 37 à partir de l'acide (7-méthoxy-napht-1-yl)acétique.

### Préparation 39 : 1,2-Dihydro-1-acénaphtylényléthylamine

### Stade A : (1,2-Dihydro-1-acénaphtylényliden)acétonitrile

On procède comme dans le stade B de la Préparation 37 en remplaçant le triéthylphosphonoacétate par le diéthylcyanométhylphosphonate.

| Microanalyse élémentaire : | | | |
|---|---|---|---|
| | C | H | N |
| % calculé | 87,93 | 4,74 | 7,32 |
| % trouvé | 87,85 | 4,79 | 7,25 |

### Stade B : (1,2-Dihydro-1-acénaphtylényl)acétonitrile

Le catalyseur est préparé à partir de 55 mg de PdCl₂ dans 5 ml de méthanol traités par 25 mg de borohydrure de sodium. Après 15 minutes d'agitation, le dérivé obtenu au stade A (1 g, 5,23.10⁻³ mol) dilué dans du méthanol (15 ml) est incorporé. Le milieu est purgé à l'argon et mis sous hydrogène. Après 3 jours d'hydrogénation, le milieu réactionnel est filtré sur célite, rincé, puis évaporé sous pression réduite. Le produit du titre est isolé sous forme d'une huile marron clair.

| Microanalyse élémentaire : | | | |
|---|---|---|---|
| | C | H | N |
| % calculé + ⅛ H₂O | 86,01 | 5,80 | 7,16 |
| % trouvé | 85,73 | 5,87 | 7,13 |

### Stade C : 1,2-Dihydro-1-acénaphtylényléthylamine

Le nitrile obtenu au stade B (900 mg, 4,66.10⁻³ mol) dilué dans du méthanol (30 ml) est hydrogéné sous vive agitation à température ambiante, en présence d'ammoniaque (2 ml) et de nickel de Raney. Après 23 heures, le produit de départ a disparu. Après filtration sur célite, puis rinçage et évaporation sous pression réduite du solvant, l'amine du titre est obtenue sous forme d'une huile. Celle-ci est utilisée sans purification.

### Préparation 40 : (8-Méthoxy-1,2-dihydro-1-acénaphtylényl)éthylamine

On procède comme dans la Préparation 39.

### Stade A : (8-Méthoxy-1,2-dihydro-1-acénaphtylényl)acétonitrile

Stade B : (8-Méthoxy-1,2-dihydro-1-acénaphtylényl)éthylamine

| Microanalyse élémentaire : | | | |
|---|---|---|---|
| | C | H | N |
| % calculé | 71,07 | 6,42 | 6,37 |
| % trouvé | 70,84 | 6,49 | 6,40 |

### Préparation 41: 2-(1-Méthoxy-7,8,9,10-tétrahydrocyclohepta[de]naphtalèn-7-yliden)acétonitrile

### Stade A : Acide 3-(2-méthoxy-napht-1-yl)buténoïque

A une suspension sous argon à 0°C dans le THF anhydre (10 ml) d'hydrure de sodium à 60 % dans l'huile (1 ,16 g, 2,90.10⁻² mol, 2,2 éq.) préalablement lavé au pentane, est ajouté au goutte à goutte le mélange de 2-méthoxy-1-naphtaldéhyde (2,45 g, 1,32.10⁻² mol, 1 éq.) et le bromure de l'acide (3-triphénylphosphonium) propanoïque (6 g, 1,44.10⁻² mol, 1,1 éq.) solubilisé dans un mélange anhydre de THF et de DMSO (17 ml / 17 ml). Le milieu réactionnel est agité durant une nuit à température ambiante, puis dilué dans de l'eau, filtré sur célite et après ajout de quelques gouttes de soude, extrait deux fois à l'éther. La phase aqueuse basique est acidifiée avec HCl à froid. L'acide désiré est extrait à l'éther. Après lavage de la phase éthérée avec une solution saturée de NaCl puis séchage sur MgSO₄ et évaporation sous pression réduite, le résidu solide est chromatographié sur colonne de gel de silice (1. CH₂Cl₂ ; 2. CH₂Cl₂ / MeOH : 97 / 3). Un solide blanc correspondant au mélange des deux isomères E / Z du composé du titre est obtenu. Point de fusion : 112°C

| Microanalyse élémentaire : | | |
|---|---|---|
| | C | H |
| % calculé | 74,36 | 5,82 |
| % trouvé | 74,47 | 5,90 |

### Stade B : Acide 4-(2-méthoxy-napht-1-yl)butanoïque

Le dérivé obtenu au stade A (3,4 g, 1,40.10⁻² mol) est solubilisé dans de l'acétate d'éthyle (90 ml) en présence de palladium sur charbon à 5 %. Le milieu est purgé à l'argon et mis sous atmosphère d'hydrogène. Après 15 heures d'hydrogénation, le milieu réactionnel est filtré sur célite, rincé, puis évaporé sous pression réduite. Le produit du titre est isolé sous forme de cristaux blancs.
Point de fusion : 88°C

| Microanalyse élémentaire : | | |
|---|---|---|
| | C | H |
| % calculé | 73,75 | 6,60 |
| % trouvé | 73,59 | 6,71 |

### Stade C : 1-Méthoxy-7,8,9,10-tétrahydrocyclohepta[de]naphtalèn-7-one

On procède comme dans le stade E de la Préparation 1.
Point de fusion : 67°C

| Microanalyse élémentaire : | | |
|---|---|---|
| | C | H |
| % calculé | 79,62 | 6,24 |
| % trouvé | 79,73 | 6,31 |

### Stade D : 2-(1-Méthoxy-7,8,9,10-tétrahydrocyclohepta[de]naphtalèn-7-yliden)acétonitrile

On procède comme dans le stade A de la Préparation 7.
Point de fusion : 96°C

| Microanalyse élémentaire : | | | |
|---|---|---|---|
| | C | H | N |
| % calculé | 81,90 | 6,06 | 5,62 |
| % trouvé | 81,88 | 6,18 | 5,64 |

### EXEMPLE 1 : N-[(4-Méthoxy-2,3-dihydro-1H-1-phénalényl)méthyl]acétamide

Dans un tricol de 100 ml, le chlorhydrate obtenu dans la Préparation 1 (300 mg, 1,14.10⁻³ mol, 1 éq.) est solubilisé dans un milieu biphasique CH₂Cl₂ / eau (17 ml / 17 ml) en présence de carbonate de sodium (854 mg, 7,96.10⁻³ mol, 7 éq.). L'anhydride acétique (110 µl, 1,17.10⁻³ mol, 1 éq.) est additionné à 0°C. Le milieu réactionnel est agité à température ambiante durant 20 minutes. Après séparation des phases, lavages de la phase organique avec une solution de NaHCO₃ saturée, eau, puis une solution de NaCl saturée, séchage sur MgSO₄, et enfin évaporation du solvant sous pression réduite, le résidu est chromatographié en chromatographie flash (1. CH₂Cl₂ ; 2. CH₂Cl₂ / MeOH : 99 / 1). Après recristallisation dans hexane / AcOEt, le composé du titre est isolé pur.
Point de fusion : 126°C

| Microanalyse élémentaire : | | | |
|---|---|---|---|
| | C | H | N |
| % calculé | 75,81 | 7,11 | 5,20 |
| % trouvé | 75,64 | 7,19 | 5,15 |

### EXEMPLE 2 : N-[(4-Méthoxy-2,3-dihydro-1H-phénalényl)méthyl]propionamide

On procède comme dans l'Exemple 1 en remplaçant l'anhydride acétique par l'anhydride propionique.
Point de fusion : 120°C

| Microanalyse élémentaire : | | | |
|---|---|---|---|
| | C | H | N |
| % calculé | 76,30 | 7,47 | 4,94 |
| % trouvé | 76,21 | 7,59 | 4,89 |

### EXEMPLE 3 : N-[(4-Méthoxy-2,3-dihydro-1H-1-phénalényl)méthyl]-cyclopropanecarboxamide

Le chlorhydrate obtenu dans la Préparation 1 (382 mg, 1,45.10⁻³ mol) est repris avec du dichlorométhane et traité avec de l'ammoniaque jusqu'à solubilisation du solide et obtention d'un pH basique pour la phase aqueuse. Après séparation des phases, l'aminé est séchée sur K₂CO₃. A 0°C, l'aminé (320 mg, 1,41.10⁻³ mol) est reprise dans du dichlorométhane anhydre (10 ml) en présence de triéthylamine sur potasse (295 µl, 2,12.10⁻³ mol, 1,5 éq.). Le chlorure de cyclopropionyle (130 µl, 1,43.10⁻³ mol, 1 éq.) est additionné goutte à goutte à 0°C. Le milieu réactionnel est agité à température ambiante durant 15 minutes. Après lavages à l'eau, séchage sur MgSO₄, puis évaporation du solvant sous pression réduite le résidu (400 mg) est chromatographié en chromatographie flash (1. CH₂Cl₂ ; 2. CH₂Cl₂ /MeOH : 99 / 1). Après recristallisation dans hexane / AcOEt, le produit du titre est isolé pur.
Point de fusion: 119°C

| Microanalyse élémentaire : | | | |
|---|---|---|---|
| | C | H | N |
| % calculé | 77,26 | 7,17 | 4,74 |
| % trouvé | 77,19 | 7,24 | 4,70 |

### EXEMPLE 4 : N-[(4-Méthoxy-2,3-dihydro-1H-1-phénalényl)méthyl]butanamide

On procède comme dans l'Exemple 1 en remplaçant l'anhydride acétique par l'anhydride butanoïque.
Point de fusion: 100°C

| Microanalyse élémentaire : | | | |
|---|---|---|---|
| | C | H | N |
| % calculé | 76,74 | 7,80 | 4,71 |
| % trouvé | 76,65 | 7,89 | 4,67 |

### EXEMPLE 5 : N-[(4-Méthoxy-2,3-dihydro-1H-1-phénalényl)méthyl]-N-méthyl-1-cyclopropanecarboxamide

Le composé obtenu dans l'Exemple 3 est mis en réaction en présence de NaH (1,5 éq) et de diméthylsulfate (1,2 éq). La réaction est suivie en CCM. Lorsque tout le produit de départ a disparu, le milieu réactionnel est hydrolysé puis extrait. Après évaporation des solvants, le produit du titre est isolé par chromatographie flash.

### EXEMPLE 6 : N-[(4-Hydroxy-2,3-dihydro-1H-1-phénalényl)méthyl]-N-méthylcyclopropanecarboxamide

Le composé obtenu dans l'Exemple 3 est soumis à une déméthylation en présence d'un agent classique comme BBr₃ par exemple.

### EXEMPLE 7 : N-[(4-Benzyloxy-2,3-dihydro-1H-1-phénalényl)méthyl] cyclopropanecarboxamide

Le composé obtenu dans l'Exemple 6 est mis en réaction dans un système basique en présence de chlorure de benzyle.

### EXEMPLE 8 : N-[(4-Allyloxy-2,3-dihydro-1H-1-phénalényl)méthyl] cyclopropanecarboxamide

On procède comme dans l'Exemple 7 en remplaçant le chlorure de benzyle par du chlorure d'allyle.

### EXEMPLE 9 : N-Cyclobutyl-N'-[(4-méthoxy-2,3-dihydro-1H-1-phénalényl)méthyl] urée

Le composé de la Préparation 1 est mis en suspension dans de la pyridine puis l'isocyanate de cyclobutyle est ajouté goutte à goutte, et le milieu réactionnel est chauffé. Lorsque la réaction est terminée, le milieu réactionnel est versé sur de l'eau glacée et acidifié par une solution d'acide chlorhydrique 1N. Après traitement classique, le composé du titre est isolé pur.

### EXEMPLE 10 : N-(2,3-Dihydro-1H-1-phénalénylméthyl)-2-iodoacétamide

On procède comme dans l'Exemple 1 en condensant l'anhydride 1-iodoacétique sur le composé obtenu dans la Préparation 2.

### EXEMPLE 11 : N-[(4-Méthoxy-2,3-dihydro-1H-1-phénalényl)méthyl]benzamide

On procède comme dans l'Exemple 1 en remplaçant l'anhydride acétique par l'anhydride benzoïque.

### EXEMPLE 12 : N-[(4,9-Diméthoxy-2,3-dihydro-1H-1-phénalényl)méthyl]hexanamide

On procède comme dans l'Exemple 1 en condensant l'anhydride hexanoïque sur le composé obtenu dans la Préparation 3.

### EXEMPLE 13 : N-[(4-Ethyl-2,3-dihydro-1H-1-phénalényl)méthyl] cyclohexanecarboxamide

On procède comme dans l'Exemple 1 en condensant l'anhydride cyclohexane carboxylique sur le composé obtenu dans la Préparation 4.

### EXEMPLE 14 : N-[(4-Chloro-2,3-dihydro-1H-1-phénalényl)méthyl]heptanamide

On procède comme dans l'Exemple 1 en condensant l'anhydride heptanoïque sur le composé obtenu dans la Préparation 5.

### EXEMPLE 15 : N-[(4-Chloro-2,3-dihydro-1H-1-phénalényl)méthyl]acétamide

On procède comme dans l'Exemple 1 à partir du composé obtenu dans la Préparation 5.

### EXEMPLE 16 : N-[(6-Méthoxy-1,3,4,5-tétrahydro-3-acénaphtylényl)méthyl]-1-cyclopropanecarboxamide

On procède comme dans l'Exemple 3 à partir du composé obtenu dans la Préparation 6.

### EXEMPLE 17 : N-[(6-Hydroxy-1,3,4,5-tétrahydro-3-acénaphtylényl)méthyl]-1-cyclopropanecarboxamide

On procède comme dans l'Exemple 6 à partir du composé obtenu dans l'Exemple 16.

### EXEMPLE 18 : N-[(6-Benzyloxy-1,3,4,5-tétrahydro-3-acénaphtylényl)méthyl]-1-cyclopropanecaboxamide

On procède comme dans l'Exemple 7 à partir du composé obtenu de l'exemple 17.

### EXEMPLE 19 : N-[2-(4-Méthoxy-2,3-dihydro-1H-1-phénalényl)éthyl]acétamide

Le nitrile obtenu dans le stade A de la Préparation 7 (440 mg, 1,85.10⁻³mol) dilué dans du tétrahydrofurane (12 ml) est hydrogéné à température ambiante en présence d'anhydride acétique (430 µl, 4,56.10⁻³mol, 2,5 éq.) et de nickel de Raney. Après 8 heures d'hydrogénation, le milieu réactionnel est filtré sur célite, rincé et évaporé sous pression réduite. Le résidu est ensuite repris dans du dichlorométhane et lavé à l'eau, puis avec une solution saturée de NaHCO₃, puis à l'eau. Après séchage sur MgSO₄ et évaporation du solvant, le résidu est purifié par chromatographie flash (CH₂Cl₂ / méthanol : 99 / 1).

| Microanalyse élémentaire : | | | |
|---|---|---|---|
| | C | H | N |
| % calculé + ¼ H₂O | 75,10 | 7,53 | 4,87 |
| % trouvé | 74,95 | 7,50 | 4,80 |

### EXEMPLE 20 : N-[2-(4-Méthoxy-2,3-dihydro-1H-1-phénalényl)éthyl]propanamide

Le nitrile obtenu dans le stade A de la Préparation 7 (500 mg, 2,11.10⁻³mol) dans du tétrahydrofurane (25 ml) est hydrogéné à température ambiante en présence d'anhydride propionique (500 µl, 3,90.10⁻³mol, 1,85 éq.) et de nickel de Raney. Après 30 heures d'hydrogénation, le milieu réactionnel est filtré sur célite, rincé et évaporé sous pression réduite. Le résidu est ensuite repris dans du dichlorométhane et lavé à l'eau, puis avec une solution saturée de NaHCO₃, puis à l'eau. Après séchage sur MgSO₄ et évaporation du solvant, le résidu de masse 650 mg est purifié par chromatographie flash (CH₂Cl₂ / méthanol : 99 / 1).

| Microanalyse élémentaire : | | | |
|---|---|---|---|
| | C | H | N |
| % calculé + ¼ H₂O | 75,59 | 7,85 | 4,64 |
| % trouvé | 75,54 | 7,88 | 4,61 |

### EXEMPLE 21 : N-[2-(4-Méthoxy-2,3-dihydro-1H-1-phénalényl)éthyl]-1-cyclopropanecarboxamide

Le chlorhydrate obtenu dans la Préparation 7 (350 mg, 1,26.10⁻³ mol) est repris dans un mélange dichlorométhane / eau (16 ml / 16 ml) en présence de carbonate de sodium (940 mg). A 0°C, le chlorure de cyclopropionyle (115 µl, 1,27.10⁻³ mol, 1 éq.) est additionné goutte à goutte. Le milieu réactionnel est agité à température ambiante durant 15 minutes. Une neutralisation est faite par ajout de quelques gouttes d'ammoniaque. Après lavages à l'eau, séchage sur MgSO₄, puis évaporation du solvant sous pression réduite, le résidu est purifié en chromatographie flash (1. CH₂Cl₂ ; 2. CH₂Cl₂ / MeOH : 99 / 1).
Point de fusion : 118°C

| Microanalyse élémentaire : | | | |
|---|---|---|---|
| | C | H | N |
| % calculé + ¼ H₂O | 76,52 | 7,55 | 4,64 |
| % trouvé | 76,55 | 7,51 | 4,42 |

### EXEMPLE 22 : N-[2-(4-Méthoxy-2,3-dihydro-1H-1-phénalényl)éthyl]-4-méthoxybenzamide

On procède comme dans l'Exemple 21 en remplaçant le chlorure de cyclopropionyle par le chlorure de 4-méthoxy-benzoyle.

### EXEMPLE 23 : N-[2-(4-Méthoxy-2,3-dihydro-1H-1-phénalényl)éthyl]-3-chlorobenzamide

On procède comme dans l'Exemple 22 en remplaçant le chlorure de cyclopropionyle par le chlorure de 3-chloro-benzoyle.

### EXEMPLE 24 : N-[2-(4-Méthoxy-2,3-dihydro-1H-1-phénalényl)éthyl]-propanethioamide

Le composé du titre est obtenu en soumettant le composé de l'Exemple 20 au réactif de Lawesson.

### EXEMPLE 25: N-[2-(4-Méthoxy-2,3-dibydro-1H-1-phénalényl)éthyl]-3-butenamide

On procède comme dans l'Exemple 21 en remplaçant le chlorure de propionyle par le chlorure de buténoyle.

### EXEMPLE 26 : N-[2-(4-Hydroxy-2,3-dihydro-1H-1-phénalényl)éthyl]acétamide

On procède comme dans l'Exemple 6 à partir du composé obtenu dans l'Exemple 19.

### EXEMPLE 27 : N-[2-(4-Cyclopropyloxy-2,3-dihydro-1H-1-phénalényl)éthyl] acétamide

On procède comme dans l'Exemple 7 en remplaçant le chlorure de benzyle par le chlorure de cyclopropyle.

### EXEMPLE 27 bis : N-[2-(6-Méthoxy-1,3,4,5-tétrahydro-3-acénaphtylényl)éthyl]-1-cyclobutanecarboxamide

On procède comme dans l'Exemple 19 en condensant le chlorure de cyclobutanoyle sur le composé obtenu dans la Préparation 8.

### EXEMPLE 28 : N-Cyclobutyl-2-(4-Méthoxy-2,3-dihydro-1H-1-phénalényl)éthyl] acétamide

Le produit du titre est obtenu par condensation de la N-cyclobutylamine sur l'acide obtenu dans la Préparation 9 après transformation en chlorure d'acide.

### EXEMPLE 29 : N-Propyl-2-(4-Méthoxy-2,3-dihydro-1H-1-phénalényl)acétamide

On procède comme dans l'Exemple 28 en remplaçant la N-cyclobutylamine par la N-propylamine.

### EXEMPLE 30 : N-Hexyl-2-(4-Chloro-2,3-dihydro-1H-1-phénalényl)éthyl]acétamide

On procède comme dans l'Exemple 28 en condensant la N-hexylamine sur le chlorure d'acide du composé obtenu dans la Préparation 10.

### EXEMPLE 31 : N-Hexyl-2-(6-Méthoxy-1,3,4,5-tétrahydro-3-acénaphtylényl) acétamide

On procède comme dans l'Exemple 30 à partir du composé obtenu dans la Préparation 11.

### EXEMPLE 32 : N-[3-(4-Méthoxy-2,3-dihydro-1H-1-phénalényl)propyl]pentanamide

On procède comme dans l'Exemple 1 à partir du composé obtenu dans la Préparation 12.

### EXEMPLE 33 : N-Méthyl-N-[3-(4-Méthoxy-2,3-dihydro-1H-1-phénalényl)propyl] pentanamide

On procède comme dans l'Exemple 5 à partir du composé obtenu dans l'Exemple 32.

### EXEMPLE 34 : N-[3-(4,9-Diméthoxy-2,3-dihydro-1H-1-phénalényl)propyl]acétamide

On procède comme dans l'Exemple 19 à partir du composé obtenu dans la Préparation 13.

### EXEMPLE 35 : N-Cyclopentyl-4-(4-méthoxy-2,3-dihydro-1H-1-phénalényl) butanamide

On procède comme dans l'Exemple 28 en condensant la N-Cyclopentylamine sur le chlorure d'acide du composé obtenu dans la Préparation 14.

### EXEMPLE 36 : N-Cyclopentyl-N-Méthyl-4-(4-méthoxy-2,3-dihydro-1H-1-phénalényl) butanamide

On procède comme dans l'Exemple 33 à partir du composé obtenu dans l'Exemple 35.

### EXEMPLE 37 : N-[2-(1,6-Diméthoxy-7,8,9,10-tétrahydrocyclohepta[de]naphtalen-7-yl) éthyl]acétamide

On procède comme dans l'Exemple 19 à partir du composé obtenu dans la Préparation 15.

### EXEMPLE 38 : N-[(1-Méthoxy-7,8,9,10-tétrahydrocyclohepta[de]naphtalen-7-yl) méthyl]acétamide

On procède comme dans l'Exemple 19 à partir du composé obtenu dans la Préparation 16.

### EXEMPLE 39 : N-(6,7,8,9-Tétrahydro-2-thiabenzo[cd]azulen-9-yl)méthyl]acétamide

On procède comme dans l'Exemple 1 à partir du composé obtenu dans la Préparation 17.

### EXEMPLE 40 : N-[(5-Méthoxy-6,7,8,9-tétrahydro-2-oxabenzo[cd]azulen-9-yl)méthyl] propionamide

On procède comme dans l'Exemple 2 à partir du composé obtenu dans la Préparation 18.

### EXEMPLE 41 : N-[(3-Méthoxy-2,3-dihydro-1H-1-phénalényl)méthyl]acétamide

On procède comme dans l'Exemple 1 à partir du composé obtenu dans la Préparation 19.

### EXEMPLE 42 : N-[3-(3,8-Diméthoxy-1,2-dihydro-1-acénaphtylényl)propyl]-2,2,2-trifluoroacétamide

On procède comme dans l'Exemple 32 en condensant le chlorure d'acétyle sur le composé obtenu dans la Préparation 20.

### EXEMPLE 43 : N-Pentyl-4-(3,8-diméthoxy-1,2-dihydro-1-acénaphtylényl) butanamide

On procède comme dans l'Exemple 35 en condensant la N-Pentylamine sur le chlorure d'acide du composé obtenu dans la Préparation 21.

### EXEMPLE 44 : N-[(4-Méthoxy-2,3-dihydro-1H-2-phénalényl)méthyl]acétamide

On procède comme dans l'Exemple 1 à partir du composé obtenu dans la Préparation 22.

### EXEMPLE 45 : N-[(4-Méthoxy-2,3-dihydro-1H-2-phénalényl)méthyl]-1-cyclobutanecarboxamide

On procède comme dans l'Exemple 1 en remplaçant l'anhydride acétique par l'anhydride cyclobutanecarboxylique à partir du composé obtenu dans la Préparation 22.

### EXEMPLE 46 : N-[(4-Hydroxy-2,3-dihydro-1H-2-phénalényl)méthyl]-1-cyclobutanecarboxamide

On procède comme dans l'Exemple 6 à partir du composé obtenu dans l'Exemple 45.

### EXEMPLE 47 : N-[(4-Benzyloxy-2,3-dihydro-1H-2-phénalényl)méthyl]-1-cyclobutanecarboxamide

On procède comme dans l'Exemple 7 à partir du composé obtenu dans l'Exemple 46.

### EXEMPLE 48 : N-[(4-Allyloxy-2,3-dihydro-1H-2-phénalényl)méthyl]-1-cyclobutanecarboxamide

On procède comme dans l'Exemple 8 à partir du composé obtenu dans l'Exemple 46.

### EXEMPLE 49 : N-[(4-Méthoxy-2,3-dihydro-1H-2-phénalényl)méthyl] éthanethioamide

On procède comme dans l'Exemple 5 à partir du composé obtenu dans l'Exemple 44.

### EXEMPLE 50 : N-[(4-Ethyl-2,3-dihydro-1H-2-phénalényl)méthyl]propanamide

On procède comme dans l'Exemple 2 à partir du composé obtenu dans la Préparation 23.

### EXEMPLE 51 : N-Cyclopropyl-2-(4,9-diméthoxy-2,3-dihydro-1H-2-phénalényl) acétamide

On procède comme dans l'Exemple 28 en condensant la N-cyclopropylamine sur le chlorure de l'acide obtenu dans la Préparation 24.

### EXEMPLE 52 : N-Méthyl-4-méthoxy-2,3-dihydro-1H-2-phénalènecarboxamide

On procède comme dans l'Exemple 28 en condensant la N-Méthylamine sur le chlorure de l'acide obtenu dans la Préparation 25.

### EXEMPLE 53 : N-[2-(4-Méthoxy-2,3-dihydro-1H-2-phénalényl)éthyl]heptanamide

On procède comme dans l'Exemple 38 à partir du composé obtenu dans la Préparation 26.

### EXEMPLE 54 : N-Méthyl-N-[2-(4-Méthoxy-2,3-dihydro-1H-2-phénalényl)éthyl] heptanamide

On procède comme dans l'Exemple 5 à partir du composé obtenu dans l'Exemple 96.

### EXEMPLE 55 : N-[3-(4-Méthoxy-2,3-dihydro-1H-2-phénalényl)propyl]acétamide

On procède comme dans l'Exemple 38 à partir du composé obtenu dans la Préparation 27.

### EXEMPLE 56 : N-[(6-Chloro-2,3-dihydro-1H-2-phénalényl)méthyl]acétamide

On procède comme dans l'Exemple 1 à partir du composé obtenu dans la Préparation 28.

### EXEMPLE 57 : N-[(1,6-Diméthoxy-7,8,9,10-tétrahydrocyclohepta[de]naphtalen-8-yl)méthyl]acétamide

On procède comme dans l'Exemple 1 à partir du composé obtenu dans la Préparation 29.

### EXEMPLE 58 : N-[(1,6-Diméthoxy-7,8,9,10-tétrahydrocyclohepta[de]naphtalen-8-yl)-méthyl]1-cyclopropanecarboxamide

On procède comme dans l'Exemple 3 à partir du composé obtenu dans la Préparation 29.

### EXEMPLE 59 : N-Ethyl-1,6-Diméthoxy-7,8,9,10-tétrahydrocyclohepta[de] naphtalen-8-carboxamide

On procède comme dans l'Exemple 28 en condensant la N-Ethylamine sur le chlorure de l'acide obtenu dans la Préparation 30.

### EXEMPLE 60 : N-(8-Méthoxy-1,2-dihydro-1-acénaphtylényl)acétamide

On procède comme dans l'Exemple 1 à partir du composé obtenu dans la Préparation 31.
Point de fusion : 217-219°C

Les composés des Exemples 61 et 62 sont obtenus par action de HBr sur le composé obtenu dans l'Exemple 41.

### EXEMPLE 61 : N-[(3-Hydroxy-2,3-dihydro-1H-1-phénalényl)méthyl]acétamide

### EXEMPLE 62 : N-[(3-Bromo-2,3-dihydro-1H-1-phénalényl)méthyl]acétamide

### EXEMPLE 63 : N-[(3-Oxo-2,3-dihydro-1H-1-phénalényl)méthyl]acétamide

Le composé du titre est obtenu par oxydation classique de l'alcool obtenu dans l'exemple 61.

### EXEMPLE 64 : N-[(4-Méthoxy-2,3-dihydro-1H-1-phénalényl)méthyl]butanamide

L'amine obtenue dans la Préparation 1 (400 mg, 1,76.10⁻³ mol, 1 éq.) est diluée dans du dichlorométhane anhydre (12 ml) en présence de triéthylamine (368 µl, 2,64.10⁻³ mol, 1,5 éq.). A 0°C, le chlorure de butanoyle (183 µl, 1,76.10⁻³ mol, 1 éq.) est ajouté lentement. Le milieu réactionnel est agité à température ambiante durant 40 minutes. Après séparation des phases, lavages de la phase organique avec une solution de NaHCO₃ saturée, eau, puis une solution de NaCl saturée et séchage sur MgSO₄, et enfin évaporation du solvant sous pression réduite, le résidu est purifié en chromatographie flash (1. CH₂Cl₂ ; 2. CH₂Cl₂ /MeOH : 99 / 1). Après recristallisation dans un mélange hexane / AcOEt, le composé du titre est obtenu sous la forme d'un solide blanc.
Point de fusion : 100°C

| Microanalyse élémentaire : | | | |
|---|---|---|---|
| | C | H | N |
| % calculé | 76,74 | 7,80 | 4,71 |
| % trouvé | 76,65 | 7,89 | 4,67 |

### EXEMPLE 65 : N-[2-(9-Méthoxy-2,3-dihydro-1H-1-phénalényl)éthyl]acétamide

Le nitrile obtenu dans la Préparation 32 (219 mg, 9,23.10⁻⁴ mol) dissous dans du tétrahydrofurane (22 ml) est hydrogéné à température ambiante en présence d'anhydride acétique (174 µl, 1,85.10⁻³ mol, 2 éq.) et de nickel de Raney. Après 4 heures d'hydrogénation, le milieu réactionnel est filtré sur célite, rincé et évaporé sous pression réduite. Le résidu est ensuite repris dans du dichlorométhane et lavé à l'eau, puis avec une solution saturée de NaHCO₃, puis à l'eau. Après séchage sur MgSO₄ et évaporation du solvant, le résidu est purifié en chromatographie flash (AcOEt / Ether de pétrole : 30 / 50) et recristallisé dans un mélange cyclohexane / AcOEt. Le produit du titre est isolé sous forme d'un solide blanc.
Point de fusion : 98°C

| Microanalyse élémentaire : | | | |
|---|---|---|---|
| | C | H | N |
| % calculé | 76,30 | 7,47 | 4,94 |
| % trouvé | 76,13 | 7,60 | 4,79 |

### EXEMPLE 66 : N-[2-(9-Méthoxy-2,3-dihydro-1H-1-phénalényl)éthyl]butanamide

On procède comme dans l'Exemple 65 en remplaçant l'anhydride acétique par l'anhydride butyrique. Le produit du titre est isolé sous forme d'une huile.

### EXEMPLE 67 : N-[2-(4-Méthoxy-2,3-dihydro-1H-1-phénalényl)éthyl]butanamide

Dans un tricol de 100 ml, le chlorhydrate obtenu dans la Préparation 7 (300 mg, 1,08.10⁻³ mol, 1 éq.) est solubilisé dans un milieu biphasique CH₂Cl₂ / eau (12 ml / 12 ml) en présence de carbonate de sodium (801 mg, 7,56.10⁻³ mol, 7 éq.). Le chlorure de butanoyle (112 µl, 1,08.10⁻³ mol, 1 éq.) est additionné à 0°C. Le milieu réactionnel est agité à température ambiante durant 20 minutes. Après séparation des phases, lavage de la phase organique avec une solution de NaHCO₃ saturée, H₂O, puis une solution de NaCl saturée, puis séchage sur MgSO₄, et enfin évaporation du solvant sous pression réduite, le résidu est purifié en chromatographie flash (1. CH₂Cl₂ ; 2. CH₂Cl₂ / MeOH : 99 / 1). Le produit du titre est isolé sous forme d'une huile.

### EXEMPLE 68 : N-[2-(4,9-Diméthoxy-2,3-dihydro-1H-1-phénalényl)éthyl] propanamide

On procède comme dans l'Exemple 65 en remplaçant l'anhydride acétique par l'anhydride propionique, à partir de l'amine obtenue dans la Préparation 33. Le produit du titre obtenu est isolé sous forme d'un solide blanc.
Point de fusion: 111°C

| Microanalyse élémentaire : | | | |
|---|---|---|---|
| | C | H | N |
| % calculé | 73,37 | 7,70 | 4,28 |
| % trouvé | 73,45 | 7,87 | 4,18 |

### EXEMPLE 69 : N-[2-(4,9-Diméthoxy-2,3-dihydro-1H-1-phénalényl)éthyl] butanamide

On procède comme dans l'Exemple 68 en remplaçant l'anhydride propionique par l'anhydride butyrique. Le produit du titre obtenu est isolé sous forme d'un solide blanc.
Point de fusion: 99°C

| Microanalyse élémentaire : | | | |
|---|---|---|---|
| | C | H | N |
| % calculé + ⅛ H₂O | 73,39 | 7,99 | 4,08 |
| % trouvé | 73,12 | 8,16 | 3,97 |

### EXEMPLE 70 : N-[2-(4,9-Diméthoxy-2,3-dihydro-1H-1-phénalényl)éthyl]-1-cyclopropane carboxamide

A 0°C, le chlorure de cyclopropanoyle (67 µl, 7,37.10⁻³ mol, 1 éq.) est additionné sur une solution de l'amine obtenue dans la Préparation 33 (200 mg, 7,37.10⁻⁴ mol, 1 éq.) dans un mélange dichlorométhane / eau (10 ml / 10 ml) en présence de carbonate de sodium (547 mg, 5,16.10⁻³ mol, 7 éq.). Le milieu réactionnel est agité à température ambiante durant 15 minutes. Une neutralisation est faite par ajout de quelques gouttes d'ammoniaque. Après lavage à l'eau, séchage sur MgSO₄, puis évaporation du solvant sous pression réduite, le résidu est purifié par chromatographie flash (AcOEt / Ether de pétrole 40 / 60). Après recristallisation dans un mélange cyclohexane / AcOEt, le produit du titre est isolé sous forme d'un solide blanc.
Point de fusion : 120°C

| Microanalyse élémentaire : | | | |
|---|---|---|---|
| | C | H | N |
| % calculé | 74,31 | 7,42 | 4,13 |
| % trouvé | 74,17 | 7,57 | 4,05 |

### EXEMPLE 71 : N-[2-(4,9-Diméthoxy-2,3-dihydro-1H-1-phénalényl)éthyl]acétamide

On procède comme dans l'Exemple 68 en remplaçant l'anhydride propionique par l'anhydride acétique. Le produit du titre est isolé sous forme d'un solide blanc.
Point de fusion: 125°C

| Microanalyse élémentaire : | | | |
|---|---|---|---|
| | C | H | N |
| % calculé | 72,82 | 7,40 | 4,47 |
| % trouvé | 72,68 | 7,60 | 4, 35 |

### EXEMPLE 72 : N-[2-(9-Méthoxy-2,3-dihydro-1H-1-phénalényl)méthyl]acétamide

Dans un tricol de 50 ml, l'amine obtenue dans la Préparation 34 (172 mg, 7,57.10⁻³ mol, 1 éq.) est solubilisée dans un milieu biphasique CH₂Cl₂ / eau (9 ml / 9 ml) en présence de carbonate de sodium (561 mg, 5,30.10⁻³ mol, 7 éq.). L'anhydride acétique. (72 µl, 7,57.10⁻³ mol, 1 éq.) est additionné à 0°C. Le milieu réactionnel est agité à température ambiante durant 20 minutes. Après séparation des phases, lavages de la phase organique avec une solution de NaHCO₃ saturée, H₂O, et une solution de NaCl saturée, puis séchage sur MgSO₄, et enfin évaporation du solvant sous pression réduite, le résidu (180 mg) est purifié en chromatographie flash (AcOEt / éther de pétrole, 40 / 60). Après recristallisation dans un mélange cyclohexane / AcOEt, le produit du titre est isolé sous forme d'un solide blanc.
Point de fusion : 192°C

| Microanalyse élémentaire : | | | |
|---|---|---|---|
| | C | H | N |
| % calculé | 75,81 | 7,11 | 5,20 |
| % trouvé | 75,43 | 7,35 | 5,12 |

### EXEMPLE 73 : N-[2-(9-Méthoxy-2,3-dihydro-1H-1-phénalényl)méthyl]butanamide

On procède comme dans l'Exemple 72 en remplaçant l'anhydride acétique par le chlorure de butanoyle. Le produit du titre est isolé sous forme d'un solide blanc. Point de fusion : 114°C

| Microanalyse élémentaire : | | | |
|---|---|---|---|
| | C | H | N |
| % calculé | 76,74 | 7,80 | 4,71 |
| % trouvé | 76,52 | 8,02 | 4,55 |

### EXEMPLE 74 : N-[2-(4,9-Diméthoxy-2,3-dihydro-1H-1-phénalényl)méthyl] acétamide

On procède comme dans l'Exemple 72 à partir de l'amine obtenue dans la Préparation 3. Le produit du titre est isolé sous forme d'un solide blanc.
Point de fusion : 184°C

| Microanalyse élémentaire : | | | |
|---|---|---|---|
| | C | H | N |
| % calculé | 72,22 | 7,07 | 4,68 |
| % trouvé | 71,85 | 7,30 | 4,52 |

### EXEMPLE 75 : N-[2-(4,9-Diméthoxy-2,3-dihydro-1H-1-phénalényl)méthyl] propanamide

On procède comme dans l'Exemple 74 en remplaçant l'anhydride acétique par le chlorure de propanoyle. Le produit du titre est isolé sous forme d'un solide blanc.
Point de fusion : 158°C

| Microanalyse élémentaire : | | | |
|---|---|---|---|
| | C | H | N |
| % calculé | 72,82 | 7,40 | 4,47 |
| % trouvé | 72,61 | 7,55 | 4,39 |

### EXEMPLE 76 : N-[2-(4,9-Diméthoxy-2,3-dihydro-1H-1-phénalényl)méthyl] butanamide

On procède comme dans l'Exemple 74 en remplaçant l'anhydride acétique par le chlorure de butanoyle. Le produit du titre est isolé sous forme d'un solide blanc.
Point de fusion: 140°C

| Microanalyse élémentaire : | | | |
|---|---|---|---|
| | C | H | N |
| % calculé | 73,37 | 7,70 | 4,23 |
| % trouvé | 73,15 | 7,80 | 4,14 |

Un dédoublement des deux énantiomères est réalisé sur colonne chirale :

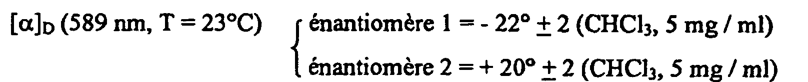

### EXEMPLE 77 : N-[2-(4,9-Diméthoxy-2,3-dihydro-1H-1-phénalényl)méthyl]-1-cyclopropane carboxamide

On procède comme dans l'Exemple 74 en remplaçant l'anhydride acétique par le chlorure de cyclopropanoyle. Le produit du titre est isolé sous forme d'un solide blanc.
Point de fusion : 192°C

| Microanalyse élémentaire : | | | |
|---|---|---|---|
| | C | H | N |
| % calculé | 73,82 | 7,12 | 4,30 |
| % trouvé | 73,62 | 7,27 | 4,18 |

### EXEMPLE 78 : (E)N-Méthyl-2-(4-méthoxy-2,3-dihydro-1H-1-phénalényliden) acétamide

L'acide obtenu dans la Préparation 35 (250 mg, 9,84.10⁻⁴ mol, 1 éq.) est solubilisé dans du dichlorométhane anhydre (20 ml). A 0 °C, sous argon, la triéthylamine sur potasse (164 µl, 1,18.10⁻³ mol, 1,2 éq.) est ajoutée, suivi du chloroformiate d'isobutyle (153 µl, 1,18.10⁻³ mol, 1,2 éq.). La formation complète de l'anhydride à partir de l'acide se fait à 0°C en 1 heure 10 minutes. Par ailleurs, le chlorhydrate de méthylamine (199 mg, 2,95.10⁻³ mol, 3 éq.) est agité sous argon avec du dichlorométhane anhydre (18 ml) et de la triéthylamine (411 µl, 2,95.10⁻³ mol, 3 éq.). Après 10 minutes d'agitation, cette suspension est ajoutée au mélange réactionnel et l'agitation est maintenue durant une nuit à température ambiante. Après un lavage à l'eau et séchage sur sulfate de magnésium, le filtrat est évaporé à sec sous pression réduite. Une purification sur colonne de gel de silice (dichlorométhane /méthanol, 98 / 2) suivie d'une recristallisation dans un mélange AcOEt / cyclohexane permet d'isoler le produit du titre sous forme d'un solide beige.
Point de fusion: 174°C

| Microanalyse élémentaire : | | | |
|---|---|---|---|
| | C | H | N |
| % calculé | 76,38 | 6,41 | 5,24 |
| % trouvé | 76,10 | 6,39 | 5,18 |

### EXEMPLE 79 : (Z)N-Méthyl-2-(4-méthoxy-2,3-dihydro-1H-1-phénalényliden) acétamide

On procède comme dans l'Exemple 78 à partir de l'acide obtenu dans la Préparation 36.
Point de fusion : 175°C

| Microanalyse élémentaire : | | | |
|---|---|---|---|
| | C | H | N |
| % calculé + ⅛ H₂O | 75,74 | 6,45 | 5,20 |
| % trouvé | 75,72 | 6,55 | 5,06 |

### EXEMPLE 80 : N-(1,2-Dihydro-1-acénaphtylénylméthyl)acétamide

Dans un tricol de 100 ml, le chlorhydrate obtenu dans la Préparation 37 (550 mg, 2,50.10⁻³ mol, 1 éq.) est solubilisé dans un milieu biphasique CH₂Cl₂ / eau (20 ml / 20 ml) en présence de carbonate de sodium (1,86 g, 1,75.10⁻² mol, 7 éq.). L'anhydride acétique (236 µl, 2,50.10⁻³ mol, 1 éq.) est additionné à 0°C. Le milieu réactionnel est agité à température ambiante durant 20 minutes. Après séparation des phases, lavages de la phase organique avec une solution de NaHCO₃ saturée, H₂O, et une solution de NaCl saturée, puis séchage sur MgSO₄, et enfin évaporation du solvant sous pression réduite, le résidu de masse 500 mg est purifié en chromatographie flash (1. CH₂Cl₂ ; 2. CH₂Cl₂ / MeOH : 99 / 1).

Après recristallisation dans un mélange cyclohexane / AcOEt, le produit du titre est isolé sous forme d'un solide blanc.
Point de fusion : 145°C

| Microanalyse élémentaire : | | | |
|---|---|---|---|
| | C | H | N |
| % calculé | 79,97 | 6,71 | 6,22 |
| % trouvé | 79,83 | 6,82 | 6,15 |

### EXEMPLE 81 : N-(1,2-Dihydro-1-acénaphtylénylméthyl)propanamide

On procède comme dans l'Exemple 80 en remplaçant l'anhydride acétique par l'anhydride propionique. Le produit du titre est isolé sous forme d'un solide blanc.
Point de fusion: 111°C

| Microanalyse élémentaire : | | | |
|---|---|---|---|
| | C | H | N |
| % calculé | 80,30 | 7,16 | 5,85 |
| % trouvé | 80,15 | 7,28 | 5,70 |

### EXEMPLE 82: N-(1,2-Dihydro-1-acénaphtylénylméthyl)butanamide

On procède comme dans l'Exemple 80 en remplaçant l'anhydride acétique par le chlorure de butanoyle. Le produit du titre est isolé sous forme d'un solide blanc.
Point de fusion : 111°C

| Microanalyse élémentaire : | | | |
|---|---|---|---|
| | C | H | N |
| % calculé | 80,57 | 7,56 | 5,53 |
| % trouvé | 80,02 | 7,70 | 5,40 |

### EXEMPLE 83 : N-(1,2-Dihydro-1-acénaphtylénylméthyl)-1-cyclopropane carboxamide

On procède comme dans l'Exemple 80 en remplaçant l'anhydride acétique par le chlorure de cyclopropanoyle.
Point de fusion : 146°C

| Microanalyse élémentaire : | | | |
|---|---|---|---|
| | C | H | N |
| % calculé | 81,24 | 6,82 | 5,57 |
| % trouvé | 81,13 | 6,88 | 5,52 |

### EXEMPLE 84 : N-(8-Méthoxy-1,2-dihydro-1-acénaphtylméthyl)acétamide

Dans un tricol de 100 ml, l'amine obtenue dans la Préparation 38 (385 mg, 1,81.10⁻³ mol, 1 éq.) est solubilisée dans un milieu biphasique CH₂Cl₂ / eau (20 ml / 20 ml) en présence de carbonate de sodium (1,34 g, 1,26.10⁻² mol, 7 éq.). L'anhydride acétique (170 µl, 1,81.10⁻³ mol, 1 éq.) est additionné à 0°C. Le milieu réactionnel est agité à température ambiante durant 40 minutes. Après séparation des phases, lavage de la phase organique avec une solution de NaHCO₃ saturée, H₂O, et une solution de NaCl saturée, puis séchage sur MgSO₄, et enfin évaporation du solvant sous pression réduite, le résidu de masse 360 mg est purifié en chromatographie flash (1. CH₂Cl₂; 2. CH₂Cl₂ / MeOH : 99/1). Après recristallisation dans un mélange cyclohexane / AcOEt, le produit du titre est isolé sous forme d'un solide blanc.
Point de fusion : 148°C

| Microanalyse élémentaire : | | | |
|---|---|---|---|
| | C | H | N |
| % calculé + ½ H₂O | 72,70 | 6,86 | 5,30 |
| % trouvé | 72,08 | 6,86 | 5,24 |

### EXEMPLE 85 : N-(8-Méthoxy-1,2-dihydro-1-acénaphtylméthyl)propanamide

On procède comme dans l'Exemple 84 en remplaçant l'anhydride acétique par l'anhydride propionique.
Point de fusion : 160°C

| Microanalyse élémentaire : | | | |
|---|---|---|---|
| | C | H | N |
| % calculé + ½ H₂O | 73,35 | 7,24 | 5,03 |
| % trouvé | 73,88 | 7,25 | 5,15 |

### EXEMPLE 86 : N-(8-Méthoxy-1,2-dihydro-1-acénaphtylméthyl)-1-cyclopropane carboxamide

On procède comme dans l'Exemple 84 en remplaçant l'anhydride acétique par le chlorure de cyclopropanoyle.
Point de fusion : 185°C

| Microanalyse élémentaire : | | | |
|---|---|---|---|
| | C | H | N |
| % calculé + ¼ H₂O | 75,63 | 6,88 | 4,90 |
| % trouvé | 75,78 | 6,98 | 4,93 |

### EXEMPLE 87 : N-(8-Méthoxy-1,2-dihydro-1-acénaphtylméthyl)butanamide

On procède comme dans l'Exemple 84 en remplaçant l'anhydride acétique par le chlorure de butanoyle. Le produit du titre est isolé sous forme d'un solide blanc.
Point de fusion : 146°C

| Microanalyse élémentaire : | | | |
|---|---|---|---|
| | C | H | N |
| % calculé | 76,30 | 7,47 | 4,94 |
| % trouvé | 76,12 | 7,51 | 4,93 |

Les deux énantiomères sont séparés sur colonne chirale :

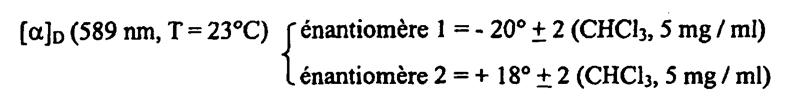

### EXEMPLE 88 : N-[2-(1,2-Dihydro-1-acénaphtyl)éthyl]acétamide

Le nitrile obtenu au stade B de la Préparation 39 (230 mg, 1,20.10⁻³ mol) dilué dans du tétrahydrofurane (25 ml) est hydrogéné à température ambiante en présence d'anhydride acétique (200 µl, 2,12.10⁻³ mol, 1,8 éq.) et de nickel de Raney. Après 5 heures d'hydrogénation, le milieu réactionnel est filtré sur célite, rincé et évaporé sous pression réduite. Le résidu est ensuite repris dans du dichlorométhane et lavé à l'eau, puis avec une solution saturée de NaHCO₃, puis à l'eau. Après séchage sur MgSO₄ et évaporation du solvant, le résidu est purifié par chromatographie flash (CH₂Cl₂ / méthanol : 99 / 1).
Point de fusion : 116°C

| Microanalyse élémentaire : | | | |
|---|---|---|---|
| | C | H | N |
| % calculé | 80,30 | 7,10 | 5,85 |
| % trouvé | 80,11 | 7,23 | 5,89 |

### EXEMPLE 89: N-[2-(1,2-Dihydro-1-acénaphtyl)éthyl]propanamide

On procède comme dans l'Exemple 88 en remplaçant l'anhydride acétique par l'anhydride propionique.
Point de fusion : 100°C

| Microanalyse élémentaire : | | | |
|---|---|---|---|
| | C | H | N |
| % calculé | 80,57 | 7,56 | 5,53 |
| % trouvé | 80,34 | 7,54 | 5,50 |

### EXEMPLE 90 :N-[2-(1,2-Dihydro-1-acénaphtyl)éthyl]butanamide

On procède comme dans l'Exemple 88 en remplaçant l'anhydride acétique par l'anhydride butyrique. Le produit du titre est isolé sous forme d'un solide blanc.
Point de fusion : 98°C

| Microanalyse élémentaire : | | | |
|---|---|---|---|
| | C | H | N |
| % calculé | 80,86 | 7,92 | 5,24 |
| % trouvé | 80,86 | 7,96 | 5,21 |

### EXEMPLE 91 : N-[2-(1,2-Dihydro-1-acénaphtyl)éthyl]cyclopropane carboxamide

A 0°C, sous argon, l'amine obtenue dans la Préparation 39 (500 mg, 2,53.10⁻³ mol, 1 éq.) est solubilisée dans du dichlorométhane anhydre (17 ml) en présence de triéthylamine (530 µl, 3,80.10⁻³ mol, 1,5 éq.). Le chlorure de cyclopropanoyle (230 µl, 2,53.10⁻³ mol, 1 éq.) est additionné goutte à goutte à 0°C. Le milieu réactionnel est agité à température ambiante durant 20 minutes. Après lavages à l'eau, avec une solution saturée de NaCl et séchage sur MgSO₄, puis évaporation du solvant sous pression réduite, le résidu est recristallisé dans un mélange cyclohexane / AcOEt.
Point de fusion : 159°C

### EXEMPLE 92 : N-[2-(8-Méthoxy-1,2-dihydro-1-acénaphtyl)éthyl]acétamide

On procède comme dans l'Exemple 88 à partir du nitrile obtenu au stade A de la Préparation 40. Le produit du titre est isolé sous forme d'un solide blanc.
Point de fusion : 118°C

| Microanalyse élémentaire : | | | |
|---|---|---|---|
| | C | H | N |
| % calculé | 80,30 | 7,10 | 5,85 |
| % trouvé | 80,11 | 7,23 | 5,89 |

### EXEMPLE 93 : N-[2-(8-Méthoxy-1,2-dihydro-1-acénaphtyl)éthyl]propanamide

On procède comme dans l'Exemple 92 en remplaçant l'anhydride acétique par l'anhydride propionique.
Point de fusion : 100°C

| Microanalyse élémentaire : | | | |
|---|---|---|---|
| | C | H | N |
| % calculé | 80,57 | 7,56 | 5,53 |
| % trouvé | 80,34 | 7,54 | 5,50 |

### EXEMPLE 94 : N-[2-(8-Méthoxy-1,2-dihydro-1-acénaphtyl)éthyl]butanamide

On procède comme dans l'Exemple 92 en remplaçant l'anhydride acétique par l'anhydride butyrique.
Point de fusion : 98°C

| Microanalyse élémentaire : | | | |
|---|---|---|---|
| | C | H | N |
| % calculé | 80,86 | 7,92 | 5,24 |
| % trouvé | 80,86 | 7,96 | 5,21 |

### EXEMPLE 95 : N-[2-(8-Méthoxy-1,2-dihydro-1-acénaphtyl)éthyl]-1-cyclopropane carboxamide

On procède comme dans l'Exemple 91 à partir de l'amine obtenue dans la Préparation 40.
Point de fusion : 159°C

### EXEMPLE 96 : N-[2-(1-Méthoxy-7,8,9,10-tétrahydrocyclohepta[de]naphtalèn-7-yliden)éthyl]propanamide

Le nitrile obtenu dans la Préparation 41 (465 mg, 1,87.10⁻³ mol) dilué dans du tétrahydrofurane (25 ml) est hydrogéné à température ambiante en présence d'anhydride propionique (480 µl, 3,74.10⁻³ mol, 2 éq.) et de nickel de Raney. Après 24 heures d'hydrogénation, le milieu réactionnel est filtré sur célite, rincé et évaporé sous pression réduite. Le résidu est ensuite repris dans du dichlorométhane et lavé à l'eau, puis avec une solution saturée de NaHCO₃, puis à l'eau. Après séchage sur MgSO₄ et évaporation du solvant, le résidu est purifié en chromatographie flash (1. CH₂Cl₂ ; 2. CH₂Cl₂ / MeOH : 99 / 1). Le produit du titre est isolé sous forme d'un solide blanc.

### Isomère E :

Après recristallisation du solide précédemment obtenu correspondant au mélange E / Z dans un mélange AcOEt / cyclohexane, l'isomère E est obtenu pur.
Point de fusion : 131 °C

| Microanalyse élémentaire : | | | |
|---|---|---|---|
| | C | H | N |
| % calculé | 77,64 | 7,49 | 4,53 |
| % trouvé | 77,28 | 7,54 | 4,36 |

### ETUDE PHARMACOLOGIQUE

### EXEMPLE A : Etude de la toxicité aiguë

La toxicité aiguë a été appréciée après administration orale à des lots de 8 souris (26 ± 2 grammes). Les animaux ont été observés à intervalles réguliers au cours de la première journée et quotidiennement pendant les deux semaines suivant le traitement. La DL 50, entraînant la mort de 50 % des animaux, a été évaluée et a montré la faible toxicité des composés de l'invention.

### EXEMPLE B : Etude de liaison aux récepteurs de la mélatonine sur des cellules de la pars tuberalis de mouton

Les études de liaison aux récepteurs de la mélatonine des composés de l'invention ont été réalisées selon les techniques classiques sur les cellules de la pars tuberalis de mouton. La pars tuberalis de l'adénohypophyse est en effet caractérisée, chez les mammifères, par une haute densité en récepteurs de la mélatonine (Journal of Neuroendocrinology, 1, pp 1-4, 1989).

### Protocole

1) Les membranes de pars tuberalis de mouton sont préparées et utilisées comme tissu cible dans des expériences de saturation pour déterminer les capacités et affinités de liaison pour la 2-[¹²⁵I] - iodomélatonine.
2) Les membranes de pars tuberalis de mouton sont utilisées comme tissu cible, avec les différents composés à tester, dans des expériences de liaison compétitive par rapport à la mélatonine.
   Chaque expérience est réalisée en triple et une gamme de concentrations différentes est testée pour chaque composé. Les résultats permettent de déterminer, après traitement statistique, les affinités de liaison du composé testé.

### Résultats

Il apparaît que les composés de l'invention possèdent une puissante affinité pour les récepteurs de la mélatonine.

### EXEMPLE C : Etude de liaison aux récepteurs mt₁ et MT₂ de la mélatonine

Les expériences de liaison aux récepteurs mt₁ ou MT₂ sont réalisées en utilisant la 2-[¹²⁵I]-mélatonine comme radioligand de référence. La radioactivité retenue est déterminée à l'aide d'un compteur à scintillation liquide Beckman® LS 6000.

Des expériences de liaison compétitive sont ensuite réalisées en triple, avec les différents composés à tester. Une gamme de concentrations différentes est testée pour chaque composé. Les résultats permettent de déterminer les affinités de liaison des composés testés (IC₅₀).

Ainsi, les valeurs d'IC₅₀ trouvées pour les composés de l'invention montrent que la liaison des composés testés est très puissante pour l'un ou l'autre des sous-types de récepteurs mt₁ ou MT₂, ces valeurs se situant dans un intervalle de 0,1 à 10nM.

### EXEMPLE D : Test des quatre plaques

Les produits de l'invention sont administrés par voie oesophagienne à des lots de dix souris. Un lot reçoit du sirop de gomme. Trente minutes après l'administration des produits à étudier, les animaux sont placés dans des habitacles dont le plancher comprend quatre plaques métalliques. Chaque fois que l'animal passe d'une plaque à l'autre, il reçoit une légère décharge électrique (0,35 mA). Le nombre de passages est enregistré pendant une minute. Après administration, les composés de l'invention augmentent de façon significative le nombre de passages ce qui montre l'activité anxiolytique des dérivés de l'invention.

### EXEMPLE E : Action des composés de l'invention sur les rythmes circadiens d'activité locomotrice du rat

L'implication de la mélatonine dans l'entraînement, par l'alternance jour/nuit, de la plupart des rythmes circadiens physiologiques, biochimiques et comportementaux a permis d'établir un modèle pharmacologique pour la recherche de ligands mélatoninergiques.

Les effets des molécules sont testés sur de nombreux paramètres et, en particulier, sur les rythmes circadiens d'activité locomotrice qui représentent un marqueur fiable de l'activité de l'horloge circadienne endogène.
Dans cette étude, on évalue les effets de telles molécules sur un modèle expérimental particulier, à savoir le rat placé en isolement temporel (obscurité permanente).

### Protocole expérimental

Des rats mâles Long Evans âgés de un mois sont soumis dès leur arrivée au laboratoire à un cycle lumineux de 12h de lumière par 24h (LD 12 : 12).
Après 2 à 3 semaines d'adaptation, ils sont placés dans des cages équipées d'une roue reliée à un système d'enregistrement afin de détecter les phases d'activité locomotrice et de suivre ainsi les rythmes nycthéméraux (LD) ou circadiens (DD).

Dès que les rythmes enregistrés témoignent d'un entraînement stable par le cycle lumineux LD 12 : 12, les rats sont mis en obscurité permanente (DD).

Deux à trois semaines plus tard, lorsque le libre-cours (rythme reflétant celui de l'horloge endogène) est clairement établi, les rats reçoivent une administration quotidienne de la molécule à tester.

Les observations sont réalisées grâce à la visualisation des rythmes d'activité :
- entraînement des rythmes d'activité par le rythme lumineux,
- disparition de l'entraînement des rythmes en obscurité permanente,
- entraînement par l'administration quotidienne de la molécule; effet transitoire ou durable.

Un logiciel permet :
- de mesurer la durée et l'intensité de l'activité, la période du rythme chez les animaux en libre cours et pendant le traitement,
- de mettre éventuellement en évidence par analyse spectrale l'existence de composants circadiens et non circadiens (ultradiens par exemple).

### Résultats :

Il apparaît clairement que les composés de l'invention permettent d'agir de façon puissante sur le rythme circadien *via* le système mélatoninergique.

### EXEMPLE F : Activité Antiarythmique

### Protocole

### (Ref : LAWSON J.W. et al. J. Pharmacol. Expert. Therap., 1968, 160, pp 22-31)

La substance testée est administrée par voie intrapéritonéale à un groupe de 3 souris 30 min avant l'exposition à une anesthésie par le chloroforme. Les animaux sont ensuite observés pendant 15 min. L'absence d'enregistrement d'arythmies et de fréquences cardiaques supérieures à 200 battements / min (témoin : 400-480 battements / min) chez deux animaux au moins indique une protection significative.

### EXEMPLE G : Composition pharmaceutique : Comprimés

| | |
|---|---|
| 1000 comprimés dosés à 5 mg de [(4-Méthoxy-2,3-dihydro-*1H*-phénalényl)méthyl] propionamide (Exemple 2) | 5 g |
| Amidon de blé | 20 g |
| Amidon de maïs | 20 g |
| Lactose | 30 g |
| Stéarate de magnésium | 2 g |
| Silice | 1 g |
| Hydroxypropylcellulose | 2 g |

## Revendications

1. Composés de formule (I) : 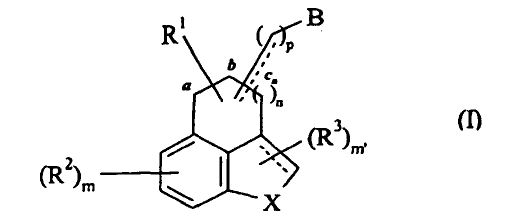 dans laquelle :
◆ R¹ représente un atome d'hydrogène, un atome d'halogène, un groupement alkyle (C₁-C₆) linéaire ou ramifié, alkoxy (C₁-C₆) linéaire ou ramifié, hydroxy, ou oxo,
◆ R² et R³, identiques ou différents, représentent, un atome d'halogène, un groupement Rₐ, ORₐ, CORₐ, OCORₐ ou COORₐ (avec Rₐ représentant un atome d'hydrogène, un groupement alkyle (C₁-C₆) linéaire ou ramifié éventuellement substitué, trihalogénoalkyle (C₁-C₆) linéaire ou ramifié, un groupement alcényle (C₂-C₆) linéaire ou ramifié éventuellement substitué, un groupement alcynyle (C₂-C₆) linéaire ou ramifié éventuellement substitué, un groupement cycloalkyle (C₃-C₈) éventuellement substitué, un groupement cycloalkyle (C₃-C₈) alkyle (C₁-C₆) linéaire ou ramifié éventuellement substitué, ou un groupement aryle éventuellement substitué),
◆ la représentation (R²)ₘ et (R³)_{m'} signifie que le cycle concerné peut être substitué par 1 à 3 groupements (identiques ou différents) appartenant aux définitions de R² et R³,
◆ X représente un groupement (CH₂)_{q} (où q vaut 1 ou 2) ou -CH=CH-,
◆ n est un entier tel que 0 ≤ n ≤ 3
◆ p est un entier tel que 1 ≤ p ≤ 3 lorsque n vaut 1, 2 ou 3 et que la chaîne 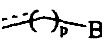 est en position b et tel que 0 ≤ p ≤ 3 dans tous les autres cas, la chaîne 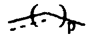 pouvant être substituée ou non par un ou plusieurs groupements, identiques ou différents, choisis parmi Rₐ, ORₐ, CORₐ, COORₐ ou atomes d'halogène,
◆ B représente :
- un groupement 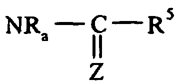 dans lequel Rₐ est tel que défini précédemment, Z représente un atome d'oxygène ou un atome de soufre, et R⁵ représente un groupement Rₐ ou un groupement NR⁶R⁷ dans lequel R⁶ et R⁷, identiques ou différents représentent un groupement Rₐ,
- ou un groupement 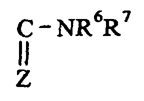 dans lequel Z, R⁶ et R⁷ sont tels que définis précédemment,
◆ la représentation ----- signifie que la liaison peut être simple ou double étant entendu que la valence des atomes est respectée,
étant entendu que par la représentation 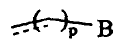 on entend les formules 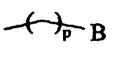
ou 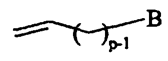 (et dans ce cas, p est différent de 0),
étant entendu que :
- le composé de formule (I) ne peut représenter le N-(4-méthyl-2,3-dihydro-*1H*-1-phénalényl)-1-cyclopropanecarboxamide, le N-(4-méthyl-2,3-dihydro-*1H*-1-phénalényl)-2-chloroacétamide, le N-(5-hydroxy-1,2,2a,3,4,5-hexahydro-4-acénaphtylényl)acétamide, le N-(5-hydroxy-1,2,2a,3,4,5-hexahydro-4-acénaphtylényl)benzamide ni le N-(1,2,2a,3,4,5-hexahydro-4-acénaphtylényl)acétamide,
étant entendu que :
- par "aryle" on entend un groupement phényle ou naphtyle éventuellement substitué par un ou plusieurs groupements, identiques ou différents, choisis parmi hydroxy, alkoxy (C₁-C₆) linéaire ou ramifié, alkyle (C₁-C₆) linéaire ou ramifié, cyano, nitro, amino, trihalogénoalkyle, ou atomes d'halogène,
- le terme "éventuellement substitué" affectant les expressions "alkyle", "alkényle", alcynyle", signifie que ces groupements sont substitués par un ou plusieurs groupements, identiques ou différents, choisis parmi hydroxy, alkoxy (C₁-C₆) linéaire ou ramifié, aryle, ou atomes d'halogène,
- le terme "éventuellement substitué" affectant les expressions "cycloalkyle" et "cycloalkyle alkyle" signifie que la partie cyclique est substituée par un ou plusieurs groupements, identiques ou différents, choisis parmi hydroxy, alkoxy (C₁-C₆) linéaire ou ramifié, oxo, ou atomes d'halogène,
leurs énantiomères et diastéréoisomères, ainsi que leurs sels d'addition à un acide ou une base pharmaceutiquement acceptable.

2. Composés de formule (I) selon la revendication 1 tels que n représente un entier égal à 0, 1 ou 2, leurs énantiomères et diastéréoisomères, ainsi que leurs sels d'addition à un acide ou une base pharmaceutiquement acceptable.

3. Composés de formule (I) selon la revendication 1 formant un système tricyclique 2,3-dihydrophénalène, 1,2-dihydroacénaphtylène ou 7,8,9,10-tétrahydrocyclohepta[*de*]naphtalène, leurs énantiomères et diastéréoisomères, ainsi que leurs sels d'addition à un acide ou une base pharmaceutiquement acceptable.

4. Composés de formule (I) selon la revendication 1 tels que p représente un entier égal à 0, 1 ou 2, leurs énantiomères et diastéréoisomères, ainsi que leurs sels d'addition à un acide ou une base pharmaceutiquement acceptable.

5. Composés de formule (I) selon la revendication 1 tels que R² et R³, identiques ou différents, représentent un groupement alkoxy, alkyle ou un atome d'hydrogène, leurs énantiomères et diastéréoisomères, ainsi que leurs sels d'addition à un acide ou une base pharmaceutiquement acceptable.

6. Composés de formule (I) selon la revendication 1 tels que R¹ représente un atome d'hydrogène, leurs énantiomères et diastéréoisomères, ainsi que leurs sels d'addition à un acide ou une base pharmaceutiquement acceptable.

7. Composés de formule (I) selon la revendication 1 tels que la chaîne 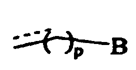 est en position a ou c, leurs énantiomères et diastéréoisomères, ainsi que leurs sels d'addition à un acide ou une base pharmaceutiquement acceptable.

8. Composés de formule (I) selon la revendication 1 tels que la chaîne 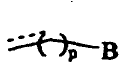 est en position a ou c et p représente un entier égal à 0 (et dans ce cas la liaison ----- est simple), 1 ou 2, leurs énantiomères et diastéréoisomères, ainsi que leurs sels d'addition à un acide ou une base pharmaceutiquement acceptable.

9. Composés de formule (I) selon la revendication 1 tels que B représente un groupement NHCOR⁵, leurs énantiomères et diastéréoisomères, ainsi que leurs sels d'addition à un acide ou une base pharmacéutiquement acceptable.

10. Composés de formule (I) selon la revendication 1 tels que B représente un groupement CONHR⁶, leurs énantiomères et diastéréoisomères, ainsi que leurs sels d'addition à un acide ou une base pharmaceutiquement acceptable.

11. Composés de formule (I) selon la revendication 1 formant un système tricyclique 2,3-dihydrophénalène, 1,2-dihydro acénaphtylène ou 7,8,9,10-tétrahydrocyclohepta[*de*]naphtalène, substitués ou non sur la partie naphtalénique par un où plusieurs groupements alkoxy ou alkyle, et substitués en a ou c par un groupement 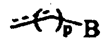 dans lequel B représente un groupement NHCOR⁵ ou CONHR⁶ , leurs énantiomères et diastéréoisomères, ainsi que leurs sels d'addition à un acide ou une base pharmaceutiquement acceptable.

12. Composés de formule (I) selon la revendication 1 formant un système tricyclique 1,2-dihydroacénaphtylène ou 7,8,9,10-tétrahydrocyclohepta[*de*]naphtalène, substitués ou non sur la partie naphtalènique par un ou deux groupements alkoxy et substitués en a ou c par un groupement =CH-B, =CH-CH₂-B, -B, -CH₂-B, -(CH₂)₂-B où B représente un groupement NHCOR⁵ ou CONHR⁶, leurs énantiomères et diastéréoisomères, ainsi que leurs sels d'addition à un acide ou une base pharmaceutiquement acceptable.

13. Composés de formule (I) selon la revendication 1 formant un système tricyclique 2,3-dihydrophénalène, substitués ou non sur la partie naphtalènique par un ou deux groupements alkoxy et substitués en a ou c par un groupement =CH-B, =CH-CH₂-B, -CH₂-B, -(CH₂)₂-B où B représente un groupement NHCOR⁵ ou CONHR⁶, leurs énantiomères et diastéréoisoméres, ainsi que leurs sels d'addition à un acide ou une base pharmaceutiquement acceptable.

14. Composés de formule (I) selon la revendication 1 qui sont : le N-[(4-Méthoxy-2,3-dihydro-*1H*-1-phénalényl)méthyl]acétamide, le N-[(4-Méthoxy-2,3-dihydro-*1H*-phénalényl)méthyl]propionamide, le N-[(4-Méthoxy-2,3-dibydro-*1H*-1-phénalényl) méthyl]-cyclopropanecarboxamide, le N-[(4-Méthoxy-2,3-dihydro-*1H*-1-phénalényl) méthyl]butanamide, le N-[(9-Méthoxy-2,3-dihydro-*1H*-1-phénalényl)méthyl] butanamide, le N-[2-(9-Méthoxy-2,3-dihydro-*1H*-1-phénalényl)méthyl]acétamide, le N-[2-(9-Méthoxy-2,3-dihydro-*1H*-1-phénalényl)méthyl]butanamide, le N-[2-(4,9-Diméthoxy-2,3-dihydro-*1H*-1-phénalényl)méthyl]acétamide, le N-[2-(4,9-Diméthoxy-2,3-dihydro-*1H*-1-phénalényl)méthyl]propanamide, le N-[2-(4,9-Diméthoxy-2,3-dihydro-*1H*-1-phénalényl)méthyl]butanamide, le N-[2-(4,9-Diméthoxy-2,3-dihydro-*1H*-1-phénalényl)méthyl]-1-cyclopropane carboxamide, leurs énantiomères et diastéréoisomères, ainsi que leurs sels d'addition à un acide ou une base pharmaceutiquement acceptable.

15. Composés de formule (I) selon la revendication 1 qui sont : le N-[2-(4-Méthoxy-2,3-dihydro-*1H*-1-phénalényl)éthyl]acétamide, le N-[2-(4-Méthoxy-2,3-dihydro-*1H*-1-phénalényl)éthyl]propanamide, le N-[2-(4-Méthoxy-2,3-dihydro-*1H*-1-phénalényl) éthyl]-1-cyclopropanecarboxamide, le N-[2-(9-Méthoxy-2,3-dihydro-*1H*-1-phénalényl)éthyl]acétamide, le N-[2-(9-Méthoxy-2,3-dihydro-*1H*-1-phénalényl)éthyl] butanamide, le N-[2-(4-Méthoxy-2,3-dihydro-*1H*-1-phénalényl)éthyl]butanamide, le N-[2-(4,9-Diméthoxy-2,3-dihydro-*1H*-1-phénalényl)éthyl]propanamide, le N-[2-(4,9-Diméthoxy-2,3-dihydro-*1H*-1-phénalényl)éthyl]butanamide, le N-[2-(4,9-Diméthoxy-2,3-dihydro-*1H*-1-phénalényl)éthyl]-1-cyclopropane carboxamide, le N-[2-(4,9-Diméthoxy-2,3-dihydro-*1H*-1-phénalényl)éthyl]acétamide, leurs énantiomères et diastéréoisomères, ainsi que leurs sels d'addition à un acide ou une base pharmaceutiquement acceptable.

16. Composé de formule (I) selon la revendication 1 qui est le N-(8-Méthoxy-1,2-dihydro-1-acénaphtylényl)acétamide, ses énantiomères et diastéréoisomères, ainsi que ses sels d'addition à un acide ou une base pharmaceutiquement acceptable.

17. Composés de formule (I) selon la revendication 1 qui sont : le *(E)*N-Méthyl-2-(4-méthoxy-2,3-dihydro-*1H*-1-phénalényliden)acétamide, le *(Z)*N-Méthyl-2-(4-méthoxy-2,3-dihydro-*1H*-1-phénalényliden)acétamide, leurs énantiomères et diastéréoisomères, ainsi que leurs sels d'addition à un acide ou une base pharmaceutiquement acceptable.

18. Composés de formule (I) selon la revendication 1 qui sont: le N-(1,2-Dihydro-1-acénaphtylénylméthyl)acétamide, le N-(1,2-Dihydro-1-acénaphtylénylméthyl) propanamide, le N-(1,2-Dihydro-1-acénaphtylénylméthyl)butanamide, le N-(1,2-Dihydro-1-acénaphtylénylméthyl)-1-cyclopropane carboxamide, le N-(8-Méthoxy-1,2-dihydro-1-acénaphtylméthyl)acétainide, le N-(8-Méthoxy-1,2-dihydro-1-acénaphtylméthyl)propanamide, le N-(8-Méthoxy-1,2-dihydro-1-acénaphtylméthyl)-1-cyclopropane carboxamide, le N-(8-Méthoxy-1,2-dihydro-1-acénaphtyl méthyl)butanamide, leurs énantiomères et diastéréoisomères, ainsi que leurs sels d'addition à un acide ou une base pharmaceutiquement acceptable.

19. Composés de formule (I) selon la revendication 1 qui sont : le N-[2-(1,2-Dihydro-1-acénaphtyl)éthyl]acétamide, le N-[2-(1,2-Dihydro-1-acénaphtyl)éthyl]propanamide, le N-[2-(1,2-Dihydro-1-acénaphtyl)éthyl]butanamide, le N-[2-(1,2-Dihydro-1-acénaphtyl)éthyl]cyclopropane carboxamide, le N-[2-(8-Méthoxy-1,2-dihydro-1-acénaphtyl)éthyl]acétamide, le N-[2-(8-Méthoxy-1,2-dihydro-1-acénaphtyl) éthyl]propanamide, le N-[2-(8-Méthoxy-1,2-dihydro-1-acénaphtyl)éthyl]butanamide, le N-[2-(8-Méthoxy-1,2-dihydro-1-acénaphtyl)éthyl]-1-cyclopropane carboxamide, leurs énantiomères et diastéréoisomères, ainsi que leurs sels d'addition à un acide ou une base pharmaceutiquement acceptable.

20. Composé de formule (I) selon la revendication 1 qui est le N-[2-(1-Méthoxy-7,8,9,10-tétrahydrocyclohepta[*de*]naphtalèn-7-yliden)éthyl]propanamide, ses énantiomères et diastéréoisomères, ainsi que ses sels d'addition à un acide ou une base pharmaceutiquement acceptable.

21. Procédé de préparation des composés de formule (I) selon la revendication 1 **caractérisé en ce que** l'on utilise comme produit de départ le composé de formule (II) : 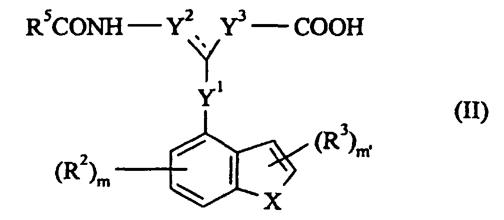 dans laquelle R², R³, R⁵, X, m, m' et la représentation ----- sont tels que définis précédemment,
Y² représente un groupement (CH₂)_{q} (où q vaut 1, 2 ou 3, ou q vaut 0 lorsque la représentation ----- est une liaison simple),
Y¹ représente un groupement (CH₂)_{q'} (où q' vaut 0, 1, 2 ou 3), substitué par un groupement R' tel que défini précédemment,
Y³ représente un groupement (CH₂)_{q"} (où q" vaut 0, 1, 2 ou 3), substitué par un groupement R¹ tel que défini précédemment avec q'+q" ≤ 3 et R¹ représente obligatoirement un atome d'hydrogène dans au moins un des deux groupements Y' et Y³,
qui est cyclisé en milieu basique pour conduire au composé de formule (III) : 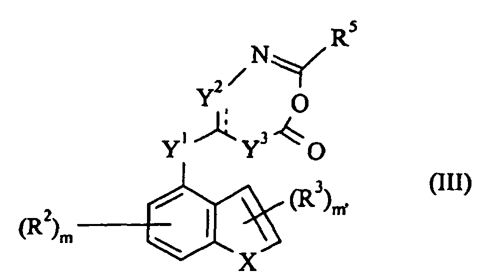 dans laquelle R², R³, R⁵, X, Y¹, Y², Y³, m, m' et la représentation ----- sont tels que définis précédemment,
qui est ensuite mis en réaction avec un acide de Lewis pour obtenir un composé de formule (I/a), cas particulier des composés de formule (I) : 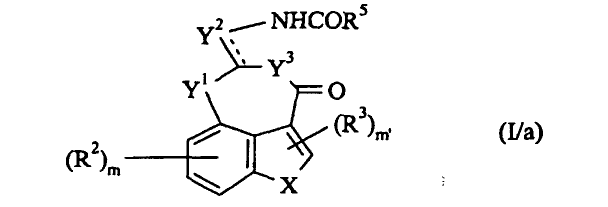 dans laquelle R², R³, R⁵, X, Y¹, Y², Y³, m, m'et la représentation ----- sont définis comme précédemment,
qui est alors réduit pour obtenir le composé de formule (I/b) cas particulier des composés de formule (I) : 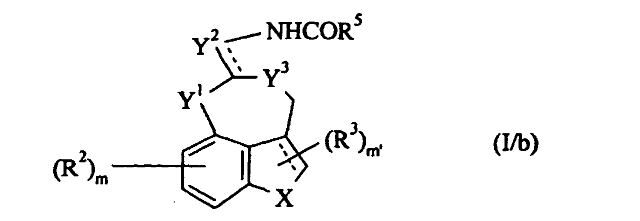 dans laquelle R², R³, R⁵, X, Y¹, Y², Y³, m, m' et la représentation ----- ont la même définition que précédemment,
◆ ou le composé de formule (IV) : 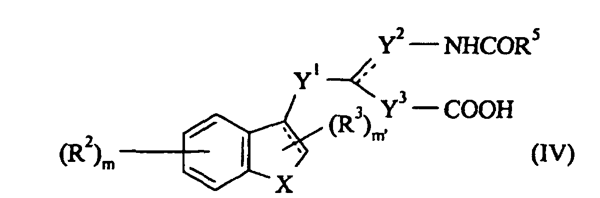
dans laquelle R², R³, R⁵, X, Y¹, Y², Y³, m, m' et la représentation ----- sont tels que définis précédemment,
qui est successivement
- cyclisé
- mis en réaction avec un acide de Lewis
pour conduire au composé de formule (I/c), cas particulier des composés de formule (I) : 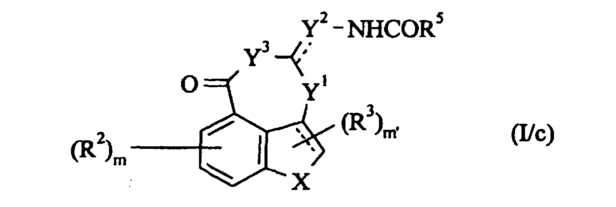 dans laquelle R², R³, R⁵, X, Y¹, Y², Y³, m, m' et la représentation ----- sont définis comme précédemment, qui est réduit pour obtenir le composé de formule (I/d), cas particulier des composés de formule (I) : 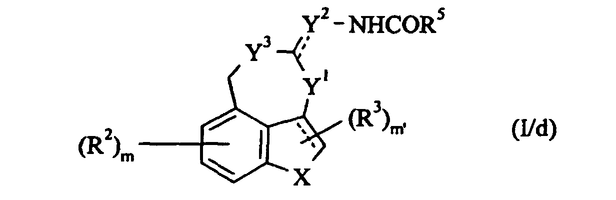 dans laquelle R², R³, R⁵, X, Y¹, Y², Y³, m, m' et la représentation ----- sont définis comme précédemment,
l'ensemble des composés (I/a), (I/b), (I/c) et (I/d) formant le composé de formule (I/e), cas particulier des composés de formule (I) : 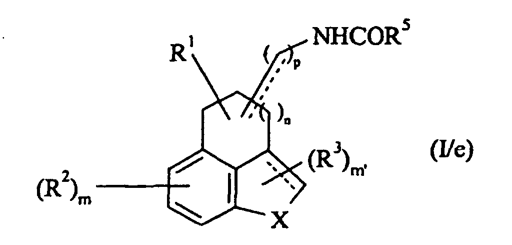 dans laquelle R¹, R², R³, R⁵, n, p, X, m,m' et la représentation ----- sont tels que définis précédemment,
qui est :
• soit soumis à l'action d'un composé de formule (V) : R'ₐ-W (V) dans laquelle R'ₐ peut prendre toutes les valeurs du groupement Rₐ tel que défini précédemment à l'exception de l'atome d'hydrogène, et W représente un groupe partant comme un atome d'halogène ou un groupement tosyle pour conduire au composé de formule (I/f), cas particulier des composés de formule (I) : 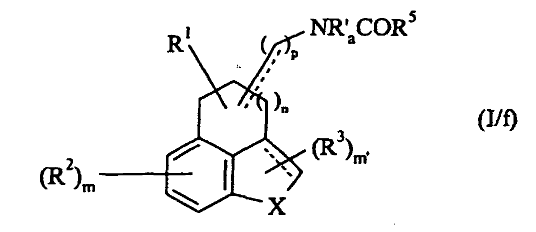
dans laquelle R¹, R², R³, R⁵, R'ₐ, n, p, X, m, m' et la représentation ----- ont la même définition que précédemment,
l'ensemble des composés de formule (I/e) et (I/f) formant le composé de formule (I/g) : 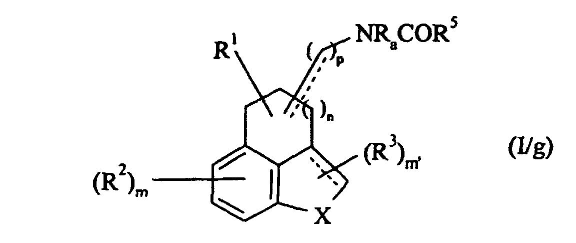 dans laquelle R¹, R², R³, R⁵, Rₐ, n, p, X, m, m' et la représentation ----- sont tels que définis précédemment,
qui peut être soumis à un agent de thionation comme le réactif de Lawesson pour obtenir le composé de formule (I/h), cas particulier des composés de formule (I) : 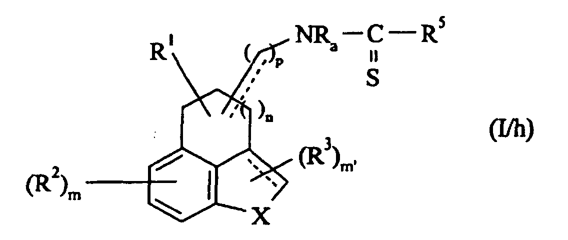 dans laquelle R¹, R², R³, R⁵, Rₐ, n, p, X, m, m' et la représentation ----- sont définis comme précédemment,
• soit hydrolysé en milieu basique afin de conduire au composé de formule (VI) : 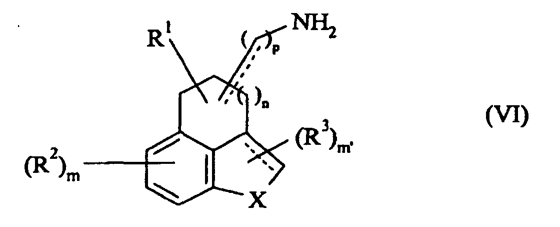
dans laquelle R¹, R², R³, n, p, X, m, m' et la représentation ----- sont tels que définis précédemment,
qui est :
- soit soumis à l'action d'un sel de pyrylium pour conduire au composé de formule (VII) : 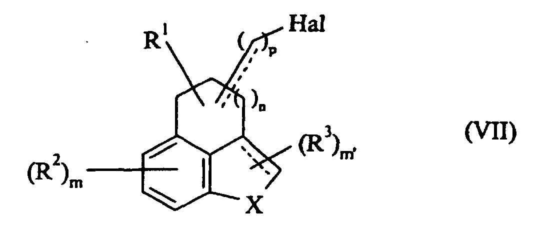
dans laquelle Hal représente un atome d'halogène et R¹, R², R³, n, p, X, m, m' et la représentation ----- sont définis de la même façon que précédemment, sur lequel on condense un sel de cyanure afin d'obtenir le composé de formule (VIII) : 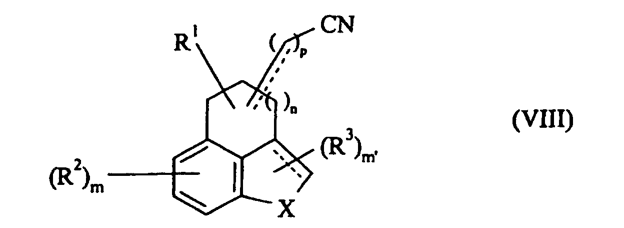 dans laquelle R¹, R², R³, n, p, X, m, m' et la représentation ----- sont tels que définis précédemment,
qui est hydrolysé en milieu acide ou basique pour conduire au composé de formule (IX) : 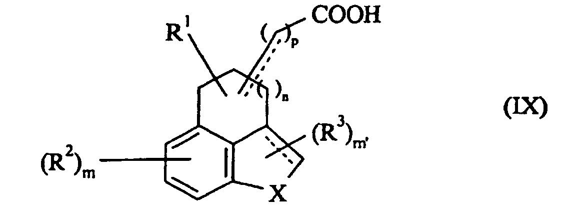 dans laquelle R¹, R², R³, n, p, X, m, m' et la représentation ----- sont définis comme précédemment,
qui est soumis, après activation sous forme de chlorure d'acide ou en présence d'agent de couplage, à l'action d'une amine HNR⁶R⁷ pour conduire au composé de formule (I/i), cas particulier des composés de formule (I) : 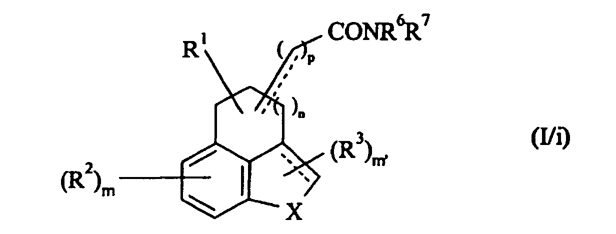 dans laquelle R¹, R², R³, R⁶, R⁷, n, p, X, m, m' et la représentation ----- sont tels que définis précédemment,
qui peut être soumis à un agent de thionation comme le réactif de Lawesson pour obtenir le composé (I/j), cas particulier des composés de formule (I) : 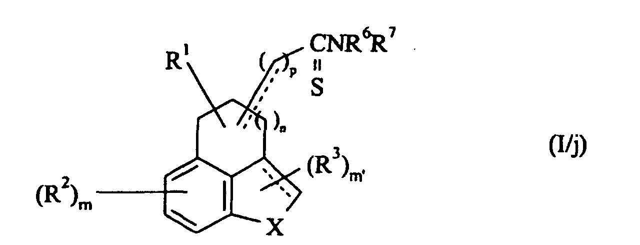 dans laquelle R¹, R², R³, R⁶, R⁷, n, p, X, m, m' et la représentation ----- sont tels que définis précédemment,
- soit soumis à l'action d'un composé de formule (X) :
Z=C=NR⁶R⁷ (X)
dans laquelle Z, R⁶ et R⁷ sont définis comme précédemment,
pour conduire au composé de formule (I/k), cas particulier des composés de formule (I) : 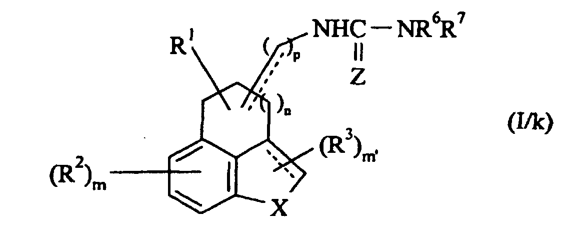 dans laquelle R¹, R², R³, R⁶, R⁷, n, p, Z, m, m' et la représentation ----- sont définis comme précédemment,
sur lequel on peut condenser un composé de formule (V) pour conduire au composé de formule (I/I), cas particulier des composés de formule (I) : 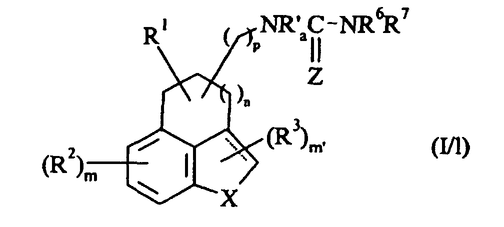 dans laquelle R¹, R², R³, R⁶, R⁷, R'ₐ, n, p, X, Z, m, m' et la représentation ----- sont tels que définis précédemment, les composés (I/a) à (I/l) pouvant être purifiés selon une technique classique de séparation, que l'on transforme si on le souhaite en leurs sels d'addition à un acide ou à une base pharmaceutiquement acceptable, et dont on sépare éventuellement les isomères selon une technique classique de séparation.

22. Procédé de préparation des composés de formule (I) selon la revendication 1
dans lesquels la chaîne 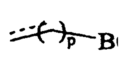 est en position a ou c, **caractérisé en ce que** l'on
utilise comme produit de départ le composé de formule (XIV) : 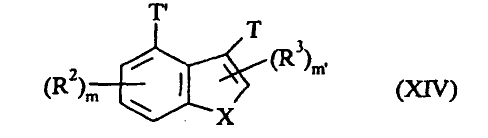 dans laquelle R², R³, X, m et m' sont tels que définis précédemment et T et T', différents, représentent un atome d'hydrogène ou un groupement -CHO,
que l'on soumet à une réaction de Wittig puis à une réduction catalytique pour obtenir le composé de formule (XV) : 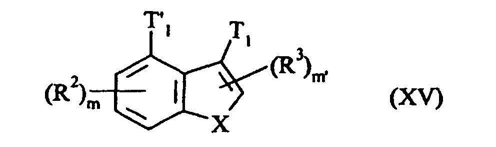 dans laquelle R², R³, X, m et m' sont définis comme précédemment et T'₁ et T₁, représentent un atome d'hydrogène ou un groupement de formule (XVI) : 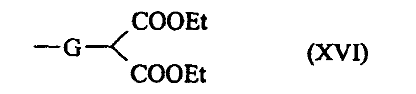 dans laquelle G représente un groupement (CH₂)_{n'} où n'=1, 2 ou 3 éventuellement substitué par un groupement R' défini comme précédemment, étant entendu qu'un des deux groupements T'₁ ou T₁ représente un atome d'hydrogène,
qui est successivement saponifié en milieu basique puis décarboxylé par chauffage pour conduire au composé de formule (XVII) : 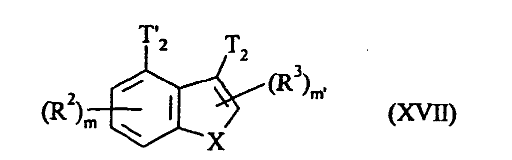 dans laquelle R², R³, X, m et m' sont définis comme précédemment et T'₂ et T₂ représentent un atome d'hydrogène ou un groupement de formule (XVIII) : 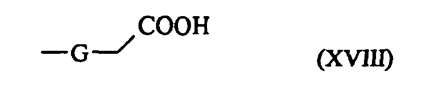 dans laquelle G est défini comme précédemment, étant entendu qu'un des deux groupements T'₂ ou T₂ représente un atome d'hydrogène,
qui est soumis à une cyclisation en présence d'un acide de Lewis après activation au chlorure d'oxalyle, pour conduire au composé de formule (XIX) : 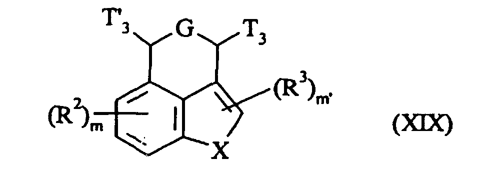 dans laquelle R², R³, X, G, m et m' sont tels que définis précédemment, et T'₃ et T₃, différents, représentent un atome d'hydrogène ou un groupement oxo,
que l'on soumet :
- soit à une réaction de Wittig (éventuellement suivie d'une réduction) puis à une saponification pour conduire au composé de formule (XX) : 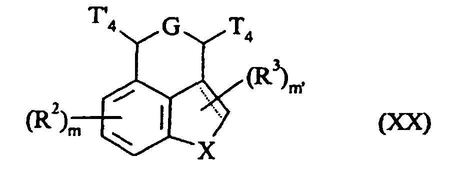
dans laquelle R², R³, X, G, m, m' et la représentation ----- sont définis comme précédemment, et T₄ et T'₄ représentent un atome d'hydrogène ou forment, avec l'atome de carbone qui les porte un groupement 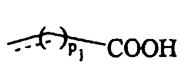 où p₁ vaut 1, 2 ou 3, étant entendu que l'un des deux groupements T₄ ou T'₄ représente un atome d'hydrogène,
- soit successivement
* à une réduction en alcool correspondant
* à une halogénation en présence de SOCl₂ par exemple
* à la condensation d'un sel de cyanure
* à une hydrolyse acide ou basique pour conduire au composé de formule (XXI) : 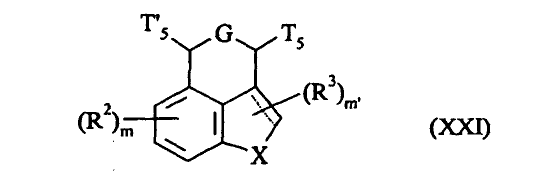
dans laquelle R², R³, X, G, m, m' et la représentation ----- sont définis comme précédemment, et T'₅ et T₅, différents, représentent un atome d'hydrogène ou un groupement COOH,
l'ensemble des composés (XX) et (XXI) formant le composé de formule (XXII) : 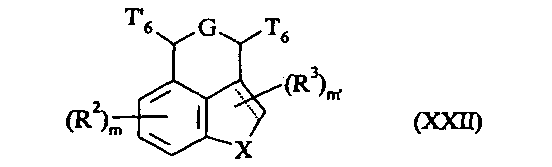 dans laquelle R², R³, X, G, m, m' et la représentation ----- sont définis comme précédemment, et T'₆ et T₆ représentent un atome d'hydrogène ou forment, avec l'atome de carbone qui les porte un groupement 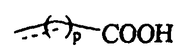 où p est défini comme précédemment, étant entendu qu'un des deux groupements T'₆ ou T₆ représente un atome d'hydrogène, le composé (XXII) pouvant être par ailleurs obtenu à partir du composé de formule (XIX) par condensation selon une réaction de Wittig, d'un composé contenant un nitrile (puis réduction éventuelle de la double liaison), et hydrolyse du nitrile, qui est :
- soit soumis, après activation sous forme de chlorure d'acide ou en présence d'un agent de couplage, à l'action d'une amine HNR⁶R⁷ pour conduire au composé de formule (I/y), cas particulier des composés de formule (I) : 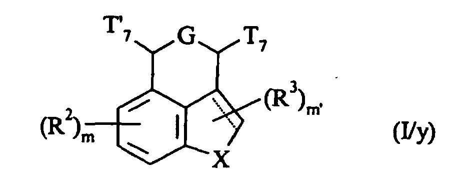
dans laquelle R², R³, X, G, m, m' et la représentation ----- sont définis comme précédemment, et T'₇ et T₇ représentent un atome d'hydrogène ou forment, avec l'atome de carbone qui les porte un groupement 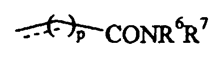 dans lequel p, R⁶ et R⁷ sont définis comme précédemment, étant entendu qu'un des deux groupements T'₇ ou T₇ représente un atome d'hydrogène,
qui peut être soumis à un agent de thionation comme le réactif de Lawesson pour obtenir le composé (I/z), cas particulier des composés de formule (I) : 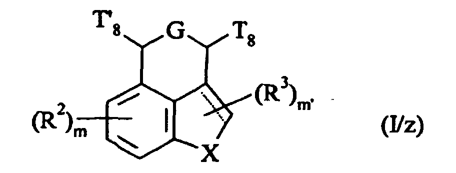 dans laquelle R², R³, X, G, m, m' et la représentation ----- sont définis comme précédemment, et T'₈ et T₈ représentent un atome d'hydrogène ou forment, avec l'atome de carbone qui les porte un groupement 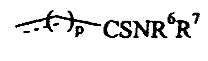 dans lequel p, R⁶ et R⁷ sont définis comme précédemment, étant entendu que l'un des deux groupements T₈ ou T'₈ représente un atome d'hydrogène,
- soit activé en chlorure d'acide, puis traité par un azoture, chauffé en isocyanate correspondant puis hydrolysé pour conduire au composé de formule (XXIII) : 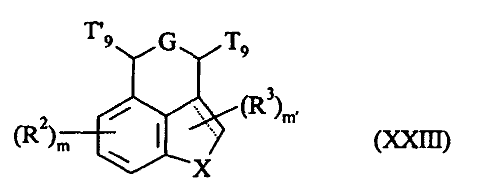
dans laquelle R², R³, X, G, m, m' et la représentation ----- sont tels que définis précédemment, et T'₉ et T₉ représentent un atome d'hydrogène ou forment, avec l'atome de carbone qui les porte un groupement 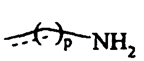 où p est tel que défini précédemment, étant entendu que l'un des deux groupements T₉ ou T'₉ représente un atome d'hydrogène,
le composé de formule (XXIII) pouvant être par ailleurs obtenu à partir du composé de formule (XIX) par condensation selon une réaction de Wittig d'un composé contenant un nitrile puis réduction du nitrile,
sur lequel on condense :
- soit un chlorure d'acyle ClCOR⁵ ou l'anhydride d'acide (mixte ou symétrique) correspondant pour lesquels R⁵ est tel que défini précédemment, pour conduire au composé de formule (I/aa), cas particulier des composés de formule (I) : 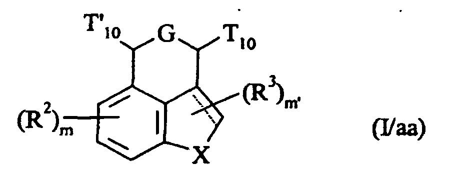
dans laquelle R², R³, X, G, m, m' et la représentation ----- sont définis comme précédemment, et T'₁₀ et T₁₀ représentent un atome d'hydrogène ou forment, avec l'atome de carbone qui les porte un groupement 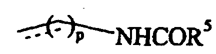 dans lequel p et R⁵ sont définis comme précédemment, étant entendu qu'un des deux groupements T'₁₀ ou T₁₀ représente un atome d'hydrogène,
qui peut être soumis à un agent de thionation comme le réactif de Lawesson, et/ou substitué après action d'un composé de formule (V) pour conduire au composé de formule (I/ab), cas particulier des composés de formule (I) : 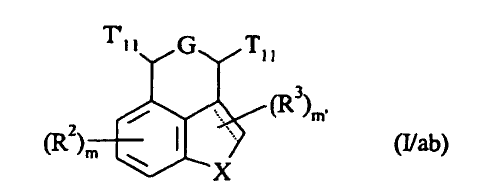 dans laquelle R², R³, X, G, m, m' et la représentation ---- sont tels que définis précédemment, et T'₁₁ et T₁₁ représentent un atome d'hydrogène ou forment, avec l'atome de carbone qui les porte un groupement 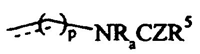 où p, Rₐ, R⁵ et Z sont définis comme précédemment, étant entendu qu'un des deux groupements T'₁₁ ou T₁₁ représente un atome d'hydrogène,
les composés (I/y) à (I/ab) pouvant être purifiés selon une technique classique de séparation, que l'on transforme, si on le souhaite en leurs sels d'addition à un acide ou à une base pharmaceutiquement acceptable, et dont on sépare éventuellement les isomères selon une technique classique de séparation.

23. Compositions pharmaceutiques contenant les produits de formule (I) selon l'une quelconque des revendications 1 à 20 ou un de leurs sels d'addition avec un acide ou une base pharmaceutiquement acceptable en combinaison avec un ou plusieurs excipients pharmaceutiquement acceptables.

24. Compositions pharmaceutiques selon la revendication 23 utiles pour le traitement des troubles liés au système mélatoninergique.

## Patentansprüche

1. Verbindungen der Formel (I): 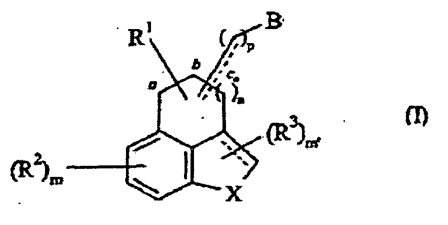 in der:
◆ R¹ ein Wasserstoffatom, ein Halogenatom, eine geradkettige oder verzweigte (C₁-C₆)-Alkylgruppe, geradkettige oder verzweigte (C₁-C₆)-Alkoxygruppe, Hydroxygruppe oder Oxogruppe darstellt,
◆ R² und R³, die gleichartig oder verschieden sind, ein Halogenatom, eine Gruppe Rₐ, ORₐ, CORₐ, OCORₐ oder COORₐ (worin Rₐ ein Wasserstoffatom, eine geradkettige oder verzweigte, gegebenenfalls substituierte (C₁-C₆)-Alkylgruppe, geradkettige oder verzweigte (C₁-C₆)-Trihalogenalkylgruppe, geradkettige oder verzweigte, gegebenenfalls substituierte (C₂-C₆)-Alkenylgruppe, eine geradkettige oder verzweigte, gegebenenfalls substituierte (C₂-C₆)-Alkinylgruppe, eine gegebenenfalls substituierte (C₃-C₈)-Cycloalkylgruppe, eine geradkettige oder verzweigte, gegebenenfalls substituierte (C₃-C₈)-Cycloalkyl-(C₁-C₆)-alkylgruppe oder eine gegebenenfalls substituierte Arylgruppe darstellt) bedeuten,
◆ das Symbol (R²)ₘ und (R³)_{m'} bedeutet, daß der fragliche Ring durch 1 bis 3 (gleichartige oder verschiedene) Gruppen mit den Bedeutungen von R² und R³ substituiert sein kann,
◆ X eine Gruppe (CH₂)_{q} (worin q 1 oder 2 darstellt) oder -CH=CH- darstellt,
◆ n eine ganze Zahl bedeutet, die die Bedingung 0 ≤ n ≤ 3 erfüllt,
◆ p eine ganze Zahl bedeutet, die die Bedingung 1 ≤ p ≤ 3 erfüllt, wenn n den Wert 1, 2 oder 3 darstellt und die Kette 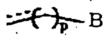 in der Position b steht, und in allen anderen Fällen die Bedingung 0 ≤ p ≤ 3 erfüllt,
wobei die Kette 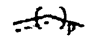 gegebenenfalls durch eine oder mehrere, gleichartige oder verschiedene Gruppen ausgewählt aus Rₐ, ORₐ, CORₐ, COORₐ oder Halogenatome substituiert sein kann,
◆ B:
- eine Gruppe 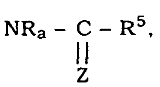 worin Rₐ die oben angegebenen Bedeutungen besitzt, Z ein Sauerstoffatom oder ein Schwefelatom darstellt und R⁵ eine Gruppe Rₐ oder eine Gruppe NR⁶R⁷ darstellt, worin R⁶ und R⁷, die gleichartig oder verschieden sind, eine Gruppe Rₐ bedeuten,
- oder eine Gruppe 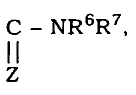 worin Z, R⁶ und R⁷ die oben angegebenen Bedeutungen besitzen, darstellt,
◆ das Symbol ----- bedeutet, daß die Bindung einfach oder doppelt sein kann, wobei es sich versteht, daß die Wertigkeit der Atome berücksichtigt wird,
wobei man unter dem Symbol 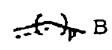 die Formeln 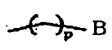 oder 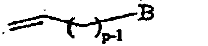 (wobei in diesem Fall p von 0 verschieden ist) versteht,
mit der Maßgabe, daß:
- die Verbindung der Formel (I) nicht N-(4-Methyl-2,3-dihydro-*1H*-1-phenalenyl)-1-cyclopropancarboxamid, N-(4-Methyl-2,3-dihydro-*1H-*1-phenalenyl)-2-chloracetamid, N-(5-Hydroxy-1,2,2a,3,4,5-hexahydro-4-acenaphthylenyl)-acetamid, N-(5-Hydroxy-1,2,2a,3,4,5-hexahydro-4-acenaphthylenyl)-benzamid noch N-(1,2,2a,3,4,5-Hexahydro-4-acenaphthylenyl)-acetamid bedeutet,
wobei:
- man unter "Aryl" eine Phenyl- oder Naphthylgruppe versteht, die gegebenenfalls durch eine oder mehrere, gleichartige oder verschiedene Gruppen ausgewählt aus Hydroxy, geradkettigem oder verzweigtem (C₁-C₆)-Alkoxy, geradkettigem oder verzweigtem (C₁-C₆)-Alkyl, Cyano, Nitro, Amino, Trihalogenalkyl oder Halogenatomen substituiert ist,
- der Begriff "gegebenenfalls substituiert" unter Bezug auf die Begriffe "Alkyl", "Alkenyl" und "Alkinyl" bedeutet, daß diese Gruppen durch eine oder mehrere, gleichartige oder verschiedenartige Gruppen ausgewählt aus Hydroxy, geradkettigem oder verzweigtem (C₁-C₆)-Alkoxy, Aryl oder Halogenatomen substituiert sind,
- der Begriff "gegebenenfalls substituiert" unter Bezug auf die Begriffe "Cycloalkyl" und "Cycloalkylalkyl" bedeutet, daß der cyclische Rest durch eine oder mehrere, gleichartige oder verschiedenartige Gruppen, ausgewählt aus Hydroxy, geradkettigem oder verzweigtem (C₁-C₆)-Alkoxy, Oxo oder Halogenatomen substituiert ist,
deren Enantiomere und Diastereoisomere sowie deren Additionssalze mit einer pharmazeutisch annehmbaren Säure oder Base.

2. Verbindungen der Formel (I) nach Anspruch 1, worin n eine ganze Zahl mit einem Wert von 0, 1 oder 2 bedeutet, deren Enantiomere und Diastereoisomere sowie deren Additionssalze mit einer pharmazeutisch annehmbaren Säure oder Base.

3. Verbindungen der Formel (I) nach Anspruch 1, die ein tricyclisches 2,3-Dihydrophenalen-, 1,2-Dihydroacenaphthylen- oder 7,8,9,10-Tetrahydrocyclohepta[*de*]naphthalin-System bildet, deren Enantiomere und Diastereoisomere sowie deren Additionssalze mit einer pharmazeutisch annehmbaren Säure oder Base.

4. Verbindungen der Formel (I) nach Anspruch 1, worin p eine ganze Zahl mit einem Wert von 0, 1 oder 2 bedeutet, deren Enantiomere und Diastereoisomere sowie deren Additionssalze mit einer pharmazeutisch annehmbaren Säure oder Base.

5. Verbindungen der Formel (I) nach Anspruch 1, worin R² und R³, die gleichartig oder verschieden sind, eine Alkoxygruppe, Alkylgruppe oder ein Wasserstoffatom bedeuten, deren Enantiomere und Diastereoisomere sowie deren Additionssalze mit einer pharmazeutisch annehmbaren Säure oder Base.

6. Verbindungen der Formel (I) nach Anspruch 1, worin R¹ ein Wasserstoffatom bedeutet, deren Enantiomere und Diastereoisomere sowie deren Additionssalze mit einer pharmazeutisch annehmbaren Säure oder Base.

7. Verbindungen der Formel (I) nach Anspruch 1, worin die Kette 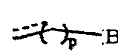 in der Position a oder c steht, deren Enantiomere und Diastereoisomere sowie deren Additionssalze mit einer pharmazeutisch annehmbaren Säure oder Base.

8. Verbindungen der Formel (I) nach Anspruch 1, worin die Kette 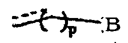 in der Position a oder c steht und p eine ganze Zahl mit einem Wert von 0 (wobei in diesem Fall die Bindung ----- einfach ist), 1 oder 2 bedeutet, deren Enantiomere und Diastereoisomere sowie deren Additionssalze mit einer pharmazeutisch annehmbaren Säure oder Base.

9. Verbindungen der Formel (I) nach Anspruch 1, worin B eine Gruppe NHCOR⁵ bedeutet, deren Enantiomere und Diastereoisomere sowie deren Additionssalze mit einer pharmazeutisch annehmbaren Säure oder Base.

10. Verbindungen der Formel (I) nach Anspruch 1, worin B eine Gruppe CONHR⁶ bedeutet. deren Enantiomere und Diastereoisomere sowie deren Additionssalze mit einer pharmazeutisch annehmbaren Säure oder Base.

11. Verbindungen der Formel (I) nach Anspruch 1, die ein tricyclisches 2,3-Dihydrophenalen-, 1,2-Dihydroacenaphthylen- oder 7,8,9,10-Tetrahydrocyclohepta[*de*]naphtalin-System bildet, das am Naphthalinrest gegebenenfalls durch eine oder mehrere Alkoxy- oder Alkylgruppen substituiert ist und in der Position a oder c durch eine Gruppe 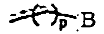 substituiert ist, in der B eine Gruppe NHCOR⁵ oder CONHR⁶ bedeutet, deren Enantiomere und Diastereoisomere sowie deren Additionssalze mit einer pharmazeutisch annehmbaren Säure oder Base.

12. Verbindungen der Formel (I) nach Anspruch 1, die ein tricyclisches 1,2-Dihydroacenaphthylen- oder 7,8,9,10-Tetrahydrocyclohepta[*de*]naphtalin - System bildet, welches gegebenenfalls am Naphthalinrest durch eine oder zwei Alkoxygruppen substituiert ist, und das in der Position a oder c durch eine Gruppe =CH-B, =CH-CH₂-B, -B, -CH₂-B, -(CH₂)₂-B substituiert ist, worin B eine Gruppe NHCOR⁵ oder CONHR⁶ bedeutet, deren Enantiomere und Diastereoisomere sowie deren Additionssalze mit einer pharmazeutisch annehmbaren Säure oder Base.

13. Verbindungen der Formel (I) nach Anspruch 1, die ein tricyclisches 2,3-Dihydrophenalen-System bildet, das gegebenenfalls am Naphthalinrest durch eine oder zwei Alkoxygruppen substituiert ist und in der Position a oder c durch eine Gruppe =CH-B, =CH-CH₂-B, -CH₂-B, -(CH₂)₂-B substituiert ist, worin B eine Gruppe NHCOR⁵ oder CONHR⁶ bedeutet, deren Enantiomere und Diastereoisomere sowie deren Additionssalze mit einer pharmazeutisch annehmbaren Säure oder Base.

14. Verbindungen der Formel (I) nach Anspruch 1, nämlich: N-[(4-Methoxy-2,3-dihydro-*1H*-1-phenalenyl)-methyl]-acetamid, N-[(4-Methoxy-2,3-dihydro-*1H*-phenalenyl)-methyl]-propionamid, N-[(4-Methoxy-2,3-dihydro-*1H*-1-phenalenyl)-methyl]-cyclopropancarboxamid, N-[(9-Methoxy-2,3-dihydro-*1H*-1-phenalenyl)-methyl]-butanamid, N-[(4-Methoxy-2,3-dihydro-*1H*-1-phenalenyl)-methyl]-butanamid, N-[2-(9-Methoxy-2,3-dihydro-*1H*-1-phenalenyl)-methyl]-acetamid, N-[2-(9-Methoxy-2,3-dihydro-*1H*-1-phenalenyl)-methyl]-butanamid, N-[2-(4,9-Dimethoxy-2,3-dihydro-*1H*-1-phenalenyl)-methyl]-acetamid, N-[2-(4,9-Dimethoxy-2,3-dihydro-*1H*-1-phenalenyl)-methyl]-propanamid, N-[2-(4,9-Dimethoxy-2,3-dihydro-*1H*-1-phenalenyl)-methyl]-butanamid, N-[2-(4,9-Dimethoxy-2,3-dihydro-*1H*-1-phenalenyl)-methyl]-cyclopropancarboxamid, deren Enantiomere und Diastereoisomere sowie deren Additionssalze mit einer pharmazeutisch annehmbaren Säure oder Base.

15. Verbindungen der Formel (I) nach Anspruch 1, nämlich: N-[2-(4-Methoxy-2,3-dihydro-*1H*-1-phenalenyl)-ethyl]-acetamid, N-[2-(4-Methoxy-2,3-dihydro-*1H*-1-phenalenyl)-ethyl]-propanamid, N-[2-(4-Methoxy-2,3-dihydro-*1H*-1-phenalenyl)-ethyl]-1-cyclopropancarboxamid, N-[2-(9-Methoxy-2,3-dihydro-*1H*-1-phenalenyl)-ethyl]-acetamid, N-[2-(9-Methoxy-2,3-dihydro-*1H*-1-phenalenyl)-ethyl]-butanamid. N-[2-(4-Methoxy-2,3-dihydro-*1H*-1-phenalenyl)-ethyl]-butanamid, N-[2-(4,9-Dimethoxy-2,3-dihydro-*1H*-1-phenalenyl)-ethyl]-propanamid, N-[2-(4,9-Dimethoxy-2,3-dihydro-*1H*-1-phenalenyl)-ethyl]-butanamid, N-[2-(4,9-Dimethoxy-2,3-dihydro-*1H*-1-phenalenyl)-ethyl]-cyclopropancarboxamid, N-[2-(4,9-Dimethoxy-2,3-dihydro-*1H*-1-phenalenyl)-ethyl]-acetamid, deren Enantiomere und Diastereoisomere sowie deren Additionssalze mit einer pharmazeutisch annehmbaren Säure oder Base.

16. Verbindung der Formel (I) nach Anspruch 1, nämlich N-(8-Methoxy-1,2-dihydro-1-acenaphthylenyl)-acetamid, dessen Enantiomere und Diastereoisomere sowie deren Additionssalze mit einer pharmazeutisch annehmbaren Säure oder Base.

17. Verbindungen der Formel (I) nach Anspruch 1, nämlich: (E)N-Methyl-2-(4-methoxy-2,3-dihydro-*1H*-1-phenalenyliden)-acetamid, (*Z*)N-Methyl-2-(4-methoxy-2,3-dihydro-*1H*-1-phenalenyliden)-acetamid, deren Enantiomere und Diastereoisomere sowie deren Additionssalze mit einer pharmazeutisch annehmbaren Säure oder Base.

18. Verbindungen der Formel (I) nach Anspruch 1, nämlich: N-(1,2-Dihydro-1-acenaphthylenylmethyl)-acetamid, N-(1,2-Dihydro-1-acenaphthylenylmethyl)-propanamid, N-(1,2-Dihydro-1-acenaphthylenylmethyl)-butanamid, N-( 1,2-Dihydro-1-acenaphthylenylmethyl)-1-cyclopropancarboxamid, N-(8-Methoxy-1,2-dihydro-1-acenaphthylmethyl)-acetamid, N-(8-Methoxy-1,2-dihydro-1-acenaphthylmethyl)-propanamid, N-(8-Methoxy-1,2-dihydro-1-acenaphthylmethyl)-1-cyclopropancarboxamid, N-(8-Methoxy-1,2-dihydro-1-acenaphthylmethyl)-butanamid, deren Enantiomere und Diastereoisomere sowie deren Additionssalze mit einer pharmazeutisch annehmbaren Säure oder Base.

19. Verbindungen der Formel (I) nach Anspruch 1, nämlich: N-[2-(1,2-Dihydro-1-acenaphthyl)-ethyl]-acetamid, N-[2-(1,2-Dihydro-1-acenaphthyl)-ethyl]-propanamid, N-[2-(1,2-Dihydro-1-acenaphthyl)-ethyl]-butanamid, N-[2-(1,2-Dihydro-1-acenaphthyl)-ethyl]-cyclopropancarboxamid, N-[2-(8-Methoxy-1,2-dihydro-1-acenaphthyl)-ethyl]-acetamid, N-[2-(8-Methoxy-1,2-dihydro-1-acenaphthyl)-ethyl]-propanamid, N-[2-(8-Methoxy-1,2-dihydro-1-acenaphthyl)-ethyl]-butanamid, N-[2-(8-Methoxy-1,2-dihydro-1-acenaphthyl)-ethyl]-1-cyclopropancarboxamid, deren Enantiomere und Diastereoisomere sowie deren Additionssalze mit einer pharmazeutisch annehmbaren Säure oder Base.

20. Verbindung der Formel (I) nach Anspruch 1, nämlich N-[2-(1-Methoxy-7,8,9,10-tetrahydrocyclohepta[*de*]naphthalin-7-yliden)-ethyl]-propanamid, dessen Enantiomere und Diastereoisomere sowie deren Additionssalze mit einer pharmazeutisch annehmbaren Säure oder Base.

21. Verfahren zur Herstellung der Verbindungen der Formel (I) nach Anspruch 1, **dadurch gekennzeichnet, daß** man als Ausgangsprodukt die Verbindung der Formel (II) verwendet: 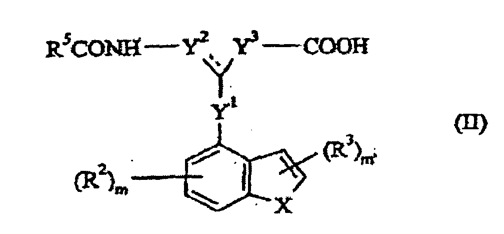 in der R², R³, R⁵, X, m, m' und das Symbol ----- die oben angegebenen Bedeutungen besitzen,
Y² eine Gruppe (CH₂)_{q} (worin q 1, 2 oder 3 bedeutet oder q 0 bedeutet, wenn das Symbol ----- für eine Einfachbindung steht), bedeutet,
Y¹ eine Gruppe (CH₂)_{q'} (worin q' 0, 1, 2 oder 3 bedeutet), die durch eine Gruppe R¹, wie sie oben definiert worden ist, substituiert ist, bedeutet,
Y³ eine Gruppe (CH₂)_{q''} (worin q" 0, 1, 2 oder 3 bedeutet), die durch eine Gruppe R¹, wie sie oben definiert worden ist, substituiert ist, worin q' + q" ≤ 3 und R¹ zwingend ein Wasserstoffatom in mindestens einer der beiden Gruppen Y¹ und Y³ darstellt, bedeutet,
welche man in basischem Medium cyclisiert zur Bildung der Verbindung der Formel (III): 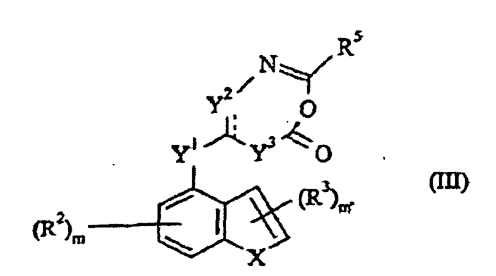 in der R², R³, R⁵, X, Y¹, Y², Y³, m, m' und das Symbol ----- die oben angegebenen Bedeutungen besitzen,
welche man anschließend mit einer Lewis-Säure umsetzt zur Bildung einer Verbindung der Formel (I/a), einem Sonderfall der Verbindungen der Formel (I): 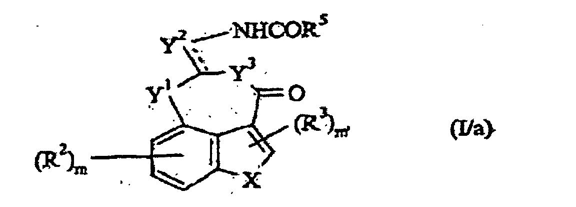 in der R², R³, R⁵, X, Y¹, Y², Y³, m, m' und das Symbol ----- die oben angegebenen Bedeutungen besitzen,
welche man anschließend reduziert zur Bildung der Verbindung der Formel (I/b), einem Sonderfall der Verbindungen der Formel (I): 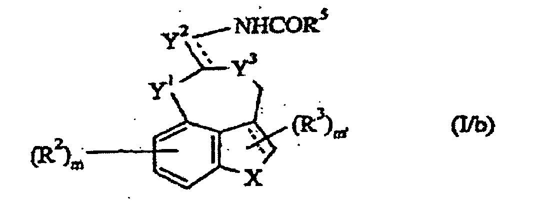 in der R², R³, R⁵, X, Y¹, Y², Y³, m, m' und das Symbol ----- die oben angegebenen Bedeutungen besitzen,
◆ oder man die Verbindung der Formel (IV): 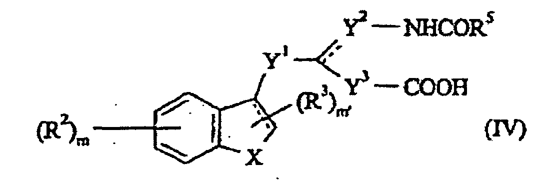
in der R², R³, R⁵, X, Y¹, Y², Y³, m, m' und das Symbol ----- die oben angegebenen Bedeutungen besitzen, nacheinander
- cyclisiert
- mit einer Lewis-Säure umsetzt,
zur Bildung der Verbindung der Formel (I/c), einem Sonderfall der Verbindungen der Formel (I): 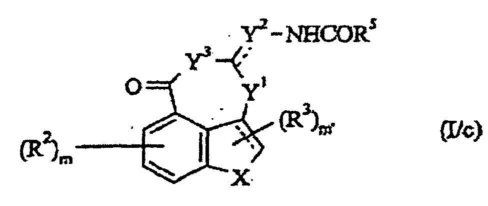 in der R², R³, R⁵, X, Y¹, Y², Y³, m, m' und das Symbol ----- die oben angegebenen Bedeutungen besitzen, welche reduziert wird zur Bildung der Verbindung der Formel (I/d), einem Sonderfall der Verbindungen der Formel (I): 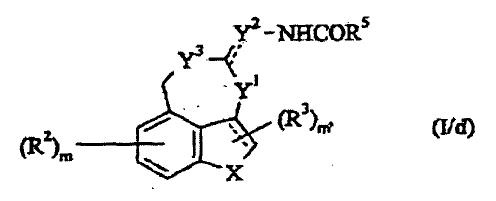 in der R², R³, R⁵, X, Y¹, Y², Y³, m, m' und das Symbol ----- die oben angegebenen Bedeutungen besitzen,
wobei die Gessamtheit der Verbindungen (I/a), (I/b), (I/c) und (I/d) die Verbindungen der Formel (I/e) bilden, einem Sonderfall der Verbindungen der Formel (I): 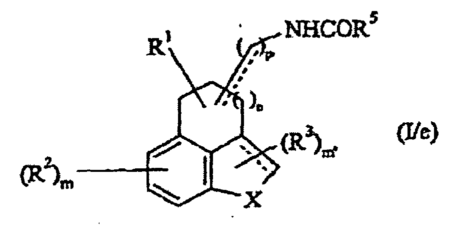 in der R¹, R², R³, R⁵, n, p, X, m, m' und das Symbol ----- die oben angegebenen Bedeutungen besitzen,
welche:
• entweder der Einwirkung einer Verbindung der Formel (V):
R'ₐ-W (V)
in der R'ₐ sämtliche Bedeutungen der Gruppe Rₐ, wie sie oben definiert worden sind, annehmen kann, mit Ausnahme des Wasserstoffatoms, und W eine austretende Gruppe bedeutet, wie ein Halogenatom oder eine Tosylgruppe, unterworfen werden zur Bildung der Verbindung der Formel (I/f), einem Sonderfall der Verbindungen der Formel (I): 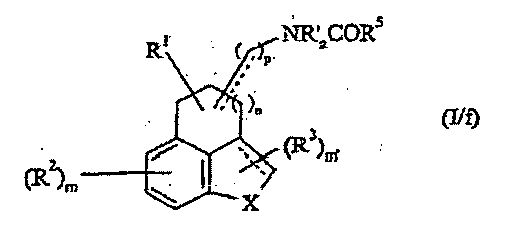 in der R¹, R², R³, R⁵, R'ₐ, n, p, X, m, m' und das Symbol ----- die oben angegebenen Bedeutungen besitzen,
wobei die Gesamtheit der Verbindungen der Formeln (I/e) und (I/f), welche die Verbindung der Formel (I/g) bildet: 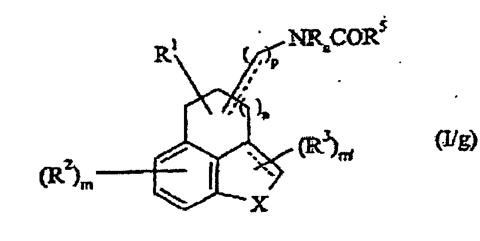 in der R¹, R², R³, R⁵, Rₐ , n, p, X, m, m' und das Symbol ----- die oben angegebenen Bedeutungen besitzen,
der Einwirkung eines Thionierungsmittels, wie dem Lawesson-Reagens, unterworfen werden kann zur Bildung der Verbindung der Formel (I/h), einem Sonderfall der Verbindungen der Formel (I): 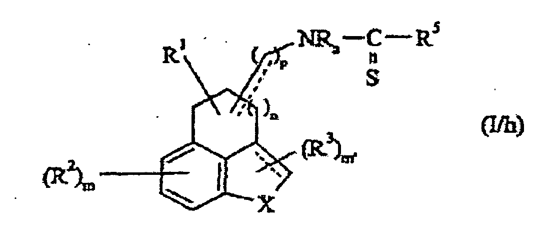 in der R¹, R², R³, R⁵, Rₐ, n, p, X, m, m' und das Symbol ----- die oben angegebenen Bedeutungen besitzen,
• oder in basischem Medium hydrolysiert werden zur Bildung der Verbindung der Formel (VI): 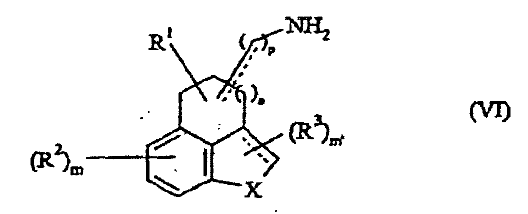
in der R¹, R², R³, n, p, X, m, m' und das Symbol ----- die oben angegebenen Bedeutungen besitzen,
welche:
- entweder der Einwirkung eines Pyriyliumsalzes unterworfen wird zur Bildung der Verbindung der Formel (VII): 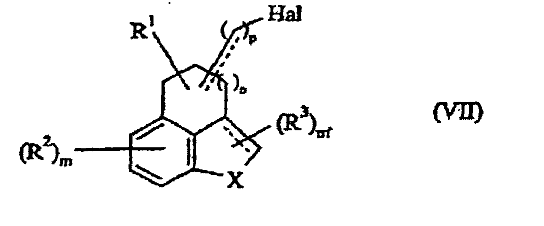
in der Hal ein Halogenatom darstellt und R¹, R², R³, n, p, X, m, m' und das Symbol ----- die oben angegebenen Bedeutungen besitzen,
welche man mit einem Cyanidsalz kondensiert zur Bildung der Verbindung der Formel (VIII): 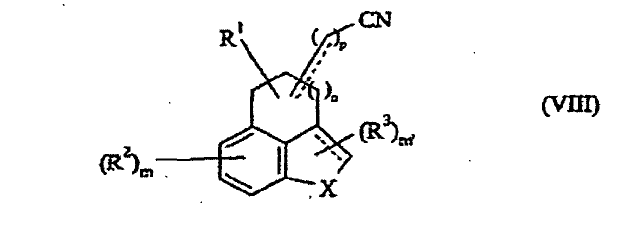 in der R¹, R², R³, n, p, X, m, m' und das Symbol ----- die oben angegebenen Bedeutungen besitzen,
welche in saurem oder basischem Medium hydrolysiert wird zur Bildung der Verbindung der Formel (IX): 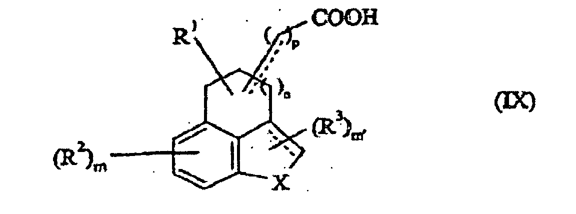 in der R¹, R², R³, n, p, X, m, m' und das Symbol ----- die oben angegebenen Bedeutungen besitzen.
welche nach der Aktivierung in Form des Säurechlorids oder in Gegenwart eines Kupplungsmittels der Einwirkung eines Amins HNR⁶R⁷ unterworfen wird zur Bildung der Verbindung der Formel (I/i), einem Sonderfall der Verbindungen der Formel (I): 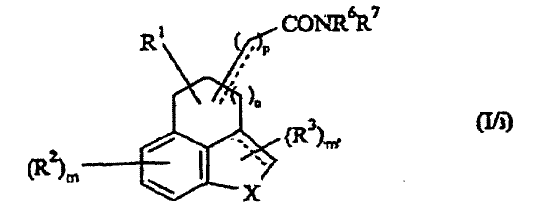 in der R¹, R², R³, R⁶, R⁷, n, p, X, m, m' und das Symbol ----- die oben angegebenen Bedeutungen besitzen,
welche der Einwirkung eines Thionierungsmittels, wie dem Lawesson-Reagens, unterworfen werden kann zur Bildung der Verbindung (I/j), einem Sonderfall der Verbindungen der Formel (I): 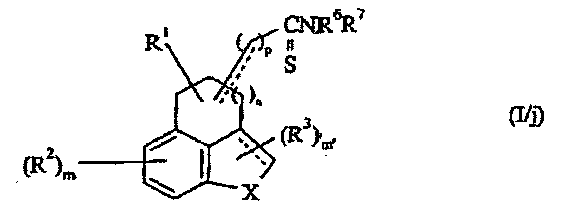 in der R¹, R², R³, R⁶, R⁷, n, p, X, m, m' und das Symbol ----- die oben angegebenen Bedeutungen besitzen,
- oder der Einwirkung einer Verbindung der Formel (X) unterworfen wird:
Z === C === NR⁶R⁷ (X)
in der Z, R⁶ und R⁷ die oben angegebenen Bedeutungen besitzen,
zur Bildung der Verbindung der Formel (I/k), einem Sonderfall der Verbindungen der Formel (I): 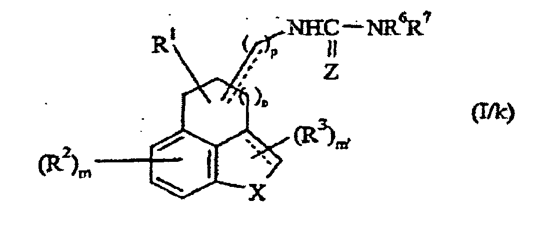 in der R¹, R², R³, R⁶, R⁷, n, p, Z, m, m' und das Symbol ----- die oben angegebenen Bedeutungen besitzen, welche man mit einer Verbindung der Formel (V) kondensieren kann zur Bildung der Verbindung der Formel (I/1), einem Sonderfall der Verbindungen der Formel (I): 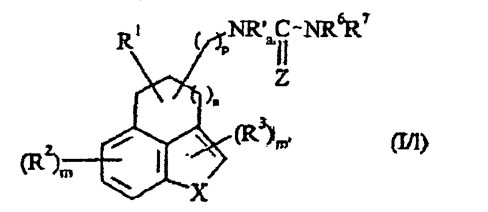 in der R¹, R², R³, R⁶, R⁷, R'ₐ, n, p, X, Z, m, m' und das Symbol ----- die oben angegebenen Bedeutungen besitzen,
wobei die Verbindungen (I/a) bis (I/l) mit Hilfe einer klassischen Trennmethode gereinigt werden können, welche man gewünschtenfalls in ihre Additionssalze mit einer pharmazeutisch annehmbaren Säure oder Base überführen kann und die man gegebenenfalls mit Hilfe einer klassischen Trennmethode in ihre Isomeren trennt.

22. Verfahren zur Herstellung der Verbindungen der Formel (I) nach Anspruch 1, worin die Kette 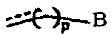 in der Position a oder c steht, **dadurch gekennzeichnet, daß** man als Ausgangsprodukt die Verbindung der Formel (XIV) verwendet: 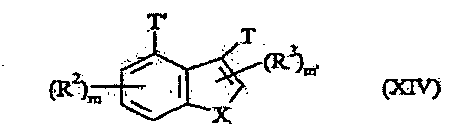 in der R², R³, X, m, m' die oben angegebenen Bedeutungen besitzen und T und T', die verschieden sind, ein Wasserstoffatom oder eine Gruppe -CHO bedeuten,
welche man einer Wittig-Reaktion und dann einer katalytischen Reduktion unterwirft zur Bildung der Verbindung der Formel (XV): 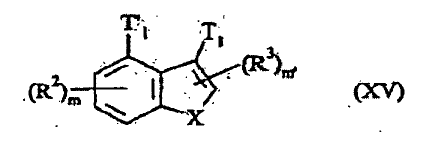 in der R², R³, X, m und m' die oben angegebenen Bedeutungen besitzen und T'₁ und T₁ ein Wasserstoffatom oder eine Gruppe der Formel (XVI) bedeuten: 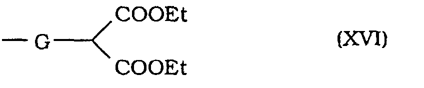 in der G eine Gruppe (CH₂)_{n'} darstellt, worin n' 1, 2 oder 3 bedeuten kann, die gegebenenfalls durch eine Gruppe R¹ substituiert sein kann, wie sie oben definiert worden ist, mit der Maßgabe, daß eine der beiden Gruppen T'₁ oder T₁ ein Wasserstoffatom bedeutet,
welche nacheinander in basischem Medium verseift und dann durch Erhitzen decarboxyliert wird zur Bildung der Verbindung der Formel (XVII): 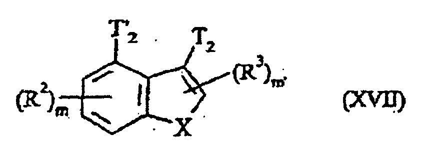 in der R², R³, X, m und m' die oben angegebenen Bedeutungen besitzen und T'₂ und T₂ ein Wasserstoffatom oder eine Gruppe der Formel (XVIII) bedeuten: 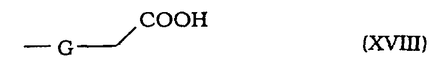 in der G die oben angegebenen Bedeutungen besitzt, mit der Maßgabe, daß eine der beiden Grupen T'₂ oder T₂ ein Wasserstoffatom bedeutet,
welche einer Cyclisierung in Gegenwart einer Lewis-Säure nach der Aktivierung mit Oxalylchlorid unterworfen wird zur Bildung der Verbindung der Formel (XIX): 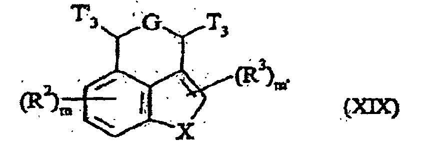 in der R², R³, X, G, m und m' die oben angegebenen Bedeutungen besitzen und T'₃ und R₃, die verschieden sind, ein Wasserstoffatom oder eine Oxogruppe bedeuten, welche man:
- entweder einer Wittig-Reaktion (gegebenenfalls gefolgt von einer Reduktion) und dann einer Verseifung unterwirft zur Bildung der Verbindung der Formel (XX): 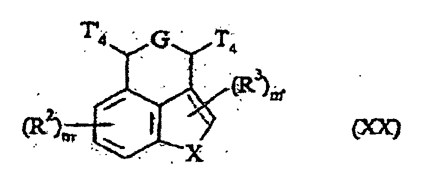 in der R², R³, X, G, m, m' und das Symbol ----- die oben angegebenen Bedeutungen besitzen und T₄ und T'₄ ein Wasserstoffatom bedeuten oder zusammen mit dem sie tragenden Kohlenstoffatom eine Gruppe 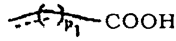 bilden, worin p₁ 1, 2 oder 3 bedeutet, mit der Maßgabe, daß eine der beiden Gruppen T₄ oder T'₄ ein Wasserstoffatom darstellt,
- oder nacheinander
* einer Reduktion zu dem entsprechenden Alkohol
* einer Halogenierung in Gegenwart von beispielsweise SOCl₂
* der Kondensation mit einem Cyanidsalz
* einer sauren oder basischen Hydrolyse
unterworfen wird zur Bildung der Verbindung der Formel (XXI): 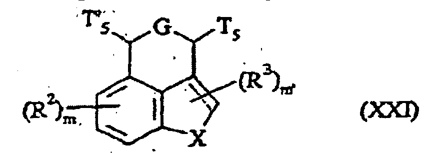 in der R², R³, X, G, m, m' und das Symbol ----- die oben angegebenen Bedeutungen besitzen und T'₅ und T₅, die verschieden sind, ein Wasserstoffatom oder eine Gruppe COOH bedeuten,
wobei die Gesamtheit der Verbindungen (XX) und (XXI), welche die Verbindung der Formel (XXII) bildet: 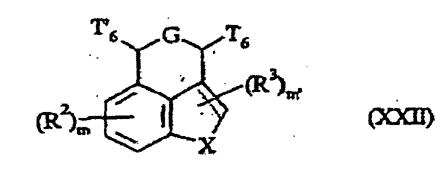 in der R², R³, X, G, m, m' und das Symbol ----- die oben angegebenen Bedeutungen besitzen und T'₆ und T₆ ein Wasserstoffatom bedeuten oder zusammen mit dem sie tragenden Kohlenstoffatom eine Gruppe 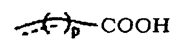 bilden, worin p die oben angegebenen Bedeutungen besitzt, mit der Maßgabe, daß eine der beiden Gruppen T'₆ oder T₆ ein Wasserstoffatom bedeutet,
wobei die Verbindung (XXII) auch ausgehend von der Verbindung der Formel (XIX) durch Kondensation gemäß einer Wittig-Reaktion einer Verbindung, die eine Nitrilgruppe aufweist (und eventueller Reduktion dieser Verbindung) und Hydrolyse des Nitrils hergestellt werden kann, welche:
- entweder nach der Aktivierung in Form des Säurechlorids oder in Gegenwart eines Kupplungsmittels der Einwirkung eines Amins HNR⁶R⁷ unterworfen wird zur Bildung der Verbindung der Formel (I/y), einem Sonderfall der Verbindungen der Formel (I): 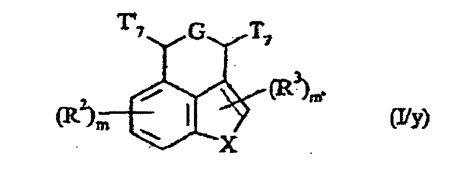
in der R², R³, X, G, m, m' und das Symbol ----- die oben angegebenen Bedeutungen besitzen und T'₇ und T₇ ein Wasserstoffatom bedeuten oder gemeinsam mit dem sie tragenden Kohlenstoffatom eine Gruppe 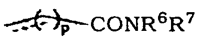 bilden, worin p, R⁶ und R⁷ die oben angegebenen Bedeutungen besitzen, mit der Maßgabe, daß eine der beiden Gruppen T'₇ oder T₇ ein Wasserstoffatom bedeutet,
welche der Einwirkung eines Thionierungsmittels, wie des Lawesson-Reagens unterworfen werden kann zur Bildung der Verbindung (I/z), einem Sonderfall der Verbindungen der Formel (I): 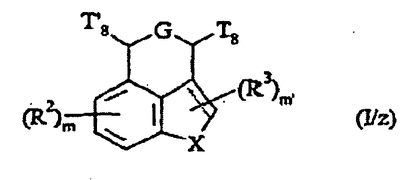 in der R², R³, X, G, m, m' und das Symbol ----- die oben angegebenen Bedeutungen besitzen und T'₈ und T₈ ein Wasserstoffatom bedeuten oder gemeinsam mit dem sie tragenden Kohlenstoffatom die Gruppe 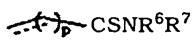 bilden, worin p, R⁶ und R⁷ die oben angegebenen Bedeutungen besitzen, mit der Maßgabe, daß eine der beiden Gruppen T₈ oder T'₈ ein Wasserstoffatom bedeutet,
- oder mit dem Säurechlorid aktiviert und dann mit einem Azid behandelt, zu dem entsprechenden Isocyanat erhitzt und dann hydrolysiert wird zur Bildung der Verbindung der Formel (XXIII): 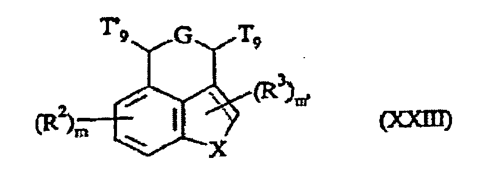
in der R², R³, X, G, m, m' und das Symbol ----- die oben angegebenen Bedeutungen besitzen und T'₉ und T₉ ein Wasserstoffatom bedeuten oder gemeinsam mit dem sie tragenden Kohlenstoffatom eine Gruppe 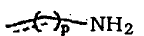 bilden, worin p die oben angegebenen Bedeutungen besitzt, mit der Maßgabe, daß eine der beiden Gruppen T₉ oder T'₉ ein Wasserstoffatom bedeutet,
wobei die Verbindung der Formel (XXIII) auch erhalten werden kann ausgehend von der Verbindung der Formel (XIX) durch Kondensation gemäß einer Wittig-Reaktion einer Verbindung, die ein Nitril aufweist, und durch Reduktion des Nitrils, welche man:
- entweder mit einem Acylchlorid CICOR⁵ oder dem entsprechenden (gemischten oder symmetrischen) Säureanhydrid, worin R⁵ die oben angegebenen Bedeutungen besitzt, kondensiert zur Bildung der Verbindung der Formel (I/aa), einem Sonderfall der Verbindungen der Formel (I): 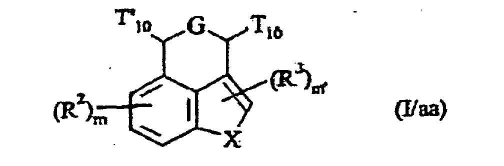
in der R², R³, X, G, m, m' und das Symbol ----- die oben angegebenen Bedeutungen besitzen und T'₁₀ und T₁₀ ein Wasserstoffatom bedeuten oder gemeinsam mit dem sie tragenden Kohlenstoffatom eine Gruppe 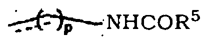 bilden, worin p und R⁵ die oben angegebenen Bedeutungen besitzen, mit der Maßgabe, daß eine der beiden Gruppen T'₁₀ oder T₁₀ ein Wasserstoffatom bedeutet,
welche der Einwirkung eines Thionierungsmittels, wie dem Lawesson-Reagens, unterworfen werden kann und/oder nach der Einwirkung einer Verbindung der Formel (V) substituiert werden kann zur Bildung der Verbindung der Formel (I/ab), einem Sonderfall der Verbindungen der Formel (I): 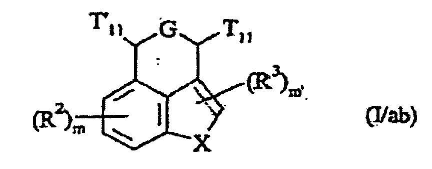 in der R², R³, X, G, m, m' und das Symbol ---- die oben angegebenen Bedeutungen besitzen und T'₁₁ und T₁₁ ein Wasserstoffatom bedeuten oder gemeinsam mit dem sie tragenden Kohlenstoffatom eine Gruppe 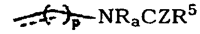 bilden, worin p, Rₐ, R⁵ und Z die oben angegebenen Bedeutungen besitzen, mit der Maßgabe, daß eine der beiden Gruppen T'₁₁ oder T₁₁ ein Wasserstoffatom bedeutet,
wobei die Verbindung (I/y) bis (I/ab) mit Hilfe einer klassischen Trennungsmethode gereinigt werden können, gewünschtenfalls in ihre Additionssalze mit einer pharmazeutisch annehmbaren Säure oder Base überführt werden und gegebenenfalls mit Hilfe einer klassischen Trennmethode in ihre Isomeren getrennt werden.

23. Pharmazeutische Zubereitungen, enthaltend die Produkte der Formel (I) nach einem der Ansprüche 1 bis 20 oder eines ihrer Additionssalze mit einer pharmazeutisch annehmbaren Säure oder Base in Kombination mit einem oder mehreren pharmazeutisch annehmbaren Trägermaterialien.

24. Pharmazeutische Zubereitungen nach Anspruch 23 für die Behandlung von Störungen, die mit dem melatoninergischen System verknüpft sind.

## Claims

1. Compounds of formula (I) : 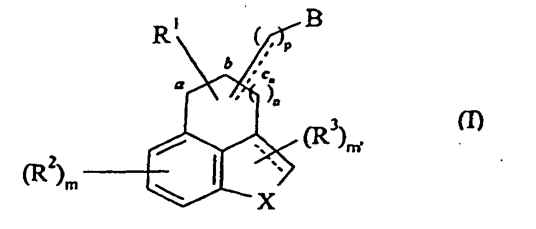 wherein :
◆ R¹ represents a hydrogen atom, a halogen atom or a linear or branched (C₁-C₆)alkyl, linear or branched (C₁-C₆)alkoxy, hydroxy or oxo group,
◆ R² and R³, which may be the same or different, represent a halogen atom or an Rₐ, ORₐ, CORₐ, OCORₐ or COORₐ group (wherein Rₐ represents a hydrogen atom, an optionally substituted linear or branched (C₁-C₆)alkyl group, linear or branched (C₁-C₆)trihaloalkyl, an optionally substituted linear or branched (C₂-C₆)alkenyl group, an optionally substituted linear or branched (C₂-C₆)alkynyl group, an optionally substituted (C₃-C₈)cycloalkyl group, an optionally substituted (C₃-C₈)cycloalkyl(C₁-C₆)alkyl group in which the alkyl moiety is linear or branched, or an optionally substituted aryl group),
◆ the symbols (R²)ₘ and (R³)_{m'} denote that the ring in question may be substituted by from 1 to 3 groups (which may be the same or different) belonging to the definitions for R² and R³,
◆ X represents a (CH₂)_{q} group (wherein q is 1 or 2) or a -CH=CH- group,
◆ n is an integer such that 0 ≤ n ≤ 3
◆ p is an integer such that 1 ≤ p ≤ 3 when n is 1, 2 or 3 and the 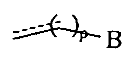 chain is in the b position and such that 0 ≤ p ≤ 3 in all other cases, it being possible for the 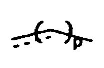 chain to be unsubstituted or substituted by one or more groups, which may be the same or different, selected from Rₐ, ORₐ, CORₐ, COORₐ and halogen atoms,
◆ B represents :
- an 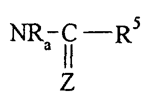 group wherein Rₐ is as defined hereinbefore, Z represents an oxygen atom or a sulphur atom, and R⁵ represents an Rₐ group or an NR⁶R⁷ group wherein R⁶ and R⁷, which may be the same or different, represent an Rₐ group,
- or a 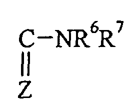 group wherein Z, R⁶ and R⁷ are as defined hereinbefore,
◆ the symbol ----- denotes that the bond may be single or double provided that the valency of the atoms is respected,
it being understood that the symbol 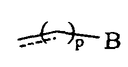 is used to denote the formula 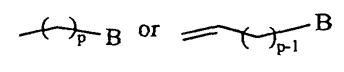 (in which case p is other than 0),
with the proviso that:
- the compound of formula (I) cannot represent N-(4-methyl-2,3-dihydro-*1H*-1-phenalenyl)-1-cyclopropanecarboxamide, N-(4-methyl-2,3-dihydro-*1H*-1-phenalenyl)-2-chloroacetamide, N-(5-hydroxy-1,2,2a,3,4,5-hexahydro-4-acenaphthylenyl)acetamide, N-(5-hydroxy-1,2,2a,3,4,5-hexahydro-4-acenaphthylenyl)benzamide or N-(1,2,2a,3,4,5-hexahydro-4-acenaphthylenyl)acetamide,
it being understood that:
- "aryl" is used to denote a phenyl or naphthyl group each optionally substituted by one or more groups, which may be the same or different, selected from hydroxy, linear or branched (C₁-C₆)alkoxy, linear or branched (C₁-C₆)alkyl, cyano, nitro, amino, trihaloalkyl, and halogen atoms,
- the expression "optionally substituted" applied to the terms "alkyl", "alkenyl" and "alkynyl" denotes that those groups may be substituted by one or more groups, which may be the same or different, selected from hydroxy, linear or branched (C₁-C₆)-alkoxy, aryl, and halogen atoms,
- the expression "optionally substituted" applied to the terms "cycloalkyl" and "cycloalkylalkyl" denotes that the cyclic moiety may be substituted by one or more groups, which may be the same or different, selected from hydroxy, linear or branched (C₁-C₆)alkoxy, oxo, and halogen atoms,
their enantiomers and diastereoisomers, and addition salts thereof with a pharmaceutically acceptable acid or base.

2. Compounds of formula (I) according to claim I wherein n represents an integer 0, 1 or 2, their enantiomers and diastereoisomers, and addition salts thereof with a pharmaceutically acceptable acid or base.

3. Compounds of formula (I) according to claim I forming a 2,3-dihydrophenalene, 1,2-dihydroacenaphthylene or 7,8,9,10-tetrahydrocyclohepta[*de*] naphtalene tricyclic system, their enantiomers and diastereoisomers, and addition salts thereof with a pharmaceutically acceptable acid or base.

4. Compounds of formula (I) according to claim 1 wherein p represents an integer 0, 1 or 2, their enantiomers and diastereoisomers, and addition salts thereof with a pharmaceutically acceptable acid or base.

5. Compounds of formula (I) according to claim 1 wherein R² and R³, which may be the same or different, represent an alkoxy or alkyl group or a hydrogen atom, their enantiomers and diastereoisomers, and addition salts thereof with a pharmaceutically acceptable acid or base.

6. Compound of formula (I) according to claim 1 wherein R¹ represents a hydrogen atom, their enantiomers and diastereoisomers, and addition salts thereof with a pharmaceutically acceptable acid or base.

7. Compounds of formula (I) according to claim 1 wherein the 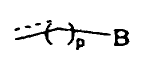 chain is in the a or c position, their enantiomers and diastereoisomers, and addition salts thereof with a pharmaceutically acceptable acid or base.

8. Compounds of formula (I) according to claim I wherein the 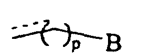 chain is in the a or c position and p represents an integer 0 (in which case the bond ----- is single), 1 or 2, their enantiomers and diastereoisomers, and addition salts thereof with a pharmaceutically acceptable acid or base.

9. Compounds of formula (I) according to claim 1 wherein B represents an NHCOR⁵ group, their enantiomers and diastereoisomers, and addition salts thereof with a pharmaceutically acceptable acid or base.

10. Compounds of formula (I) according to claim 1 wherein B represents a CONHR⁶ group, their enantiomers and diastereoisomers, and addition salts thereof with a pharmaceutically acceptable acid or base.

11. Compounds of formula (I) according to claim 1 forming a 2,3-dihydrophenalene, 1,2-dihydroacenaphthylene or 7,8,9,10-tetrahydrocyclohepta[*de*]naphtalene tricyclic system, each unsubstituted or substituted on the naphtalene moiety by one or more alkoxy or alkyl groups, and substituted in the a or c position by a 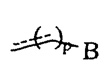 group wherein B represents an NHCOR⁵ or CONHR⁶ group, their enantiomers and diastereoisomers, and addition salts thereof with a pharmaceutically acceptable acid or base.

12. Compounds of formula (I) according to claim 1 forming a 1,2-dihydroacenaphthylene or 7,8,9,10-tetrahydrocyclohepta[*de*]naphtalene tricyclic system, each unsubstituted or substituted on the naphtalene moiety by one or two alkoxy groups and substituted in the a or c position by a =CH-B, =CH-CH₂-B, -B, -CH₂-B or -(CH₂)₂-B group wherein B represents an NHCOR⁵ or CONHR⁶ group, their enantiomers and diastereoisomers, and addition salts thereof with a pharmaceutically acceptable acid or base.

13. Compounds of formula (I) according to claim 1 forming a 2,3-dihydrophenalene tricyclic system, unsubstituted or substituted on the naphtalene moiety by one or two alkoxy groups and substituted in the a or c position by a =CH-B, =CH-CH₂-B, -CH₂-B or -(CH₂)₂-B group wherein B represents an NHCOR⁵ or CONHR⁶ group, their enantiomers and diastereoisomers, and addition salts thereof with a pharmaceutically acceptable acid or base.

14. Compounds of formula (I) according to claim 1 which are : N-[(4-methoxy-2,3-dihydro-*1H*-1-phenalenyl)methyl]acetamide, N-[(4-methoxy-2,3-dihydro-*1H*-phenalenyl)methyl]propionamide, N-[(4-methoxy-2,3-dihydro-*1H*-1-phenalenyl)methyl]-cyclopropanecarboxamide, N-[(4-methoxy-2,3-dihydro-*1H*-1-phenalenyl)methyl]-butanamide, N-[(9-methoxy-2,3-dihydro-*1H*-1-phenalenyl)methyl]butanamide, N-[2-(9-methoxy-2,3-dihydro-*1H*-1-phenalenyl)methyl]acetamide, N-[2-(9-methoxy-2,3-dihydro-*1H*-1-phenalenyl)methyl]butanamide, N-[2-(4,9-dimethoxy-2,3-dihydro-*1H*-1-phenalenyl)methyl]acetamide, N-[2-(4,9-dimethoxy-2,3-dihydro-*1H*-1-phenalenyl)-methyl]propanamide, N-[2-(4,9-dimethoxy-2,3-dihydro-*1H*-1-phenalenyl)methyl]-butanamide and N-[2-(4,9-dimethoxy-2,3-dihydro-*1H*-1-phenalenyl)methyl]-1-cyclopropanecarboxamide, their enantiomers and diastereoisomers, and addition salts thereof with a pharmaceutically acceptable acid or base.

15. Compounds of formula (I) according to claim 1 which are : N-[2-(4-methoxy-2,3-dihydro-*1H*-1-phenalenyl)ethyl]acetamide, N-[2-(4-methoxy-2,3-dihydro-*1H*-1-phenalenyl)ethyl]propanamide, N-[2-(4-methoxy-2,3-dihydro-*1H*-1-phenalenyl)-ethyl]-1-cyclopropanecarboxamide, N-[2-(9-methoxy-2,3-dihydro-*1H*-1-phenalenyl)-ethyl]acetamide, N-[2-(9-methoxy-2,3-dihydro-*1H*-1-phenalenyl)ethyl]butanamide, N-[2-(4-methoxy-2,3-dihydro-*1H*-1-phenalenyl)ethyl]butanamide, N-[2-(4,9-dimethoxy-2,3-dihydro-*1H*-1-phenalenyl)ethyl]propanamide, N-[2-(4,9-dimethoxy-2,3-dihydro-*1H*-1-phenalenyl)ethyl]butanamide, N-[2-(4,9-dimethoxy-2,3-dihydro-*1H*-1-phenalenyl)ethyl]-1-cyclopropanecarboxamide and N-[2-(4,9-dimethoxy-2,3-dihydro-*1H*-1-phenalenyl)ethyl]acetamide, their enantiomers and diastereoisomers, and addition salts thereof with a pharmaceutically acceptable acid or base

16. Compound of formula (I) according to claim 1 which is N-(8-methoxy-1,2-dihydro-1-acenaphthylenyl)acetamide, its enantiomers and diastereoisomers, and addition salts thereof with a pharmaceutically acceptable acid or base.

17. Compounds of formula (I) according to claim 1 which are : *(E)*-N-methyl-2-(4-methoxy-2,3-dihydro-*1H*-1-phenalenylidene)acetamide and *(Z)*-N-methyl-2-(4-methoxy-2,3-dihydro-*1H*-1-phenalenylidene)acetamide, their enantiomers and diastereoisomers, and addition salts thereof with a pharmaceutically acceptable acid or base.

18. Compounds of formula (I) according to claim 1 which are : N-(1,2-dihydro-1-acenaphthylenylmethyl)acetamide, N-(1,2-dihydro-1-acenaphthylenylmethyl)propanamide, N-(1,2-dihydro-1-acenaphthylenylmethyl)butanamide, N-(1,2-dihydro-1-acenaphthylenylmethyl)-1-cyclopropanecarboxamide, N-(8-methoxy-1,2-dihydro-1-acenaphthylmethyl)acetamide, N-(8-methoxy-1,2-dihydro-1-acenaphthylmethyl)-propanamide, N-(8-methoxy-1,2-dihydro-1-acenaphthylmethyl)-1-cyclopropane-carboxamide and N-(8-methoxy-1,2-dihydro-1-acenaphthylmethyl)butanamide, their enantiomers and diastereoisomers, and addition salts thereof with a pharmaceutically acceptable acid or base.

19. Compounds of formula (I) according to claim 1 which are : N-[2-(1,2-dihydro-1-acenaphthyl)ethyl]acetamide, N-[2-(1,2-dihydro-1-acenaphthyl)ethyl]propanamide, N-[2-(1,2-dihydro-1-acenaphthyl)ethyl]butanamide, N-[2-(1,2-dihydro-1-acenaphthyl)ethyl]cyclopropanecarboxamide, N-[2-(8-methoxy-1,2-dihydro-1-acenaphthyl)-ethyl]acetamide, N-[2-(8-methoxy-1,2-dihydro-1-acenaphthyl)ethyl]propanamide, N-[2-(8-methoxy-1,2-dihydro-1-acenaphthyl)ethyl]butanamide and N-[2-(8-methoxy-1,2-dihydro-1-acenaphthyl)ethyl]-1-cyclopropanecarboxamide, their enantiomers and diastereoisomers, and addition salts thereof with a pharmaceutically acceptable acid or base.

20. Compound of formula (I) according to claim 1 which is N-[2-(1-methoxy-7,8,9,10-tetrahydrocyclohepta[*de*]naphthalen-7-ylidene)ethyl]propanamide, its enantiomers and diastereoisomers, and addition salts thereof with a pharmaceutically acceptable acid or base.

21. Process for the preparation of compounds of formula (I) according to claim 1, which process is **characterised in that** there is used as starting material a compound of formula (II) : 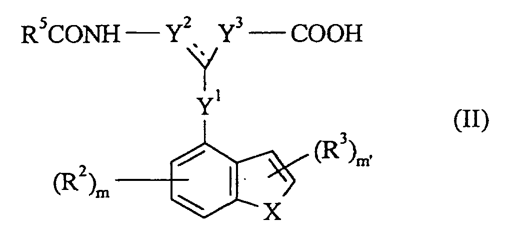 wherein R², R³, R⁵, X, m, m' and the symbol ----- are as defined hereinbefore,
Y² represents a (CH₂)_{q} group (wherein q is 1, 2 or 3, or q is 0 when the symbol ----- is a single bond),
Y¹ represents a (CH₂)_{q'} group (wherein q' is 0, 1, 2 or 3), substituted by an R¹ group as defined hereinbefore,
Y³ represents a (CH₂)_{q"} group (wherein q" is 0, 1, 2 or 3), substituted by an R¹ group as defined hereinbefore, where q'+q" ≤ 3 and R¹ must represent a hydrogen atom in at least one of the two groups Y¹ and Y³,
which is cyclised in a basic medium to yield a compound of formula (III) : 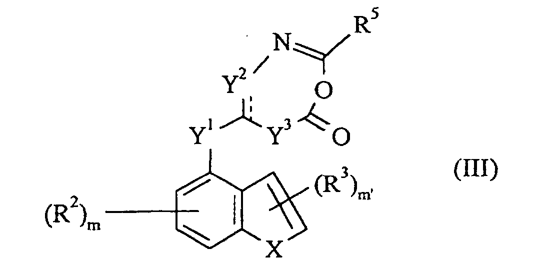 wherein R², R³, R⁵, X, Y¹, Y², Y³, m, m' and the symbol ----- are as defined hereinbefore, which is then reacted with a Lewis acid to obtain a compound of formula (I/a), which is a particular case of the compounds of formula (I) : 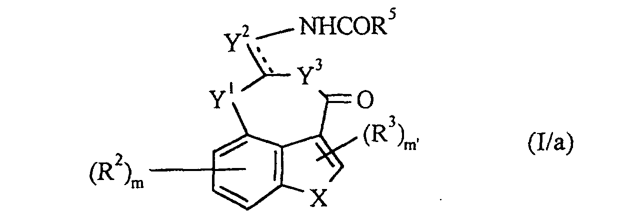 wherein R², R³, R⁵, X, Y¹, Y², Y³, m, m' and the symbol ----- are as defined hereinbefore, which is then reduced to obtain a compound of formula (I/b), which is a particular case of the compounds of formula (I) : 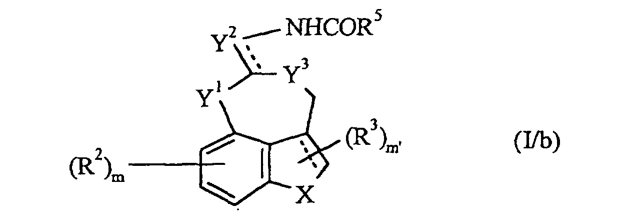 wherein R², R³, R⁵, X, Y¹, Y², Y³, m, m' and the symbol ----- are as defined hereinbefore,
◆ or a compound of formula (IV) : 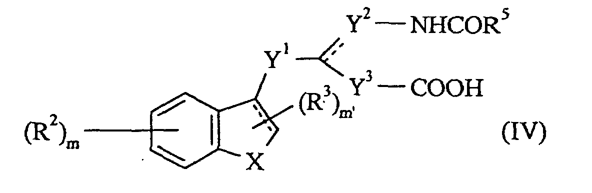
wherein R², R³, R⁵, X, Y¹, Y², Y³, m, m' and the symbol ----- are as defined hereinbefore, which is successively
- cyclised
- reacted with a Lewis acid
to yield a compound of formula (I/c), which is a particular case of the compounds of formula (I) : 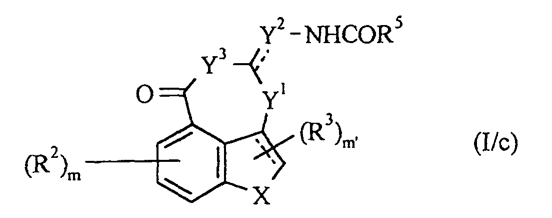 wherein R², R³, R⁵, X, Y¹, Y², Y³, m, m' and the symbol ----- are as defined hereinbefore, which is reduced to obtain a compound of formula (I/d), which is a particular case of the compounds of formula (I) : 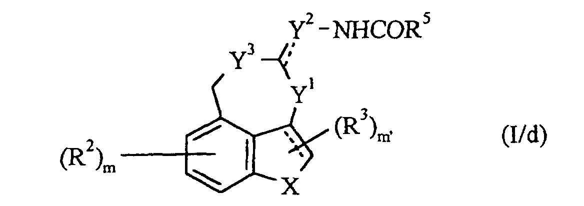 wherein R², R³, R⁵, X, Y¹, Y², Y³, m, m' and the symbol ----- are as defined hereinbefore, the totality of the compounds (I/a), (I/b), (I/c) and (I/d) constituting the compounds of formula (I/e), a particular case of the compounds of formula (I) : 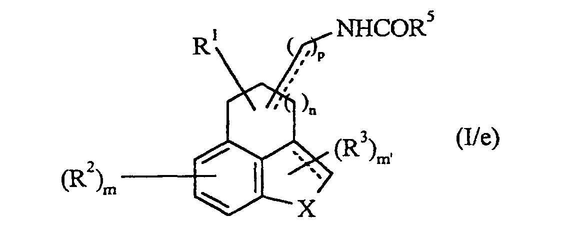 wherein R¹, R², R³, R⁵, n, p, X, m, m' and the symbol ----- are as defined hereinbefore,
which is either:
• subjected to the action of a compound of formula (V) : R'ₐ-W (V) wherein R'ₐ may have any of the meanings of the Rₐ group as defined hereinbefore with the exception of a hydrogen atom, and W represents a leaving group, such as a halogen atom or a tosyl group, to yield a compound of formula (I/f), which is a particular case of the compounds of formula (I) : 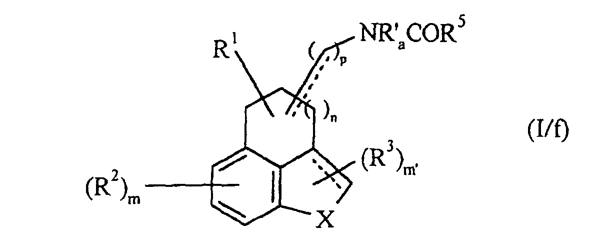
wherein R¹, R², R³, R⁵, R'ₐ, n, p, X, m, m' and the symbol ----- are as defined hereinbefore, the totality of the compounds of formulae (I/e) and (I/f) constituting the compounds of formula (I/g) : 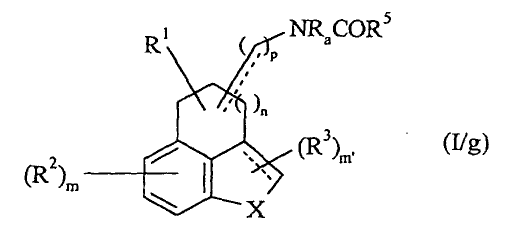 wherein R¹, R², R³, R⁵, Rₐ, n, p, X, m, m' and the symbol ----- are as defined hereinbefore, which may be subjected to the action of a thionisation agent, such as Lawesson's reagent, to obtain a compound of formula (I/h), which is a particular case of the compounds of formula (I) : 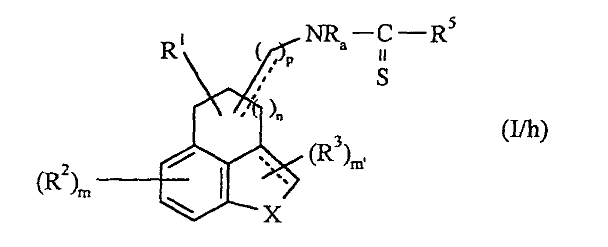 wherein R¹, R², R³, R⁵, Rₐ, n, p, X, m, m' and the symbol ----- are as defined hereinbefore,
• or hydrolysed in a basic medium to yield a compound of formula (VI) : 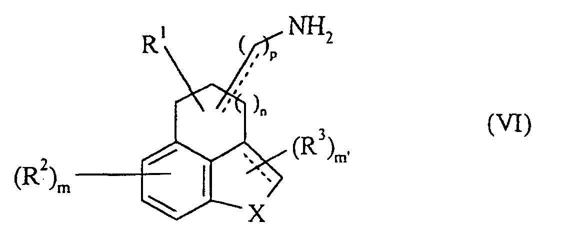
wherein R¹, R², R³, n, p, X, m, m' and the symbol ----- are as defined hereinbefore,
which is either:
- subjected to the action of a pyrylium salt to yield a compound of formula (VII) : 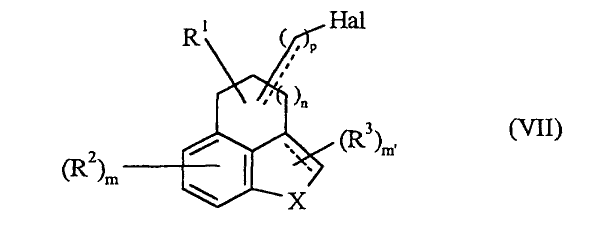
wherein Hal represents a halogen atom and R¹, R², R³, n, p, X, m, m' and the symbol ----- are as defined hereinbefore,
which is condensed with a cyanide salt to obtain a compound of formula (VIII) : 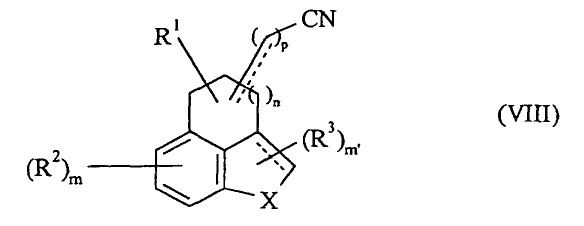 wherein R¹, R², R³, n, p, X, m, m' and the symbol ----- are as defined hereinbefore,
which is hydrolysed in an acidic or basic medium to yield a compound of formula (IX) : 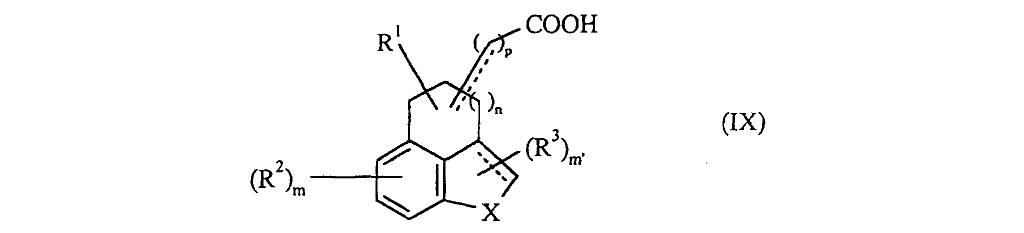 wherein R¹, R², R³, n, p, X, m, m' and the symbol ----- are as defined hereinbefore,
which is subjected, after activation to the acid chloride or in the presence of a coupling agent, to the action of an amine HNR⁶R⁷ to yield a compound of formula (I/i), which is a particular case of the compounds of formula (I) : 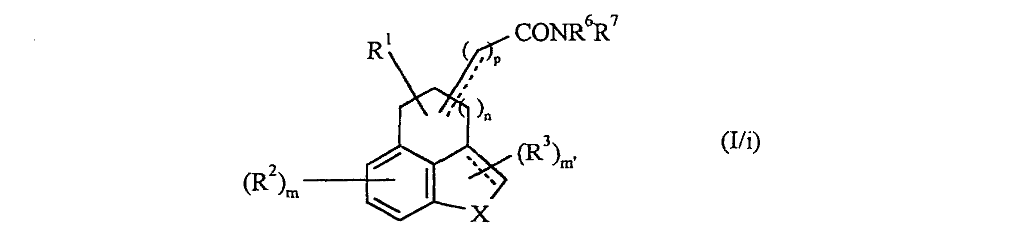 wherein R¹, R², R³, R⁶, R⁷, n, p, X, m, m' and the symbol ----- are as defined hereinbefore,
which may be subjected to the action of a thionisation agent, such as Lawesson's reagent, to obtain a compound (I/j), which is a particular case of the compounds of formula (I) : 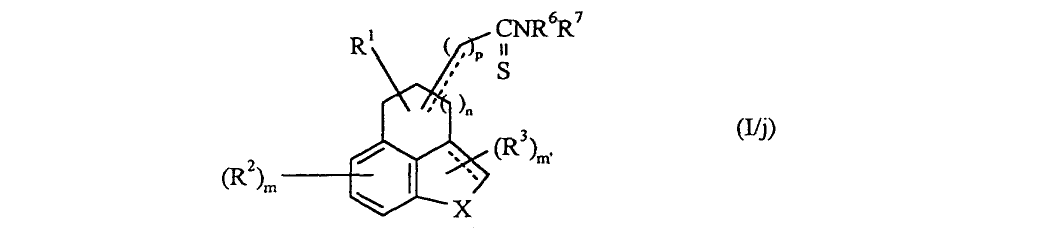 wherein R¹, R², R³, R⁶, R⁷, n, p, X, m, m' and the symbol ----- are as defined hereinbefore,
- or subjected to the action of a compound of formula (X) :
Z=C=NR⁶R⁷ (X)
wherein Z, R⁶ and R⁷ are as defined hereinbefore,
to yield a compound of formula (I/k), which is a particular case of the compounds of formula (1) : 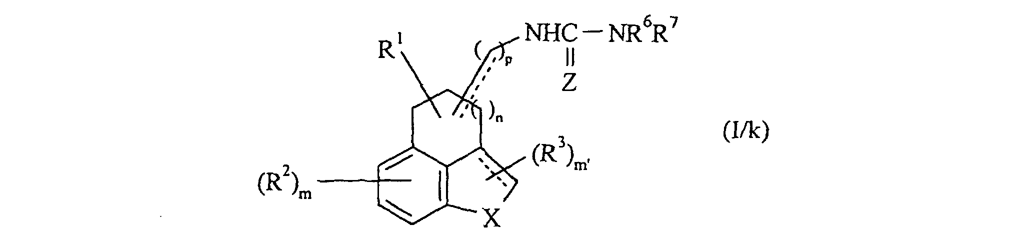 wherein R¹, R², R³, R⁶, R⁷, n, p, Z, m, m' and the symbol ----- are as defined hereinbefore, which may be condensed with a compound of formula (V) to yield a compound of formula (I/1), which is a particular case of the compounds of formula (I) : 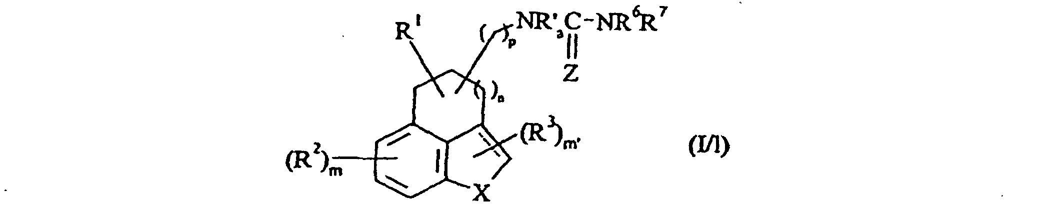 wherein R¹, R², R³, R⁶, R⁷, R'ₐ, n, p, X, Z, m, m' and the symbol ----- are as defined hereinbefore,
which compounds (I/a) to (I/I) can be purified according to a conventional separation technique, are converted, if desired, into their addition salts with a pharmaceutically acceptable acid or base, and separated, where appropriate, into their isomers according to a conventional separation technique.

22. Process for the preparation of compounds of formula (I) according to claim 1 wherein the 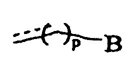 chain is in the a or c position, which process is **characterised in that** there is used as starting material a compound of formula (XIV): 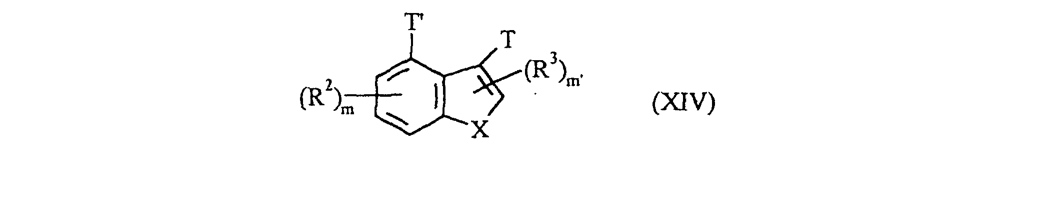 wherein R², R³, X, m and m' are as defined hereinbefore and T and T', which are different, represent a hydrogen atom or a -CHO group,
which is subjected to a Wittig reaction and then to catalytic reduction to obtain a compound of formula (XV) : 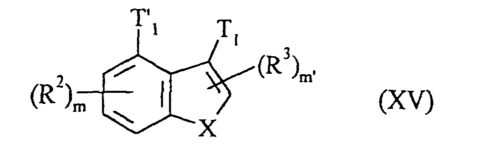 wherein R², R³, X, m and m' are as defined hereinbefore and T'₁ and T₁ represent a hydrogen atom or a group of formula (XVI) : 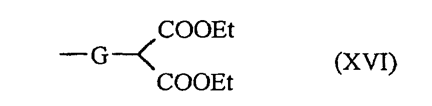 wherein G represents a (CH₂)_{n'} group wherein n'=1, 2 or 3 optionally substituted by an R¹ group as defined hereinbefore, with the proviso that one of the two groups T'₁ and T₁ represents a hydrogen atom,
which is successively hydrolysed in a basic medium and then decarboxylated by heating to yield a compound of formula (XVII) : 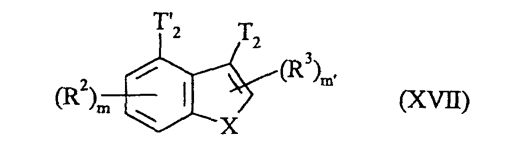 wherein R², R³, X, m and m' are as defined hereinbefore and T'₂ and T₂ represent a hydrogen atom or a group of formula (XVIII) : 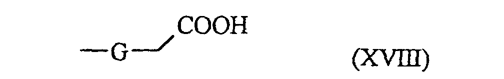 wherein G is as defined hereinbefore, with the proviso that one of the two groups T'₂ and T₂ represents a hydrogen atom,
which is subjected to cyclisation in the presence of a Lewis acid after activation to the oxalyl chloride, to yield a compound of formula (XIX) : 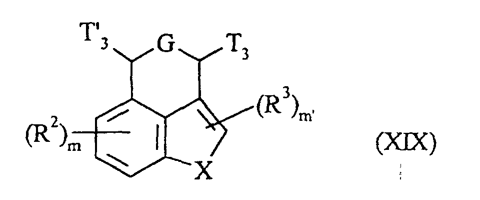 wherein R², R³, X, G, m and m' are as defined hereinbefore, and T'₃ and T₃, which are different, represent a hydrogen atom or an oxo group,
which is subjected either :
- to a Wittig reaction (optionally followed by reduction) and then to hydrolysis to yield a compound of formula (XX) : 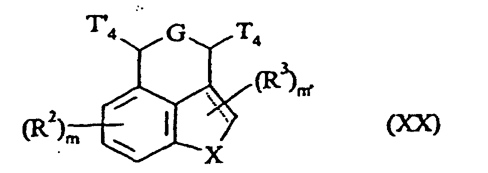
wherein R², R³, X, G, m, m' and the symbol ----- are as defined hereinbefore, and T₄ and T'₄ represent a hydrogen atom or form, with the carbon atom carrying them,
a 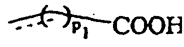 group wherein p₁ is 1, 2 ou 3, with the proviso that one of the two groups T₄ and T'₄ represents a hydrogen atom,
- or successively
* to reduction to the corresponding alcohol
* to halogenation in the presence of SOCl₂ for example
* to condensation with a cyanide salt
* to acidic or basic hydrolysis
to yield a compound of formula (XXI) : 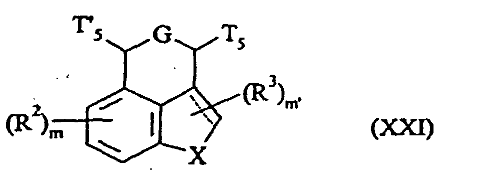 wherein R², R³, X, G, m, m' and the symbol ----- are as defined hereinbefore, and T'₅ and T₅, which are different, represent a hydrogen atom or a COOH group,
the totality of the compounds (XX) and (XXI) constituting the compounds of formula (XXII) : 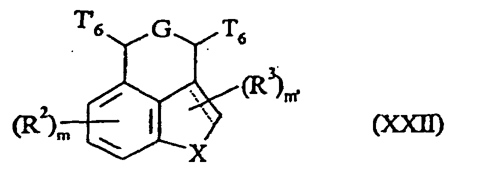 wherein R², R³, X, G, m, m' and the symbol ----- are as defined hereinbefore, and T'₆ and T₆ represent a hydrogen atom or form, with the carbon atom carrying them,
a 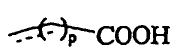 group wherein p is as defined hereinbefore, with the proviso that one of the two groups T'₆ and T₆ represents a hydrogen atom,
which compound (XXII) can also be obtained starting from a compound of formula (XIX) by condensation according to a Wittig reaction with a compound containing a nitrile group (followed by optional reduction of the double bond), and hydrolysis of the nitrile, which compound (XXII) is either :
- subjected, after activation to the acid chloride or in the presence of a coupling agent, to the action of an amine HNR⁶R⁷ to yield a compound of formula (I/y), which is a particular case of the compounds of formula (1) : 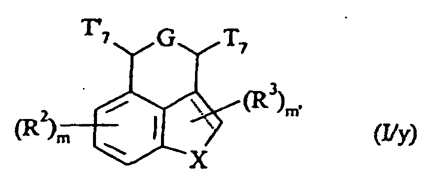
wherein R², R³, X, G, m, m' and the symbol ----- are as defined hereinbefore, and T'₇ and T₇ represent a hydrogen atom or form, with the carbon atom carrying them, a 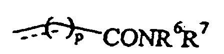 group wherein p, R⁶ and R⁷ are as defined hereinbefore, with the proviso that one of the two groups T'₇ and T₇ represents a hydrogen atom,
which may be subjected to the action of a thionisation agent, such as Lawesson's reagent, to obtain a compound (I/z), which is a particular case of the compounds of formula (I) : 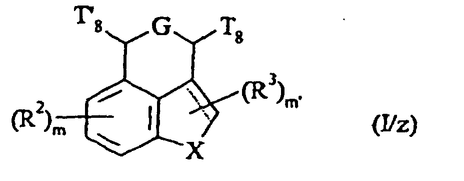 wherein R², R³, X, G, m, m' and the symbol ----- are as defined hereinbefore, and T'₈ and T₈ represent a hydrogen atom or form, with the carbon atom carrying them; a 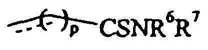 group wherein p, R⁶ and R⁷ are as defined hereinbefore, with the proviso that one of the two groups T₈ and T'₈ represents a hydrogen atom,
- or activated to the acid chloride, and then treated with an azide, heated to the corresponding isocyanate and then hydrolysed to yield a compound of formula (XXIII) : 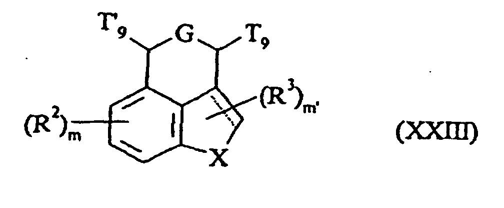
wherein R², R³, X, G, m, m' and the symbol ----- are as defined hereinbefore, and T'₉ and T₉ represent a hydrogen atom or form, with the carbon atom carrying them, a 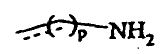 group wherein p is as defined hereinbefore, with the proviso that one of the two groups T₉ and T'₉ represents a hydrogen atom,
which compound of formula (XXIII) can also be obtained starting from a compound of formula (XIX) by condensation according to a Wittig reaction with a compound containing a nitrile group followed by reduction of the nitrile,
which compound of formula (XXIII) is condensed with:
- an acyl chloride ClCOR⁵ or the corresponding acid anhydride (mixed or symmetrical) wherein R⁵ is as defined hereinbefore, to yield a compound of formula (I/aa), which is a particular case of the compounds of formula (I) : 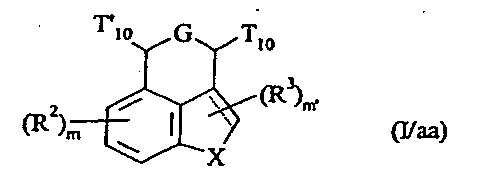
wherein R², R³, X, G, m, m' and the symbol ----- are as defined hereinbefore, and T'₁₀ and T₁₀ represent a hydrogen atom or form, with the carbon atom carrying them, a 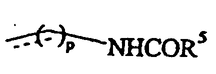 group wherein p and R⁵ are as defined hereinbefore, with the proviso that one of the two groups T'₁₀ and T₁₀ represents a hydrogen atom,
which may be subjected to the action of a thionisation agent, such as Lawesson's reagent, and/or substituted after the action of a compound of formula (V) to yield a compound of formula (I/ab), which is a particular case of the compounds of formula (1) : 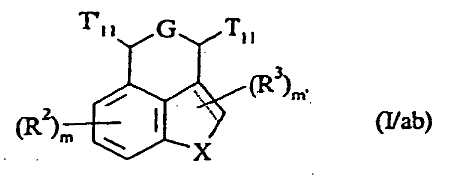 wherein R², R³, X, G, m, m' and the symbol ----- are as defined hereinbefore, and T₁₁ and T₁₁ represent a hydrogen atom or form, with the carbon atom carrying them, a 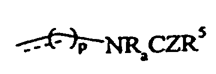 group wherein p, Rₐ, R⁵ and Z are as defined hereinbefore, with the proviso that one of the two groups T'₁₁ and T₁₁ represents a hydrogen atom,
which compounds (I/y) to (I/ab) can be purified according to a conventional separation technique, are converted, if desired, into their addition salts with a pharmaceutically acceptable acid or base, and separated, where appropriate, into their isomers according to a conventional separation technique.

23. Pharmaceutical compositions comprising the compounds of formula (I) according to any one of claims 1 to 20 or an addition salt thereof with a pharmaceutically acceptable acid or base in combination with one or more pharmaceutically acceptable excipients.

24. Pharmaceutical compositions according to claim 23 for use in the treatment of disorders associated with the melatoninergic system.
